(19) **Europäisches Patentamt / European Patent Office / Office européen des brevets**

(11) **EP 4 596 577 A2**

(12) # EUROPEAN PATENT APPLICATION

(43) Date of publication:
**06.08.2025   Bulletin 2025/32**

(21) Application number: **25175113.7**

(22) Date of filing: **30.12.2021**

(51) International Patent Classification (IPC):
**C07K 14/805** (2006.01)

(52) Cooperative Patent Classification (CPC):
**A23L 13/424; A23J 3/227; A23J 3/26;**
**A23L 13/426; A23L 13/43; C07K 14/805;**
C12R 2001/84

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **31.12.2020   EP 20218000**
**19.05.2021   EP 21174597**

(62) Document number(s) of the earlier application(s) in
accordance with Art. 76 EPC:
**21847742.0 / 4 271 200**

(71) Applicant: **Paleo B.V.**
**3290 Diest (BE)**

(72) Inventors:
• **SANCTORUM, Hermes**
**3290 Diest (BE)**

• **DE JONG, Andy**
**3290 Diest (BE)**

(74) Representative: **Nederlandsch Octrooibureau**
**P.O. Box 29720**
**2502 LS The Hague (NL)**

Remarks:
•The complete document including Reference Table(s) and the Sequence Listing(s) can be downloaded from the EPO website
•This application was filed on 08-05-2025 as a divisional application to the application mentioned under INID code 62.

(54) **MEAT SUBSTITUTE COMPRISING ANIMAL MYOGLOBIN**

(57)    Described herein is a meat substitute or food ingredient comprising an animal myoglobin protein, gene construct comprising a nucleic acid encoding said protein, a host cell comprising said gene construct and a method for producing said myoglobin or said meat substitute.

EP 4 596 577 A2

**Description**

**Field**

Aspects and embodiments described herein relate to the field of meat substitute comprising a myoglobin protein, gene construct comprising a nucleic acid encoding said protein, a host cell comprising said gene construct and a method for producing said myoglobin or said meat substitute.

**Background**

Several meat substitutes have already been developed and commercialized. The flavor, color, texture and nutritional qualities of such products are not always optimal in the sense that they do not properly mimick "meat". Moreover, they may also not have the same nutritional qualities as meat.

Therefore, there is still a need for new inventions which do not have all the drawbacks of existing food products aiming at substituting meat.

**Description**

Accordingly, the aspects and embodiments of the present invention as described herein solve at least some of the problems and needs as discussed herein.

Meat substitute

In a first aspect there is provided a meat substitute or a food ingredient comprising an animal myoglobin, preferably a mammalian myoglobin, more preferably from mammoth, pig, sheep or cow, more preferably wherein said mammoth is a woolly mammoth (*Mammuthus primigenius*) or a steppe mammoth (*Mammuthus trogontherii*), most preferably wherein said mammoth is a steppe mammoth, or avian myoglobin, preferably from chicken, or a fish myoglobin, preferably from tuna, or a myoglobin derived therefrom.

In an embodiment, the meat substitute or food ingredient comprises an animal myoglobin from mammoth, pig, sheep, cow, chicken or tuna or derived therefrom, preferably wherein said mammoth is a woolly mammoth (*Mammuthus primigenius*) or a steppe mammoth (*Mammuthus trogontherii*), more preferably wherein said mammoth is a steppe mammoth.

An animal myoglobin may be said to be derived from a mammoth, which is preferably a woolly mammoth or a steppe mammoth, more preferably a steppe mammoth, pig, sheep, cow, chicken or tuna myoglobin when its amino acid sequence is derived from the one of a mammoth, preferably from a woolly mammoth, or a steppe mammoth, pig, sheep, cow, chicken or tuna, respectively, by addition, deletion and/or substitution of at least an amino acid. Addition, deletion and/or substitutions of two, three, four, five, six, seven, eight, nine or ten amino acids is also contemplated by the invention. Examples of myoglobin amino acid sequence from a woolly mammoth, steppe mammoth, pig, sheep, cow, chicken or tuna have been disclosed later on by a given SEQ ID NO. Such animal myoglobin derived from a mammoth, woolly mammoth, steppe mammoth, pig, sheep, cow, chicken or tuna myoglobin may also exert at least a detectable level of an activity of an animal myoglobin as explained later herein. A mammoth may be any species in the genus *Mammuthus,* such as a woolly mammoth (*Mammuthus primigenius*) or a steppe mammoth (*Mammuthus trogontherii*), in the context of this application, unless explicitly stated otherwise.

In an embodiment, the myoglobin in the meat substitute or in the food ingredient is from a mammoth or derived therefrom.

In a preferred embodiment, the myoglobin in the meat substitute or in the food ingredient is from a woolly mammoth or derived therefrom.

In a preferred embodiment, the myoglobin in the meat substitute or in the food ingredient is from a steppe mammoth or derived therefrom.

In an embodiment, the myoglobin in the meat substitute or in the food ingredient is represented by one of the following amino acid sequences:

- a) comprising at least 70% sequence identity with SEQ ID NO: 3, and having at least one of the following amino acid combinations:

  ○ F at position 30, and/or
  ○ Q at position 65, and/or
  ○ H at position 92, and/or

- H at position 94, and/or
- F at position 30 and Q at position 65, and/or
- F at position 30 and H at position 92, and/or
- F at position 30 and H at position 94, and/or
- Q at position 65 and H at position 92, and/or
- Q at position 65 and H at position 94, and/or
- H at position 92 and H at position 94, and/or
- F at position 30 and Q at position 65 and H at position 92, and/or
- F at position 30 and Q at position 65 and H at position 94, and/or
- F at position 30 and H at position 92 and H at position 94, and/or
- Q at position 65 and H at position 92 and H at position 94, and/or
- F at position 30 and Q at position 65 and H at position 92 and H at position 94;

- b) comprising at least 70% sequence identity with SEQ ID NO: 2 or 3, Q or H at position 65 and H at position 94 and optionally having at least one of the following amino acids at the following places within SEQ ID NO: 2 or 3: E at position 9, K at position 13, T as position 14, P at position 23, L at position 27, V at position 31, G at position 54, Q at position 65, V at position 67, Q at position 84, Q at position 88, I at position 102, E at position 123 and/or I at position 143;
- c) comprising at least 70% sequence identity with SEQ ID NO: 1, Q or H at position 65 and H at position 94 and optionally having at least one of the following amino acids at the following places within SEQ ID NO: 1: E at position 9, K at position 13, T as position 14, P at position 23, L at position 27, V at position 31, G at position 54, Q at position 65, V at position 67, Q at position 84, Q at position 88, I at position 102 and/or E at position 123;
- d) comprising at least 70% identity with SEQ ID NO: 4, 5 or 6, Q or H at position 65 and H at position 94 and optionally having at least one of the following amino acids at the following places within SEQ ID NO: 4, 5 or 6: N at position 13, Q at position 27, I at position 31, N at position 67, A at position 128, S at position 133, A at position 145 and/or L at position 150;
- e) comprising at least 70% identity with SEQ ID NO: 7, Q or H at position 65 and H at position 94 and optionally having at least one of the following amino acids at the following places within SEQ ID NO: 7: Q at position 6, Q at position 10, T at position 13, I at position 14, H at position 27, M at position 31, H at position 35, D at position 36, D at position 42, R at position 43, G at position 49, P at position 53, Q at position 55, G at position 58, A at position 67, Q at position 72, K at position 75, Q at position 79, N at position 82, S at position 85, T at position 93, V at position 111, I at position 116, A at position 117, E at position 118, A at position 121, S at position 128, K at position 133, S at position 145 and/or F at position 150; or
- f) comprising at least 70% identity with SEQ ID NO: 8, Q or H at position 65 and H at position 94.

SEQ ID NO: 1 represents part of the amino acid sequence of a woolly mammoth myoglobin, SEQ ID NO: 2 represent the amino acid sequence of a woolly mammoth; SEQ ID NO: 3 represents the amino acid sequence of a steppe mammoth myoglobin, SEQ ID NO: 4 represents the amino acid sequence of a sheep myoglobin, SEQ ID NO: 5 represents the amino acid sequence of a cattle (cow) myoglobin, SEQ ID NO: 6 represents the amino acid sequence of a pig myoglobin, SEQ ID NO: 7 represents the amino acid sequence of a chicken myoglobin and SEQ ID NO: 8 represents the amino acid sequence of a tuna myoglobin. It is clear that SEQ ID NO: 1, representing a part of the amino acid sequence of a woolly mammoth myoglobin (i.e. a partial sequence), is comprised in SEQ ID NO: 2, representing the (full) amino acid sequence of a woolly mammoth myoglobin. SEQ ID NO: 9 is the nucleic acid sequence coding for SEQ ID NO: 1. SEQ ID NO: 10 is the nucleic acid sequence coding for SEQ ID NO: 2, SEQ ID NO: 11 is the nucleic acid sequence coding for SEQ ID NO: 3. SEQ ID NO: 12 is the nucleic acid sequence coding for SEQ ID NO: 4. SEQ ID NO: 13 is the nucleic acid sequence coding for SEQ ID NO: 5. SEQ ID NO: 14 is the nucleic acid sequence coding for SEQ ID NO: 6. SEQ ID NO: 15 is the nucleic acid sequence coding for SEQ ID NO: 7. SEQ ID NO: 16 is the nucleic acid sequence coding for SEQ ID NO: 8.

A meat substitute is per definition not meat, not natural meat, not genuine meat. A meat substitute may be considered as a non-naturally occurring food or edible product. It means that within the context of this invention, a meat substitute is not meat from or derived from pig, sheep, cow, chicken or tuna (or from or derived from mammoth if this animal would still exist which is not the case). A meat substitute is synonymous of a meat replica or a meat analogue product or a meat-like product.

A meat substitute may have the same aspect (such as pattern, lettering), form, structure, composition (such as similar fat, protein and/or heme iron content), texture, color, flavor, aroma and/or appearance as meat.

Alternatively, it might have an aspect, form, structure, texture, color, flavor, aroma and/or appearance which is distinct from the one of meat.

Meat substitutes may be formulated as a liquid, solid, semi-solid food product.

Suitable examples of liquid food product include soup.

Suitable examples of solid food products include a cell-based meat or cultured meat or plant-based meat. Such plant-based meat may comprise plant proteins such as soy proteins. In addition they comprise an animal myoglobin as defined herein. In an embodiment, this animal myoglobin is the sole source of heme-containing protein.

Suitable examples of snacks include a protein bar or a protein shake.

A food ingredient may be any edible ingredient that can be added into an edible product in order to get a distinct edible product. Alternatively, the food ingredient may be consumed as such. The term "food ingredient" may be replaced by the term "food supplement".

Suitable examples of food ingredients comprising the myoglobin identified above may be solid (such as a powder, spice mix) or liquid (such as an extract) or semi-liquid or semi-solid (such as a paste or a gel or a sauce or a broth or a cream) food ingredients that may be sold to customers. Customers may add these food ingredients into their own food to supplement their food with the myoglobin comprised herein to create their own meat substitutes.

Suitable examples of solid food supplement include a tablet or a pill or a powder comprising the animal myoglobin identified above. The tablet or the pill or the powder may be formulated with other compounds such as vitamins or minerals.

The inventors discovered that "meat experience" could be mimicked in a meat substitute of the invention using an animal myoglobin as defined herein.

In an embodiment, a meat substitute of the invention is expected to mimick the aspect (such as pattern, lettering), form, structure, composition (such as similar fat, protein and/or heme iron content), flavor, texture, color, aroma, appearance and/or nutritional value of meat. This is typically the case when the meat substitute is a cell-based meat or cultured meat or plant-based meat. This is mostly due to the presence of the animal myoglobin as described herein. The meat substitute may not have all drawbacks of meat. For example, the meat substitute is healthier than meat. Moreover, the impact of the meat substitute on the environment (long term direct/indirect effect on climate change) is expected to be less detrimental than the known impact of meat.

In an embodiment, a meat substitute of the invention is expected to mimick the aspect (such as pattern, lettering), form, structure, composition (such as similar fat, protein and/or heme iron content), flavor, texture, color, aroma and/or appearance of meat. This is typically the case when the meat substitute is a cell-based meat or cultured meat or plant-based meat. This is mostly due to the presence of the animal myoglobin as described herein. The meat substitute may not have all drawbacks of meat. For example, the meat substitute is healthier than meat. Moreover, the impact of the meat substitute on the environment (long term direct/indirect effect on climate change) is expected to be less detrimental than the known impact of meat.

In an embodiment, a meat substitute of the invention is expected to mimick the composition (such as similar fat, protein and/or heme iron content), flavor, color and/or aroma of meat without having all its drawbacks. This is mostly due to the presence of the animal myoglobin as described herein. In an embodiment, a meat substitute of the invention is expected to mimick the flavor and/or aroma of meat without having all its drawbacks. This is mostly due to the presence of the animal myoglobin as described herein.

In an embodiment, a meat substitute of the invention is expected to mimick the flavor of meat without having all its drawbacks. This is mostly due to the presence of the animal myoglobin as described herein.

In an embodiment, a meat substitute of the invention is expected to mimick the aroma of meat without having all its drawbacks. This is mostly due to the presence of the animal myoglobin as described herein.

The inventors further discovered that "meat experience" could be mimicked with a food ingredient as described herein.

In an embodiment, a food ingredient of the invention is expected to mimick the composition (such as similar fat, protein and/or heme iron content), flavor, color, aroma and/or nutritional value of meat without having all its drawbacks flavor of meat without having all its drawbacks. This is mostly due to the presence of the animal myoglobin as described herein.

In an embodiment, a food ingredient of the invention is expected to mimick the composition (such as similar fat, protein and/or heme iron content), flavor, color and/or aroma of meat without having all its drawbacks flavor of meat without having all its drawbacks. This is mostly due to the presence of the animal myoglobin as described herein.

In an embodiment, a food ingredient of the invention is expected to mimick the flavor, color and/or aroma of meat without having all its drawbacks flavor of meat without having all its drawbacks. This is mostly due to the presence of the animal myoglobin as described herein.

In an embodiment, a food ingredient of the invention is expected to mimick the composition (such as similar fat, protein and/or heme iron content), flavor and/or aroma of meat without having all its drawbacks flavor of meat without having all its drawbacks. This is mostly due to the presence of the animal myoglobin as described herein.

In an embodiment, a food ingredient of the invention is expected to mimick the composition (such as similar fat, protein and/or heme iron content), flavor of meat without having all its drawbacks flavor of meat without having all its drawbacks. This is mostly due to the presence of the animal myoglobin as described herein.

In an embodiment, a food ingredient of the invention is expected to mimick the composition (such as similar fat, protein and/or heme iron content) aroma of meat without having all its drawbacks flavor of meat without having all its drawbacks. This is mostly due to the presence of the animal myoglobin as described herein.

Within the context of the invention, the meat experience may be mimicked when the meat substitute and/or the food ingredient generates an aroma and/or a flavor compound recognizable by humans and characteristic of some odorants and/or flavor compound released when meat is cooked. For example, the formation of some of these odorants may be catalyzed by the iron of heme present in myoglobin. Without being bound to this theory, the formation may be due to lipid oxidation and/or Maillard reactions. This situation may be mimicked with the myoglobin of the meat substitute and/or food ingredient of the invention.

In an embodiment, a meat substitute or a food ingredient of the invention, preferably a raw meat substitute of the invention, is expected to mimick the bloody and/or metallic aroma of meat, preferably of raw meat. Preferably, the meat substitute or food ingredient of the invention is able to mimic the bloody and/or metallic aroma of meat to a higher degree than other meat substitutes or food ingredients (i.e. not according to the invention). In the context of this invention, raw means not subjected to a heat treatment, preferably uncooked and ungrilled.

In an embodiment, a meat substitute or a food ingredient of the invention, preferably a grilled meat substitute of the invention, is expected to mimick the roasted aroma of meat, particularly of grilled meat. Preferably, the meat substitute or food ingredient of the invention is able to mimic the roasted aroma of meat to a higher degree than other meat substitutes or food ingredients (i.e. not according to the invention).

In the context of this invention, and specifically in the embodiments in this section, an "other meat substitute" or "a meat substitute not according to the invention" is preferably a plant-based burger comprising a recombinant soy leghemoglobin (LegH), more preferably the commercially available Impossible Burger.

In an embodiment, a meat substitute or a food ingredient of the invention, preferably a grilled meat substitute of the invention, has an aroma characterized by a higher concentration of oxidized lipids, lipid oxidation products, pyrazines and/or pyrroles in the volatile compounds obtained from the (grilled) meat substitute or food ingredient relative to other meat substitutes or food ingredients (i.e. not according to the invention). Higher preferably means at least 10%, 20%, 30%, 40%, 50%, 60%, 70%, 80%, 90%, 100%, 110%, 120%, 130%, 140%, 150%, 160%, 170%, 180%, 190%, 200%, 210%, 220%, 230%, 240%, 250%, 260%, 270%, 280%, 290%, or 300%. Preferred pyrazines are methylpyrazine, 2,5-dimethylpyrazine, 2-ethyl-6-methylpyrazine. A preferred pyrrole is pyrrole. Preferred lipid oxidation products are 2-methylbutanal and 3-methylbutanal.

In an embodiment, a meat substitute or a food ingredient of the invention, preferably a grilled meat substitute of the invention, has an aroma characterized by a higher concentration of 2,5-dimethylpyrazine in the volatile compounds obtained from the (grilled) meat substitute or food ingredient relative to other meat substitutes or food ingredients (i.e. not according to the invention). Higher preferably means at least 10%, 20%, 30%, 40%, 50%, 60%, 70%, 80%, 90%, 100%, 110%, 120%, 130%, 140%, 150%, 160%, 170%, 180%, 190%, 200%, 210%, 220%, 230%, 240%, 250%, 260%, 270%, 280%, 290%, or 300%.

In an embodiment, a meat substitute or a food ingredient of the invention, preferably a grilled meat substitute of the invention, has an aroma characterized by a higher concentration of 2-ethyl-6-methylpyrazine in the volatile compounds obtained from the (grilled) meat substitute or food ingredient relative to other meat substitutes or food ingredients (i.e. not according to the invention). Higher preferably means at least 10%, 20%, 30%, 40%, 50%, 60%, 70%, 80%, 90%, 100%, 110%, 120%, 130%, 140%, 150%, 160%, 170%, 180%, 190%, 200%, 210%, 220%, 230%, 240%, 250%, 260%, 270%, 280%, 290%, or 300%.

In an embodiment, a meat substitute or a food ingredient of the invention, preferably a grilled meat substitute of the invention, has an aroma characterized by a higher concentration of pyrrole in the volatile compounds obtained from the (grilled) meat substitute or food ingredient relative to other meat substitutes or food ingredients (i.e. not according to the invention). Higher preferably means at least 10%, 20%, 30%, 40%, 50%, 60%, 70%, 80%, 90%, 100%, 110%, 120%, 130%, 140%, 150%, 160%, 170%, 180%, 190%, 200%, 210%, 220%, 230%, 240%, 250%, 260%, 270%, 280%, 290%, or 300%.

In an embodiment, a meat substitute or a food ingredient of the invention, preferably a grilled meat substitute of the invention, has an aroma characterized by a higher concentration of 2-methylbutanal in the volatile compounds obtained from the (grilled) meat substitute or food ingredient relative to other meat substitutes or food ingredients (i.e. not according to the invention). Higher preferably means at least 10%, 20%, 30%, 40%, 50%, 60%, 70%, 80%, 90%, 100%, 110%, 120%, 130%, 140%, 150%, 160%, 170%, 180%, 190%, 200%, 210%, 220%, 230%, 240%, 250%, 260%, 270%, 280%, 290%, or 300%.

In an embodiment, a meat substitute or a food ingredient of the invention, preferably a grilled meat substitute of the invention, has an aroma characterized by a higher concentration of 3-methylbutanal in the volatile compounds obtained from the (grilled) meat substitute or food ingredient relative to other meat substitutes or food ingredients (i.e. not according to the invention). Higher preferably means at least 10%, 20%, 30%, 40%, 50%, 60%, 70%, 80%, 90%, 100%, 110%, 120%, 130%, 140%, 150%, 160%, 170%, 180%, 190%, 200%, 210%, 220%, 230%, 240%, 250%, 260%, 270%, 280%, 290%, or 300%.

In Example 2.5, an aromatic analysis of a meat substitute according to the invention is provided. The aroma of meat and meat substitutes, and the concentration in the volatile compounds, may be determined by the methods described in this example, i.e. by gas chromatography coupled with mass spectrometry of volatile compounds.

Within the context of the invention, the meat experience may be mimicked when the color of the meat substitute is similar with the color of meat. The color of meat is determined by the concentration of heme-containing protein and/or by its oxidation state in the meat. Therefore, the myoglobin as defined herein will define the color of the meat substitute. The same holds for the food ingredient as invented herein.

In an embodiment, a meat substitute or a food ingredient of the invention, preferably a raw meat substitute of the invention, is expected to mimic the color of a meat, preferably of raw meat. Without being bound to this theory, the color of the meat substitute is mostly due to the presence of the animal myoglobin. Preferably, the meat substitute or food ingredient of the invention is able to mimic the color of meat to a higher degree than other meat substitutes or food ingredients (i.e. not according to the invention).

In a preferred embodiment, the color of a meat substitute or a food ingredient of the invention, preferably of a raw meat substitute of the invention, is essentially stable for at least 1, 2, 3, 4, 5, 6, 7, 8, 9, 10, 11, 12, 13, 14, 15, 16, 17, 18, 19 or 20 days, preferably upon storage under constant light at 4°C, more preferably under the conditions and measured via the techniques of Example 2.4. Preferably, the color of the meat substitute or food ingredient of the invention is stable for 1, 2, 3, 4, 5, 6, 7, 8, 9, 10, 11, 12, 13, 14, 15, 16, 17, 18, 19 or 20 days longer than the time period wherein the color of meat or other meat substitutes or food ingredients (i.e. not according to the invention) is stable, more preferably wherein a $\Delta E$ change from 0% up to 10% is defined as stable (see further). Essentially stable for at least X days preferably means a change of $\Delta E$ of the meat substitute or food ingredient between 0% and 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, 10%, 10.5%, 11%, 11.5%, 12%, 12.5%, 13%, 13.5%, 14%, 14.5%, 15%, 15.5%, 16%, 16.5%, 17%, 17.5%, 18%, 18.5%, 19%, 19.5%, or 20% between day 0 and day X, wherein $\Delta E$ is as determined in Example 2.4. Most preferably, $\Delta E$ of the meat substitute or food ingredient does not change by more than 10% upon storage for at least 1, 2, 3, 4, 5, 6, 7, 8, 9, 10, 11, 12, 13, 14, 15, 16, 17, 18, 19 or 20 days.

Example 2.4 provides color stability analyses of a meat substitute according to the invention. The color and color stability of meat and meat substitutes may be determined by the methods described in this example.

Within the context of the invention, the meat experience may be mimicked when the composition of the meat substitute is similar with meat composition. The composition of the meat substitute may be seen to be similar to the meat composition when the same or similar components are present and optionally when the quantities in which they are present is the same or similar with the quantities present in meat. For example a similar fat, protein and/or heem iron content may be present in a meat substitute as in meat.

These animal myoglobins when formulated into a meat substitute or as a food ingredient are also expected to provide roughly the same amount of total available iron or heme iron as meat. Within this context, "roughly" means at least 80%, 81%, 82%, 83%, 84%, 85%, 86%, 87%, 88%, 89%, 90%, 91%, 92%, 93%, 94%, 95%, 96%, 97%, 98%, 99%, 100% or more. Usually the amount of total bioavailable iron or heme iron in the meat substitute of the invention is not more than 120% of the total bioavailable iron or heme iron in genuine meat. It means these animal myoglobins may be considered as a unique alternative fortificant to improve the iron status of the population.

These animal myoglobins are also expected to provide roughly a higher amount of total bioavailable iron or heme iron than the amount of total bioavailable iron or heme iron that is provided in a plant-based meat analogue product. Within this context, "higher" means at least 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30% or more.

In a preferred definition, nutritional value means the amount of total available iron or heme iron. According to this preferred definition, "mimicking the nutritional value of meat" should be interpreted as "provide roughly the same amount of total available iron or heme iron as meat", as outlined above.

Example 2.6 provides a nutritional analysis of a meat substitute according to the invention, assessing the bioavailability of iron.

Within the context of the invention, total iron is the total amount of iron which is bioavailable in a meat substitute. Heme iron is the part of iron which is transported by a protein comprising a heme domain. Heme iron is available for absorption by the human body. Such a protein is myoglobin as defined herein. Availability of iron may be assessed as described in Proulx A.K., et al (2006)., J. Agric. Food. Chem., (54), 1518-1522) and/or the methods in Example 2.6. Briefly the ferritin concentration of a preparation is first normalized to cell protein concentration. Subsequently, the percentages of normalized ferritin is compared to the percentage of FeSO4 and expressed as relative biological values (RBV).

In an embodiment, the meat substitute does not comprise a leghemoglobin which has been produced by a bacterium living in symbiosis in the root nodules of a soy plant and/or comprises as sole heme-containing protein the animal myoglobin as earlier defined herein.

In an embodiment, the animal myoglobin disclosed herein may be the sole source of heme-containing protein present in the meat substitute of the invention. It means the meat substitute of the invention may comprise other proteins than the animal myoglobin as disclosed herein. Examples of proteins that may be present include soy proteins.

Within the context of the invention, a heme-containing protein as defined can refer to all proteins or protein subunits that are capable of covalently or noncovalently binding a heme moiety. Heme-containing polypeptides can transport or store oxygen. Some examples of heme-containing protein include globin, hemoglobin, leghemoglobin.

In another embodiment, the meat substitute does not comprise a leghemoglobin which has been produced by a bacterium living in symbiosis in the root nodules of a soy plant. In an embodiment, the meat substitute does not comprise a symbiotic hemoglobin. In an embodiment, the meat substitute does not comprise a leghemoglobin. In an embodiment, the meat substitute does not comprise a protein which has been produced by a bacterium living in symbiosis in the root nodules of a soy plant.

In an embodiment, the meat substitute does not comprise a leghemoglobin which has been produced by a bacterium living in symbiosis in the root nodules of a soy plant and comprises as sole heme-containing protein the animal myoglobin as earlier defined herein.

In an embodiment, the meat substitute comprises a similar amount of animal myoglobin as the counterpart genuine meat. In an embodiment such amount is ranged from 0.1 to 5 weight % animal myoglobin. In an embodiment, the amount is at least 0.1, 0.2, 0.3, 0.4, 0.5. 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 4.0, 4.5, 5.0 or more weight % animal myoglobin. It is also encompassed by the invention to have amounts of at least 10%, 20%, 30%, 40%, 50%, of the final weight of the product. The skilled person knows that depending on the meat substitute the amount may vary.

In an embodiment, the weight percentage of the myoglobin in the meat substitute according to the invention is from 0.1% up to 5%, 4.9%, 4.8%, 4.7%, 4.6%, 4.5%, 4.4%, 4.3%, 4.2%, 4.1%, 4%, 3.9%, 3.8%, 3.7%, 3.6%, 3.5%, 3.4%, 3.3%, 3.2%, 3.1%, 3%, 2.95%, 2.9%, 2.85%, 2.8%, 2.75%, 2.7%, 2.65%, 2.6%, 2.55%, 2.5%, 2.45%, 2.4%, 2.35%, 2.3%, 2.25%, 2.2%, 2.15%, 2.1%, 2.05%, 2%, 1.95%, 1.9%, 1.85%, 1.8%, 1.75%, 1.7%, 1.65%, 1.6%, 1.55%, 1.5%, 1.45%, 1.4%, 1.35%, 1.3%, 1.25%, 1.2%, 1.15%, 1.1%, 1.05%, 1%, 0.95%, 0.9%, 0.85%, 0.8%, 0.75%, 0.7%, 0.65%, 0.6%, 0.55%, or 0.5%.

In an embodiment, the weight percentage of the myoglobin in the meat substitute according to the invention is from 0.5% up to 5%, 4.9%, 4.8%, 4.7%, 4.6%, 4.5%, 4.4%, 4.3%, 4.2%, 4.1%, 4%, 3.9%, 3.8%, 3.7%, 3.6%, 3.5%, 3.4%, 3.3%, 3.2%, 3.1%, 3%, 2.95%, 2.9%, 2.85%, 2.8%, 2.75%, 2.7%, 2.65%, 2.6%, 2.55%, 2.5%, 2.45%, 2.4%, 2.35%, 2.3%, 2.25%, 2.2%, 2.15%, 2.1%, 2.05%, 2%, 1.95%, 1.9%, 1.85%, 1.8%, 1.75%, 1.7%, 1.65%, 1.6%, 1.55%, 1.5%, 1.45%, 1.4%, 1.35%, 1.3%, 1.25%, 1.2%, 1.15%, 1.1%, 1.05%, 1%, 0.95%, 0.9%, 0.85%, 0.8%, 0.75%, 0.7%, 0.65%, or 0.6.

In an embodiment, the food ingredient comprises an amount of animal myoglobin that may be ranged from 0.1 to 100 weight % animal myoglobin. In an embodiment, the amount is at least 0.1%, 1.0%, 1.5%, 2.0%, 2.5%, 3.0%, 4.0%, 4.5%, 5.0%, 10%, 20%, 30%, 40%, 50%, 60%, 70%, 80%, 90%, 95%, 100% of the final weight of the product. The skilled person knows that depending on the food ingredient the amount may vary.

In an embodiment, the meat substitute comprises or is prepared by mixing from 10% up to 40% of a protein source, from 5% up to 30% of a lipid source, from 0.1% up to 5% of NaCl, from 0.1% up to 5% of a fiber source, and from 0.1% up to 5% of a myoglobin according to the invention. In a more preferred embodiment, the meat substitute comprises or is prepared by mixing from 20% up to 30% of a protein source, from 10% up to 20% of a lipid source, from 0.5% up to 2% of NaCl, from 0.5% up to 1.5% of a fiber source, and from 0.1% up to 3% of a myoglobin according to the invention.

Preferably, the protein source is texturized soy protein, pea protein isolate, mung bean protein, textured wheat protein, rice protein or potato protein. In this context, texturized soy protein is a defatted soy flour product, also known as texturized vegetable protein (TVP) or soy meat, as is clear to the skilled person.

Preferably, the lipid source is sunflower oil, coconut oil, canola oil or cocoa butter.

Preferably, the fiber source is methylcellulose or potato starch.

In a more preferred embodiment, the meat substitute comprises or is prepared by mixing from 20% up to 30% of texturized soy protein, from 10% up to 20% of sunflower oil, from 0.5% up to 2% of NaCl, from 0.5% up to 1.5% of methylcellulose, and from 0.1% up to 10% of a myoglobin according to the invention. Even more preferably, the weight percentage of the myoglobin in the meat substitute according to the more preferred embodiment is from 0.1% up to 5%, 4.9%, 4.8%, 4.7%, 4.6%, 4.5%, 4.4%, 4.3%, 4.2%, 4.1%, 4%, 3.9%, 3.8%, 3.7%, 3.6%, 3.5%, 3.4%, 3.3%, 3.2%, 3.1%, 3%, 2.95%, 2.9%, 2.85%, 2.8%, 2.75%, 2.7%, 2.65%, 2.6%, 2.55%, 2.5%, 2.45%, 2.4%, 2.35%, 2.3%, 2.25%, 2.2%, 2.15%, 2.1%, 2.05%, 2%, 1.95%, 1.9%, 1.85%, 1.8%, 1.75%, 1.7%, 1.65%, 1.6%, 1.55%, 1.5%, 1.45%, 1.4%, 1.35%, 1.3%, 1.25%, 1.2%, 1.15%, 1.1%, 1.05%, 1%, 0.95%, 0.9%, 0.85%, 0.8%, 0.75%, 0.7%, 0.65%, 0.6%, 0.55%, or 0.5%.

In an embodiment, the meat substitute comprises or is prepared by mixing from 20% up to 30% texturized soy protein, from 10% up to 20% sunflower oil, from 1% up to 2% NaCl, from 0.5% up to 1.5% methylcellulose, from 50% up to 70% water and from 0.2% up to 0.8% myoglobin.

In a preferred embodiment, the meat substitute comprises or is prepared by mixing from 23% up to 27% texturized soy protein, from 13% up to 17% sunflower oil, from 1.25% up to 1.75% NaCl, from 0.75% up to 1.25% methylcellulose, from 55% up to 60% water and from 0.45% up to 0.55% myoglobin.

In an embodiment, the meat substitute comprises or is prepared by mixing 25% texturized soy protein, 15% sunflower oil, 1.5% NaCl, 1.0% methylcellulose, 57% water and 0.5% myoglobin, wherein these weight percentages are rounded to two significant digits.

In an embodiment, the meat substitute comprises or is prepared by mixing from 15% up to 35% texturized soy protein, from 10% up to 20% sunflower oil, from 0.5% up to 2.5% NaCl, from 0.5% up to 1.5% methylcellulose, from 50% up to 70% water and from 0.5% up to 1.5% myoglobin.

In a preferred embodiment, the meat substitute comprises or is prepared by mixing from 22% up to 27% texturized soy protein, from 13% up to 17% sunflower oil, from 1.25% up to 1.75% NaCl, from 0.75% up to 1.25% methylcellulose, from 50% up to 60% water and from 0.75% up to 1.25% myoglobin.

In an embodiment, the meat substitute comprises or is prepared by mixing 25% texturized soy protein, 15% sunflower oil, 1.5% NaCl, 1.0% methylcellulose, 57% water and 1.0% myoglobin, wherein these weight percentages are rounded to two significant digits.

In the meat substitutes above, % refers to a weight percentage. Insofar as the weight percentages describing the composition of a meat substitute do not add up to 100%, the addition of water to a corresponding percentage is assumed.

Example 1.4 provides an example of the preparation of a meat substitute according to the invention.

In an embodiment, the meat substitute comprises a similar amount of bioavailable iron or heme iron as the counterpart genuine meat. In an embodiment such amount is ranged from 0.3 to 20 mg bioavailable iron or heme iron per 100 gram meat substitute. In an embodiment such amount is ranged from 0.1 mg up to 16.5 mg, 16.17 mg, 15.84 mg, 15.51 mg, 15.18 mg, 14.85 mg, 14.52 mg, 14.19 mg, 13.86 mg, 13.53 mg, 13.2 mg, 12.87 mg, 12.54 mg, 12.21 mg, 11.88 mg, 11.55 mg, 11.22 mg, 10.89 mg, 10.56 mg, 10.23 mg, 9.9 mg, 9.735 mg, 9.57 mg, 9.405 mg, 9.24 mg, 9.075 mg, 8.91 mg, 8.745 mg, 8.58 mg, 8.415 mg, 8.25 mg, 8.085 mg, 7.92 mg, 7.755 mg, 7.59 mg, 7.425 mg, 7.26 mg, 7.095 mg, 6.93 mg, 6.765 mg, 6.6 mg, 6.435 mg, 6.27 mg, 6.105 mg, 5.94 mg, 5.775 mg, 5.61 mg, 5.445 mg, 5.28 mg, 5.115 mg, 4.95 mg, 4.785 mg, 4.62 mg, 4.455 mg, 4.29 mg, 4.125 mg, 3.96 mg, 3.795 mg, 3.63 mg, 3.465 mg, 3.3 mg, 3.135 mg, 2.97 mg, 2.805 mg, 2.64 mg, 2.475 mg, 2.31 mg, 2.145 mg, 1.98 mg, 1.815 mg or 1.65 mg bioavailable iron or heme iron per 100 gram meat substitute. In another embodiment such amount is 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, 10, 10.1, 10.2, 10.3, 10.4, 10.5, 10.6, 10.7, 10.8, 10.9, 11, 11.1, 11.2, 11.3, 11.4, 11.5, 11.6, 11.7, 11.8, 11.9, 12, 12.1, 12.2, 12.3, 12.4, 12.5, 12.6, 12.7, 12.8, 12.9, 13, 13.1, 13.2, 13.3, 13.4, 13.5, 13.6, 13.7, 13.8, 13.9, 14, 14.1, 14.2, 14.3, 14.4, 14.5, 14.6, 14.7, 14.8, 14.9, 15, 15.1, 15.2, 15.3, 15.4, 15.5, 15.6, 15.7, 15.8, 15.9, 16, 16.1, 16.2, 16.3, 16.4, 16.5, 16.6, 16.7, 16.8, 16.9, 17, 17.1, 17.2, 17.3, 17.4, 17.5, 17.6, 17.7, 17.8, 17.9, 18, 18.1, 18.2, 18.3, 18.4, 18.5, 18.6, 18.7, 18.8, 18.9, 19, 19.1, 19.2, 19.3, 19.4, 19.5, 19.6, 19.7, 19.8, 19.9, or 20 mg bioavailable iron or heme iron per 100 gram meat substitute.

In an embodiment, the amino acid sequence of the animal myoglobin identified earlier herein comprises a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with the sequences identified earlier herein in combination with at least one of the following amino acid combinations:

- F at position 30, and/or
- Q at position 65, and/or
- H at position 92, and/or
- H at position 94, and/or
- F at position 30 and Q at position 65, and/or
- F at position 30 and H at position 92, and/or
- F at position 30 and H at position 94, and/or
- Q at position 65 and H at position 92, and/or
- Q at position 65 and H at position 94, and/or
- H at position 92 and H at position 94, and/or
- F at position 30 and Q at position 65 and H at position 92, and/or
- F at position 30 and Q at position 65 and H at position 94, and/or
- F at position 30 and H at position 92 and H at position 94, and/or
- Q at position 65 and H at position 92 and H at position 94, and/or
- F at position 30 and Q at position 65 and H at position 92 and H at position 94.

In an embodiment, the amino acid sequence of the animal myoglobin identified earlier herein comprises a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with the sequences identified earlier herein in combination with Q or H at position 65 and H at position 94 for each of the sequences identified, preferably further in combination with I at position 143.

In an embodiment, the amino acid sequence of the animal myoglobin identified earlier herein comprises a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with the sequences identified earlier herein in combination with Q or H at position 65, Q at position 92 and H at position 94 for each of the sequences identified, preferably further in combination with I at position 143.

In an embodiment, the amino acid sequence of the animal myoglobin identified earlier herein comprises a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with the sequences identified earlier herein in combination with Q or H at position 65, H at position 92 and H at position 94 for each of the sequences identified, preferably further in combination with I at position 143.

In an embodiment, the amino acid sequence of the animal myoglobin identified earlier herein comprises a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with the sequences identified earlier herein in combination with H at position 92 for each of the sequences identified, preferably further in combination with I at position 143.

In an embodiment, a meat substitute or a food ingredient comprising a myoglobin that is represented by an amino acid sequence comprising at least 70% sequence identity with SEQ ID NO: 1 in combination with Q or H at position 65 and H at position 94 and optionally having at least one of the following amino acids at the following places within SEQ ID NO: 1: E at position 9, K at position 13, T as position 14, P at position 23, L at position 27, V at position 31, G at position 54, Q at position 65, V at position 67, Q at position 84, Q at position 88, I at position 102 and/or E at position 123. In an embodiment, a meat substitute or a food ingredient comprising a myoglobin that is represented by an amino acid sequence comprising at least 70% sequence identity with SEQ ID NO: 2 or 3 in combination with Q or H at position 65 and H at position 94 and optionally having at least one of the following amino acids at the following places within SEQ ID NO: 2 or 3: E at position 9, K at position 13, T as position 14, P at position 23, L at position 27, V at position 31, G at position 54, Q at position 65, V at position 67, Q at position 84, Q at position 88, I at position 102, E at position 123 and/or I at position 143. Preferably, the amino acid sequence of the animal myoglobin identified comprises a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 1, 2 or 3 in combination with Q or H at position 65 and H at position 94. SEQ ID NO: 1 is part of a myoblobin from a mammoth. SEQ ID NO: 2 and 3 are myoglobins from mammoths. In an embodiment, the meat substitute comprises a myoglobin comprising the amino acid sequence SEQ ID NO: 1. In an embodiment, the meat substitute comprises a mammoth myoglobin represented by amino acid sequence SEQ ID NO: 2 or 3. It is expected that such a myoglobin has attractive properties as this myoglobin is stable against oxidation especially at low pH. It is expected that the color of the meat substitute of the invention is stable as mammoth myoglobin resists oxidation. This stability against oxidation also provides additional health properties to the meat substitute of the invention as it will be less likely converted into a carcinogenic product than genuine meat.

In an embodiment, a meat substitute or a food ingredient comprising a myoglobin that is represented by an amino acid sequence comprising at least 70% sequence identity with SEQ ID NO: 2 in combination with Q or H at position 65, Q at position 92 and H at position 94 and optionally having at least one of the following amino acids at the following places within SEQ ID NO: 2: E at position 9, K at position 13, T as position 14, P at position 23, L at position 27, V at position 31, G at position 54, Q at position 65, V at position 67, Q at position 84, Q at position 88, I at position 102 and/or E at position 123. Preferably, the amino acid sequence of the animal myoglobin identified comprises a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 2 in combination with Q or H at position 65, Q at position 92 and H at position 94. SEQ ID NO: 2 is a myoglobin from woolly mammoth. In an embodiment, the meat substitute comprises a woolly mammoth myoglobin represented by amino acid sequence SEQ ID NO: 2. It is expected that this myoglobin has attractive properties as this myoglobin is stable against oxidation especially at low pH. It is expected that the color of the meat substitute of the invention is stable as woolly mammoth myoglobin resists oxidation. This stability against oxidation also provides additional health properties to the meat substitute of the invention as it will be less likely converted into a carcinogenic product than genuine meat.

In an embodiment, a meat substitute or a food ingredient comprising a myoglobin that is represented by an amino acid sequence comprising at least 70% sequence identity with SEQ ID NO: 3 in combination with Q or H at position 65, H at position 92 and H at position 94 and optionally having at least one of the following amino acids at the following places within SEQ ID NO: 3: E at position 9, K at position 13, T as position 14, P at position 23, L at position 27, V at position 31, G at position 54, Q at position 65, V at position 67, Q at position 84, Q at position 88, I at position 102 and/or E at position 123. Preferably, the amino acid sequence of the animal myoglobin identified comprises a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 3 in combination with Q or H at position 65, H at position 92 and H at position 94. SEQ ID NO: 3 is a myoglobin from steppe mammoth. In an embodiment, the meat substitute comprises a steppe mammoth myoglobin represented by amino acid sequence SEQ ID NO: 3. It is expected that this myoglobin has attractive properties as this myoglobin is stable against oxidation especially at low pH. It is expected that the color of the meat substitute of the invention is stable as steppe mammoth myoglobin resists oxidation. This stability against oxidation also provides additional health properties to the meat substitute of the invention as it will be less likely converted into a carcinogenic product than genuine meat.

In an embodiment, a meat substitute or a food ingredient comprising a myoglobin that is represented by an amino acid sequence comprising at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identity or similarity with SEQ ID NO: 3 in combination with F at position 30.

In an embodiment, a meat substitute or a food ingredient comprising a myoglobin that is represented by an amino acid sequence comprising at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identity or similarity with SEQ ID NO: 3 in combination with Q at position 65.

In an embodiment, a meat substitute or a food ingredient comprising a myoglobin that is represented by an amino acid sequence comprising at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identity or similarity with SEQ ID NO: 3 in combination with H at position 92.

In an embodiment, a meat substitute or a food ingredient comprising a myoglobin that is represented by an amino acid sequence comprising at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identity or similarity with SEQ ID NO: 3 in combination with H at position 94.

In an embodiment, a meat substitute or a food ingredient comprising a myoglobin that is represented by an amino acid sequence comprising at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identity or similarity with SEQ ID NO: 3 in combination with F at position 30 and Q at position 65.

In an embodiment, a meat substitute or a food ingredient comprising a myoglobin that is represented by an amino acid sequence comprising at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identity or similarity with SEQ ID NO: 3 in combination with F at position 30 and H at position 92.

In an embodiment, a meat substitute or a food ingredient comprising a myoglobin that is represented by an amino acid sequence comprising at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identity or similarity with SEQ ID NO: 3 in combination with F at position 30 and H at position 94.

In an embodiment, a meat substitute or a food ingredient comprising a myoglobin that is represented by an amino acid sequence comprising at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identity or similarity with SEQ ID NO: 3 in combination with Q at position 65 and H at position 92.

In an embodiment, a meat substitute or a food ingredient comprising a myoglobin that is represented by an amino acid sequence comprising at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identity or similarity with SEQ ID NO: 3 in combination with Q at position 65 and H at position 94.

In an embodiment, a meat substitute or a food ingredient comprising a myoglobin that is represented by an amino acid sequence comprising at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identity or similarity with SEQ ID NO: 3 in combination with H at position 92 and H at position 94.

In an embodiment, a meat substitute or a food ingredient comprising a myoglobin that is represented by an amino acid sequence comprising at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identity or similarity with SEQ ID NO: 3 in combination with F at position 30 and Q at position 65 and H at position 92.

In an embodiment, a meat substitute or a food ingredient comprising a myoglobin that is represented by an amino acid sequence comprising at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identity or similarity with SEQ ID NO: 3 in combination with F at position 30 and Q at position 65 and H at position 94.

In an embodiment, a meat substitute or a food ingredient comprising a myoglobin that is represented by an amino acid sequence comprising at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identity or similarity with SEQ ID NO: 3 in combination with F at position 30 and H at position 92 and H at position 94.

In an embodiment, a meat substitute or a food ingredient comprising a myoglobin that is represented by an amino acid sequence comprising at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identity or similarity with SEQ ID NO: 3 in combination with Q at position 65 and H at position 92 and H at position 94.

In an embodiment, a meat substitute or a food ingredient comprising a myoglobin that is represented by an amino acid sequence comprising at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identity or similarity with SEQ ID NO: 3 in combination with F at position 30 and Q at position 65 and H at position 92 and H at position 94.

In an embodiment, the meat substitute comprises a steppe mammoth myoglobin represented by amino acid sequence SEQ ID NO: 3. It is expected that such a myoglobin has attractive properties as this myoglobin is stable against oxidation especially at low pH. It is expected that the color of the meat substitute of the invention is stable as mammoth myoglobin resists oxidation. This stability against oxidation also provides additional health properties to the meat substitute of the invention as it will be less likely converted into a carcinogenic product than genuine meat and/or than meat substituted not comprising a steppe mammoth myoglobin represented by amino acid sequence SEQ ID NO: 3. Example 2.4 provides color analyses of a steppe mammoth myoglobin according to the invention and of a meat substitute according to the invention comprising a steppe mammoth myoglobin. Moreover, Example 2.5 provides an aromatic analysis of the meat substitute, while Example 2.6 provides a nutritional analysis of the myoglobin, assessing the bioavailability of iron.

In an embodiment, the meat substitute does not comprise one or more milk proteins selected from the group consisting of β-casein, κ-casein, α-S1-casein, α-S2-casein, α-lactalbumin, β-lactoglobulin, lactoferrin and transferrin, wherein said milk protein is from a woolly mammoth. In another embodiment, the meat substitute does not comprise one or more milk proteins selected from the group consisting of β-casein, κ-casein, α-S1-casein, α-S2-casein, α-lactalbumin, β-lactoglobulin, lactoferrin and transferrin, wherein said milk protein is from a mammoth.

In an embodiment, the meat substitute does not comprise one or more milk proteins selected from the group consisting of β-casein, κ-casein, α-S1-casein, α-S2-casein, α-lactalbumin, β-lactoglobulin, lactoferrin and transferrin, wherein said milk protein has not been produced by a mammal or a mammalian cell.

In a more preferred embodiment, the meat substitute does not comprise a milk protein such as β-casein, κ-casein, α-S1-casein, α-S2-casein, α-lactalbumin, β-lactoglobulin, lactoferrin or transferrin, wherein said milk protein is from a woolly mammoth. In another more preferred embodiment, the meat substitute does not comprise a milk protein such as β-casein, κ-casein, α-S1-casein, α-S2-casein, α-lactalbumin, β-lactoglobulin, lactoferrin or transferrin, wherein said milk protein is from a mammoth.

In another more preferred embodiment, the does not comprise a milk protein such as β-casein, κ-casein, α-S1-casein, α-S2-casein, α-lactalbumin, β-lactoglobulin, lactoferrin or transferrin, wherein said milk protein has not been produced by a mammal or a mammalian cell.

In an embodiment, the meat substitute does not comprise a κ-casein from a woolly mammoth. In a further embodiment, the meat substitute does not comprise a protein represented by a sequence having at least 90%, 91%, 92%, 93%, 94%, 95%, 96%, 97%, 98%, 99% sequence identity with SEQ ID NO: 17. In a further embodiment, the meat substitute does not comprise a protein represented by SEQ ID NO: 17.

In an embodiment, the meat substitute does not comprise a β-casein from a woolly mammoth. In a further embodiment, the meat substitute does not comprise a protein represented by a sequence having at least 90%, 91%, 92%, 93%, 94%, 95%, 96%, 97%, 98%, 99% sequence identity with SEQ ID NO: 18. In a further embodiment, the meat substitute does not comprise a protein represented by SEQ ID NO: 18.

<u>Meat</u>

In a second aspect there is provided a meat product comprising an animal myoglobin, preferably a mammalian myoglobin, more preferably from mammoth, pig, sheep or cow, more preferably wherein said mammoth is a woolly mammoth (*Mammuthus primigenius*) or a steppe mammoth (*Mammuthus trogontherii*), preferably steppe mammoth, or avian myoglobin, preferably from chicken, or a fish myoglobin, preferably from tuna, or a myoglobin derived therefrom; wherein said animal myoglobin was not produced by the animal, or any cells comprised therein, from which said meat product is derived. In other words, the meat product is derived from an animal, but comprises a myoglobin not produced therein.

In an embodiment is provided a meat product comprising an exogenous myoglobin, preferably an exogenous animal myoglobin, more preferably from mammoth, pig, sheep or cow, more preferably wherein said mammoth is a woolly mammoth (*Mammuthus primigenius*) or a steppe mammoth (*Mammuthus trogontherii*), preferably steppe mammoth, or avian myoglobin, preferably from chicken, or a fish myoglobin, preferably from tuna. Herein, an exogenous myoglobin is defined as a myoglobin which is not endogenously present in said meat.

In an embodiment, the myoglobin in the meat product in the embodiments above is represented by one of the following amino acid sequences:

- a) comprising at least 70% sequence identity with SEQ ID NO: 3, and having at least one of the following amino acid combinations:

  ○ F at position 30, and/or
  ○ Q at position 65, and/or
  ○ H at position 92, and/or
  ○ H at position 94, and/or
  ○ F at position 30 and Q at position 65, and/or
  ○ F at position 30 and H at position 92, and/or
  ○ F at position 30 and H at position 94, and/or
  ○ Q at position 65 and H at position 92, and/or
  ○ Q at position 65 and H at position 94, and/or
  ○ H at position 92 and H at position 94, and/or
  ○ F at position 30 and Q at position 65 and H at position 92, and/or
  ○ F at position 30 and Q at position 65 and H at position 94, and/or
  ○ F at position 30 and H at position 92 and H at position 94, and/or
  ○ Q at position 65 and H at position 92 and H at position 94, and/or
  ○ F at position 30 and Q at position 65 and H at position 92 and H at position 94;;

- b) comprising at least 70% sequence identity with SEQ ID NO: 2 or 3, Q or H at position 65 and H at position 94 and optionally having at least one of the following amino acids at the following places within SEQ ID NO: 2 or 3: E at position 9, K at position 13, T as position 14, P at position 23, L at position 27, V at position 31, G at position 54, Q at position 65, V at position 67, Q at position 84, Q at position 88, I at position 102, E at position 123 and/or I at position 143;
- c) comprising at least 70% sequence identity with SEQ ID NO: 1, Q or H at position 65 and H at position 94 and optionally having at least one of the following amino acids at the following places within SEQ ID NO: 1: E at position 9, K at position 13, T as position 14, P at position 23, L at position 27, V at position 31, G at position 54, Q at position 65, V at position 67, Q at position 84, Q at position 88, I at position 102 and/or E at position 123;
- d) comprising at least 70% identity with SEQ ID NO: 4, 5 or 6, Q or H at position 65 and H at position 94 and optionally having at least one of the following amino acids at the following places within SEQ ID NO: 4, 5 or 6: N at position 13, Q at position 27, I at position 31, N at position 67, A at position 128, S at position 133, A at position 145 and/or L at position 150;
- e) comprising at least 70% identity with SEQ ID NO: 7, Q or H at position 65 and H at position 94 and optionally having at least one of the following amino acids at the following places within SEQ ID NO: 7: Q at position 6, Q at position 10, T at position 13, I at position 14, H at position 27, M at position 31, H at position 35, D at position 36, D at position 42, R at position 43, G at position 49, P at position 53, Q at position 55, G at position 58, A at position 67, Q at position 72, K at position 75, Q at position 79, N at position 82, S at position 85, T at position 93, V at position 111, I at position 116, A at position 117, E at position 118, A at position 121, S at position 128, K at position 133, S at position 145 and/or F at position 150; or
- f) comprising at least 70% identity with SEQ ID NO: 8, Q or H at position 65 and H at position 94.

In an embodiment is provided a meat product comprising myoglobin, wherein the concentration of myoglobin in said meat product is higher than the average concentration of myoglobin in meat from which said meat product is derived. Herein, the concentration of myoglobin should be interpreted as the total concentration of all myoglobin variants. The average concentration of myoglobin in meat from which said meat product is derived means the average concentration, determined over several corresponding animals, of myoglobin in said meat, wherein no exogeneous myoglobin has been added after harvesting.

Myoglobin

In a third aspect, the invention provides a protein which may be represented by a sequence comprising SEQ ID NO: 3.

In a preferred embodiment is provided a protein which may be represented by a sequence comprising SEQ ID NO: 3, wherein said sequence has a length of 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, or 300 amino acid residues.

In a preferred embodiment is provided a protein which may be represented by a sequence consisting of SEQ ID NO: 3 and a terminal sequence at the N-terminal of SEQ ID NO: 3. Preferably, said terminal sequence has a length of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 amino acid residues.

In an alternative embodiment is provided a protein which may be represented by a sequence consisting of SEQ ID NO: 3 and a terminal sequence at the C-terminal of SEQ ID NO: 3. Preferably, said terminal sequence has a length of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 amino acid residues.

In a preferred embodiment is provided a protein which may be represented by a sequence comprising or consisting of SEQ ID NO: 3 and a tag, preferably wherein said tag is a poly-histidine tag.

In a preferred embodiment, the invention provides a myoglobin, wherein said myoglobin is from steppe mammoth, preferably wherein said myoglobin can be represented by SEQ ID NO: 3. The myoglobin may be for use in a meat substitute, food ingredient or meat product as described above.

In Example 2.2, the structural characteristics of a myoglobin represented by SEQ ID NO: 3 are investigated. Specifically, it is shown that SEQ ID NO: 3 surprisingly corresponds to a higher positive surface charge compared to other myoglobins. Without being bound to this theory, a myoglobin represented by SEQ ID NO: 3 may therefore be characterized by an increased stability and reduced tendency to aggregate. This would be advantageous for the use of these myoglobins in a meat substitute, food ingredient or meat product as described above.

The invention further provides a composition comprising a protein according to this third aspect, as described above.

Nucleic acid

In a fourth aspect, the invention provides a nucleic acid, wherein said nucleic acid may be represented by a sequence comprising a subsequence encoding for a protein which may represented by SEQ ID NO: 3, preferably wherein said subsequence may be represented by SEQ ID NO: 11.

In a preferred embodiment is provided a nucleic acid which may be represented by a sequence comprising a subsequence encoding for a protein which may represented by SEQ ID NO: 3, preferably wherein said subsequence may be represented by SEQ ID NO: 11, wherein said nucleic acid has a length of 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509, 510, 511, 512, 513, 514, 515, 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, or 600 bases.

In a preferred embodiment is provided a nucleic acid which may be represented by a sequence comprising a subsequence encoding for a protein which may represented by SEQ ID NO: 3, preferably wherein said subsequence may be represented by SEQ ID NO: 11, and a terminal sequence at the 5' end of said subsequence. Preferably, said subsequence has a length of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 bases.

In a preferred embodiment is provided a nucleic acid which may be represented by a sequence comprising a subsequence encoding for a protein which may represented by SEQ ID NO: 3, preferably wherein said subsequence may be represented by SEQ ID NO: 11, and a terminal sequence at the 3' end of said subsequence. Preferably, said subsequence has a length of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 bases.

In a preferred embodiment is provided a nucleic acid, wherein said nucleic acid encodes a myoglobin from steppe mammoth, preferably wherein said myoglobin can be represented by SEQ ID NO: 3, most preferably wherein said nucleic acid can be represented by SEQ ID NO: 11.

The invention further provides a composition comprising a nucleic acid according to this fourth aspect, as described above.

<u>Gene construct</u>

In a fifth aspect, there is provided a gene construct comprising a nucleic acid encoding a myoglobin as defined earlier herein.

A "gene construct" as described herein has its customary and ordinary meaning as understood by one of skill in the art in view of this disclosure. A "gene construct" can also be called "expression cassette" or "expression construct" and refers to a gene or a group of genes, including a gene that encodes a protein of interest, which is operably linked to a promoter that controls its expression. The part of this application entitled "general information" comprises more detail as to a "gene construct". "Operably linked" as used herein is further described in the part of this application entitled "general information".

A nucleotide sequence encoding an animal myoglobin may be derived from any animal myoglobin gene or animal myoglobin coding sequence, preferably an animal myoglobin gene or an animal myoglobin coding sequence from mammoth, pig, sheep, cow, chicken or tuna; or a mutated animal myoglobin gene or animal myoglobin coding sequence, preferably from mammoth, pig, sheep, cow, chicken or tuna.

Accordingly, in some embodiments, a preferred nucleotide sequence encoding an animal myoglobin encodes a polypeptide represented by an amino acid sequence comprising a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 1, 2, 3, 4, 5, 6, 7 or 8 and having the respective amino acids at the positions identified earlier herein (i.e. in combination with Q or H at position 65 and H at position 94). Optional amino acid positions may also be taken into account.

In some embodiments, a preferred nucleotide sequence encoding an animal myoglobin encodes a polypeptide represented by an amino acid sequence comprising a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 3 and has at least one of the following amino acid combinations:

- F at position 30, and/or
- Q at position 65, and/or
- H at position 92, and/or
- H at position 94, and/or
- F at position 30 and Q at position 65, and/or
- F at position 30 and H at position 92, and/or
- F at position 30 and H at position 94, and/or
- Q at position 65 and H at position 92, and/or
- Q at position 65 and H at position 94, and/or
- H at position 92 and H at position 94, and/or
- F at position 30 and Q at position 65 and H at position 92, and/or
- F at position 30 and Q at position 65 and H at position 94, and/or
- F at position 30 and H at position 92 and H at position 94, and/or
- Q at position 65 and H at position 92 and H at position 94, and/or
- F at position 30 and Q at position 65 and H at position 92 and H at position 94;.

In some embodiments, a nucleotide sequence encoding an animal myoglobin present in a gene construct according to the invention has at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with any sequence selected from the group consisting of SEQ ID NO's: 9, 10, 11, 12, 13, 14, 15 or 16.

A description of "identity" or "sequence identity" and "similarity" or "sequence similarity" has been provided under the section entitled "general information".

In some embodiments, there is provided a gene construct as described herein, wherein the nucleotide sequence encoding an animal myoglobin is selected from the group consisting of:

(a) a nucleotide sequence encoding a polypeptide represented by an amino acid sequence comprising a sequence that has at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% sequence identity or similarity with the amino acid sequence SEQ ID NO: 1, 2, 3, 4, 5, 6, 7 or 8 and in combination with Q or H at position 65 and H at position 94. Optional amino acid positions may also be taken into account.

(b) a nucleotide sequence that has at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% sequence identity with the nucleotide sequence of SEQ ID NO: 9, 10, 11, 12, 13, 14, 15 or 16.

(c) a nucleotide sequence that differs from the sequence of a nucleotide sequence of (b) due to the degeneracy of the genetic code.

In some embodiments, there is provided a gene construct as described herein, wherein the nucleotide sequence encoding an animal myoglobin is selected from the group consisting of:

(a) a nucleotide sequence encoding a polypeptide represented by an amino acid sequence comprising a sequence that has at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% sequence identity or similarity with the amino acid sequence SEQ ID NO: 3 and having at least one of the following amino acid combinations:

- F at position 30, and/or
- Q at position 65, and/or
- H at position 92, and/or
- H at position 94, and/or
- F at position 30 and Q at position 65, and/or
- F at position 30 and H at position 92, and/or
- F at position 30 and H at position 94, and/or
- Q at position 65 and H at position 92, and/or
- Q at position 65 and H at position 94, and/or
- H at position 92 and H at position 94, and/or
- F at position 30 and Q at position 65 and H at position 92, and/or
- F at position 30 and Q at position 65 and H at position 94, and/or
- F at position 30 and H at position 92 and H at position 94, and/or
- Q at position 65 and H at position 92 and H at position 94, and/or
- F at position 30 and Q at position 65 and H at position 92 and H at position 94;.

(b) a nucleotide sequence that has at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% sequence identity with the nucleotide sequence of SEQ ID NO: 11.
(c) a nucleotide sequence that differs from the sequence of a nucleotide sequence of (b) due to the degeneracy of the genetic code.

An animal myoglobin encoded by the nucleotide sequences described herein or derived from an animal myoglobin as described herein exerts at least a detectable level of an activity of an animal myoglobin as known to a person of skill in the art.

An activity of an animal myoglobin can be to transport iron via its heme domain. This activity could be assessed by methods known to a person of skill in the art, for example as earlier defined herein.

In some embodiments, there is provided a gene construct as described herein, wherein the nucleotide sequence encoding an animal myoglobin is selected from SEQ ID NO: 19, 20, 21, 22, 23 and 24.

Herein, SEQ ID NO: 19 represents a nucleotide sequence encoding a steppe mammoth myoglobin represented by SEQ ID NO: 3, SEQ ID NO: 20 represents a nucleotide sequence encoding a sheep myoglobin represented by SEQ ID NO: 4, SEQ ID NO: 21 represents a nucleotide sequence encoding a cattle myoglobin represented by SEQ ID NO: 5, SEQ ID NO: 22 represents a nucleotide sequence encoding a pig myoglobin represented by SEQ ID NO: 6, SEQ ID NO: 23 represents a nucleotide sequence encoding a chicken myoglobin represented by SEQ ID NO: 7, SEQ ID NO: 24 represents a nucleotide sequence encoding a tuna myoglobin represented by SEQ ID NO: 8, wherein these nucleotide sequences are codon optimized for expression in *Pichia pastoris* (*Komagataella phaffii*). The codon optimization is detailed in Example 2.1.

In some embodiments, the gene construct further comprises a regulatory region. In an embodiment, the gene construct further comprises a promoter. In an embodiment, the gene construct comprises a promoter, a terminator and optionally a signal peptide. The gene construct may also comprise a marker. The signal peptide as used herein preferably facilitates excretion of the expressed myoglobin. The skilled person knows that depending on the host cell used, the identity of the regulatory regions and markers will have to be optimized.

In some embodiments, there is provided a gene construct as described herein, wherein the gene construct is represented by SEQ ID NO: 25, 26, 27, 28, 29 or 30.

Herein, SEQ ID NOs: 25-30 represent gene constructs, codon optimized for expression in *Pichia pastoris* (*Komagataella phaffii*), comprising a nucleotide sequence encoding a steppe mammoth myoglobin represented by SEQ ID NOs: 19-24, respectively, and the signal peptide of *Saccharomyces cerevisiae* mating factor alpha. In other words, SEQ ID NOs: 25-30 correspond to gene constructs for the expression of a steppe mammoth, sheep, cattle, pig, chicken and tuna myoglobin, respectively. The codon optimization is detailed in Example 2.1.

For example if a bacterium (preferably *E. coli*) is used as host cell, the following regulatory regions may be used. A promoter suitable to be used in a bacterium is lac, trp, tac, T7 (used in example), phoA, ara, xapA, cad, recA, spc, bla, P1 and P2 from rrnB ribosomal RNA operon, P$^L$ promoter from phage λ. A terminator suitable to be used in a bacterium is lac, trp, tac, T7 (used in example), phoA, ara, xapA, cad, recA, spc, bla, P1 and P2 from rrnB ribosomal RNA operon, P$^L$ terminator from phage λ. A preferred promoter used is a T7 promoter and/or a preferred terminator is the T7 terminator. A preferred signal peptide for excretion is *E. coli* Sec-recognition peptide (SecA), E. coli Tet-recognition peptide, *E. coli* dsbA, E. coli phoA, E. coli pelB, E. coli MBP (maltose binding protein). A marker suitable for *E coli* is ampicillin. *Alternatively, the proBA* operon from *E. coli* strain K12, including its original transcription regulatory elements may be used to facilitate selection without antibiotics.

In another example, if a yeast is used as host cell, the following regulatory regions may be used. A promoter suitable to be used in yeast may be a constitutive promoter. Examples of suitable constitutive promoters include: a glycolytic promoter selected from FBA1, TPI1, PGK1, PYK1, TDH3, ENO2, HXK2, PGI1, PFK1, PFK2, GPM1 gene or a non-glycolytic promoter of the TEF2 gene.

A suitable promoter to be used in yeast may be inducible. If the yeast is a *Pichia,* the methanol inducible promoter AOX1 is preferred. Otherwise the GAL1 promoter (galactose-inducible) may be used when the yeast is *S. cerevisiae.* The genes mentioned from which a promoter could be derived for a yeast as host cell could also be used to derive a terminator for the same yeast. A preferred signal peptide for excretion for *Pichia* (and *Saccharomyces*) includes: the *S. cerevisiae* alpha mating factor pre- pro- secretion signal peptide, the *S. cerevisiae* Ost1 signal peptide, the *S. cerevisiae* Aga2 signal peptide and fusions thereof.

Examples 2.1 describes the synthesis of a gene construct encoding a myoglobin as defined earlier herein. In the example, AOX1 is used for expression in *Pichia pastoris* strain GS115.

In another example, if a filamentous fungus is used as host cell, the following regulatory regions may be used. The following promoters may be used: the *Aspergillus niger* glucoamylase promoter (*glaA*), the *Aspergillus nidulans* alcohol dehydrogenase promoter (*alcA*) or the *Aspergillus oryzae* taka-amylase A promoter (*amyB*), the *Aspergillus niger* alcohol dehydrogenase promoter (*adhA*), the *Trichoderma reesei* pyruvate kinase promoter (*pki*) or the *Aspergillus nidulas* glyceraldehyde-3-phosphate dehydrogenase promoter (*gpdA*). The genes mentioned from which a promoter could be derived for a filamentous fungus as host cell could also be used to derive a terminator for the same filamentous fungus. A preferred signal peptide for excretion for a filamentous fungus, preferably an *Aspergillus* includes: the *Aspergillus niger* glucoamylase signal peptide (*glaA*), the *Aspergillus niger* a-galactosidase signal peptide (*AglB*) and the *Trichoderma reesei* cellobiohydrolase I (*Cbhl*).

A preferred promoter and terminator for *Aspergillus* (more preferably for *Aspergillus niger*) are the glucoamylase promoter and the glucoamylase terminator of *Aspergillus niger.*

Expression vector

Gene constructs described herein can be placed in expression vectors. Thus, in another aspect there is provided an expression vector comprising a gene construct as described in any of the preceding embodiments.

A description of "expression vector" has been provided under the section entitled "general information".

Composition

In a further aspect there is provided a composition comprising a gene construct as described above and/or an expression vector as described above, optionally further comprising one or more ingredients.

Host cell

In a further aspect, there is provided a host cell comprising a gene construct as defined herein. This host cell may be prokaryote or eukaryote.

A prokaryote may be a bacterium. The bacterium may be a Gram positive/Gram negative bacterium slected from the following list: *Absidia, Achromobacter, Acinetobacter, Aeribacillus, Aneurinibacillus, Agrobacterium, Aeromonas, Alcaligenes, Arthrobacter, Arzoarcus, Azomonas, Azospirillum, Azotobacter, Bacillus, Beijerinckia, Bradyrhizobium, Brevibacills, Burkholderia, Byssochlamys, Citrobacter, Clostridium, Comamonas, Cupriavidus, Corynebacterium, Deinococcus,* **Escherichia,** *Enterobacter, Flavobacterium, Fusobacterium, Gossypium, Klebsiella, Lactobacillus, Listeria, Megasphaera, Micrococcus, Mycobacterium, Norcadia, Porphyromonas, Propionibacterium, Pseudomonas, Ralstonia, Rhizobium, Rhodopseudomonas, Rhodospirillum*, *Rodococcus, Roseburia, Shewanella, Streptomycetes, Xanthomonas, Xylella, Yersinia*, *Treponema, Vibrio, Streptococcus, Lactococcus, Zymomonas, Staphylococcus, Salmonella, Sphingomonas, Sphingobium, Novosphingobium, Brucella* and *Microscilla.*

Preferred bacteria include *Aeribacillus pallidus, Aneurinibacillus terranovensis, Bacillus subtilis, Bacillus amyloliquefaciens, Bacillus coagulans, Bacillus licheniformis, Bacillus megaterium, Bacillus halodurans, Bacillus pumilus, Brevibacillus thermoruber, Brevibacillus panacihumi, Cupriavidus basilensis, G. Iraustophilus, Gluconobacter oxydans, Caulobacter crescentus CB 15, Methylobacterium extorquens, Rhodobacter sphaeroides, Pelotomaculum thermopropionicum*, *Pseudomonas zeaxanthinifaciens, Pseudomonas putida, Paracoccus denitrificans,* **Escherichia coli,** *Corynebacterium glutamicum, Staphylococcus carnosus, Streptomyces lividans, Sinorhizobium melioti, Sphingobium sp., Novosphingobium sp., Sphingomonas henshuiensis,* and *Rhizobium radiobacter.* A preferred bacterium is Escherichia coli.

Preferred Escherichia coli strains include: 58, 679, WG1, DH5$\alpha$, TG1, TOP10, K12 (used in the examples), BL21, BL21 DE3, XL1-Blue, XL10-Gold, TB1, REG-12, W945, HB101, DH1, DP50, AB284, JC9387, AG1, C600, Cavalli Hfr, Y10.

A eukaryote may be a yeast or a filamentous fungus.

Preferred yeasts include **Saccharomyces,** *Kluyveromyces, Candida,* **Pichia,** *Schizosaccharomyces, Hansenula, Kloeckera, Schwanniomyces, Yarrowia, Cryptococcus, Debaromyces, Saccharomycecopsis, Saccharomycodes, Wickerhamia, Debayomyces, Hanseniaspora, Ogataea, Kuraishia, Komagataella, Metschnikowia, Williopsis, Nakazawaea, Torulaspora, Bullera, Rhodotorula, Sporobolomyces.* Within yeasts, the species *Kluyveromyces lactis,* **Saccharomyces cerevisiae,** *Hansenula polymorpha* (also known as *Ogataea henrich*), *Yarrowia lipolytica, Candida tropicalis* and **Pichia pastoris** (also known as *Komagataella phaffii*) are preferred.

Preferred Pichia strains are selected from the following list: Bg09, Bg10, Bg11, Bg12 (exemplified), Bg20, Bg21, Bg22, Bg23, Bg24, Bg25, Bg26, Bg40, Bg43, Bg44, Bg45, Y-11430, X-33, GS115, KM71, SMD1168, SMD1165, MC100-3, most preferred Bg10 and derivatives.

In example 2.3, *Pichia pastoris* strain GS115 (*his4*) is used as the host cell.

Preferred Saccharomyces strains are selected from the following list: S288C, CEN.PK family, CBS 2354, ATCC 2360, ATCC 4098, ATCC 4124, ATCC 4126, ATCC 4127, ATCC 4921, ATCC 7754, ATCC 9763, ATCC 20598, ATCC 24855, ATCC 24858, ATCC 24860, ATCC 26422, ATCC 46523, ATCC 56069, ATCC 60222, ATCC 60223, ATCC 60493, ATCC 66348, ATCC 66349, ATCC 96581. A preferred yeast is a *Pichia* strain, more preferably *Pichia pastoris.*

A filamentous fungus may be selected from the following list including: *Acremonium, Agaricus,* **Aspergillus,** *Aureobasidium, Chrysosporium, Coprinus, Cryptococcus, Filibasidium, Fusarium, Humicola, Magnaporthe, Mucor,*

*Myceliophthora, Neocallinastix, Neurospora, Paecilomyces, Penicillium, Piromyces, Panerochaete, Pleurotus, Schizophyllum, Talaromyces, Thermoascus, Thielavia, Tolypocladium, Ustilago* and *Trichoderma.*

Preferred filamentous fungus are selected from the following list: **Aspergillus niger,** *Aspergillus nidulans, Aspergillus fumigatus, Aspergillus oryzae, Aspergillus vadensis, Penicillium chrysogenum, Penicillium citrinum, Penicillium rubens, Penicillium oxalicum, Penicillium subrubescens, Rasamsonia emersonii, Talaromyces emersonii, Acremonium chrysogenum, Trichoderma reesei, Aspergillus sojae,* and *Chrysosporium lucknowense.*

Preferred strains of filamentous fungus are selected from the following list: *Aspergillus niger* CBS 513.88, N593, CBS 120.49 (as used in the examples), N402, ATCC 1015 *Aspergillus oryzae* ATCC 20423, IFO 4177, ATCC 1011, ATCC 9576, ATCC 14488-14491, ATCC 11601, ATCC12892, *Aspergillus vadensis* CBS 113365, CBS 102787, IMI 142717, IBT 24658, CBS 113226, *Penicillium chrysogenum* CBS 455.95, *Penicillium citrinum* ATCC 38065, *Penicillium chrysogenum* P2, Wisconsin 54-1255, *Penicillium subrubescens* CBS 132785, FBCC 1632, *Talaromyces emersonii* CBS 393.64, *Acremonium chrysogenum* ATCC 36225 or ATCC 48272, *Trichoderma reesei* ATCC 26921 or ATCC56765 or ATCC 26921, *Aspergillus sojae* ATCC11906, *Chrysosporium lucknowense* ATCC44006.

In a preferred embodiment, *Aspergillus* is used as a filamentous fungus. More preferably, an *Aspergillus niger* strain is used.

Methods

The host cells of the invention are useful for the production of a myoglobin. In some embodiments, the myoglobin is from a mammoth, preferably from a woolly mammoth or a steppe mammoth. In some embodiments, the myoglobin is from pig. In some embodiments, the myoglobin is from sheep. In some embodiments, the myoglobin is from cow. In some embodiments, the myoglobin is from chicken. In some embodiments, the myoglobin is from tuna. Accordingly, in a further aspect, the invention provides a method for the production of a myoglobin as defined earlier herein, comprising culturing the host cell of the invention in a suitable medium and optionally recovering the host cell and/or myoglobin. Optionally, the produced myoglobin does not comprise a signal peptide as defined elsewhere herein.

In an embodiment, the invention provides a method for the production of a myoglobin from mammoth as defined earlier herein, preferably a myoglobin comprising at least 70% sequence identity with SEQ ID NO: 1, more preferably a myoglobin comprising at least 70% sequence identity with SEQ ID NO: 1 in combination with Q or H at position 65 and H at position 94 and optionally having at least one of the following amino acids at the following places within SEQ ID NO: 1: E at position 9, K at position 13, T as position 14, P at position 23, L at position 27, V at position 31, G at position 54, Q at position 65, V at position 67, Q at position 84, Q at position 88, I at position 102 and/or E at position 123 comprising culturing the host cell of the invention in a suitable medium and optionally recovering the host cell and/or myoglobin.

In an embodiment, the invention provides a method for the production of a myoglobin from a woolly mammoth as defined earlier herein, preferably a myoglobin comprising at least 70% sequence identity with SEQ ID NO: 2, more preferably a myoglobin comprising at least 70% sequence identity with SEQ ID NO: 2 in combination with Q or H at position 65, Q at position 92 and H at position 94 and optionally having at least one of the following amino acids at the following places within SEQ ID NO: 2: E at position 9, K at position 13, T as position 14, P at position 23, L at position 27, V at position 31, G at position 54, Q at position 65, V at position 67, Q at position 84, Q at position 88, I at position 102, E at position 123 and/or I at position 143 comprising culturing the host cell of the invention in a suitable medium and optionally recovering the host cell and/or myoglobin.

In an embodiment, the invention provides a method for the production of a myoglobin from a steppe mammoth as defined earlier herein, preferably a myoglobin comprising at least 70% sequence identity with SEQ ID NO: 3, more preferably a myoglobin comprising at least 70% sequence identity with SEQ ID NO: 3 in combination with Q or H at position 65, H at position 92 and H at position 94 and optionally having at least one of the following amino acids at the following places within SEQ ID NO: 3: E at position 9, K at position 13, T as position 14, P at position 23, L at position 27, V at position 31, G at position 54, Q at position 65, V at position 67, Q at position 84, Q at position 88, I at position 102, E at position 123 and/or I at position 143 comprising culturing the host cell of the invention in a suitable medium and optionally recovering the host cell and/or myoglobin.

In an embodiment, the invention provides a method for the production of a myoglobin from a steppe mammoth as defined earlier herein, preferably a myoglobin comprising at least 70% sequence identity with SEQ ID NO: 3, more preferably a myoglobin comprising at least 70% sequence identity with SEQ ID NO: 3 in combination with at least one of the following amino acid combinations:

- F at position 30, and/or
- Q at position 65, and/or
- H at position 92, and/or
- H at position 94, and/or
- F at position 30 and Q at position 65, and/or

- F at position 30 and H at position 92, and/or
- F at position 30 and H at position 94, and/or
- Q at position 65 and H at position 92, and/or
- Q at position 65 and H at position 94, and/or
- H at position 92 and H at position 94, and/or
- F at position 30 and Q at position 65 and H at position 92, and/or
- F at position 30 and Q at position 65 and H at position 94, and/or
- F at position 30 and H at position 92 and H at position 94, and/or
- Q at position 65 and H at position 92 and H at position 94, and/or
- F at position 30 and Q at position 65 and H at position 92 and H at position 94;

comprising culturing the host cell of the invention in a suitable medium and optionally recovering the host cell and/or myoglobin.

In an embodiment, the invention provides a method for the production of a myoglobin from sheep as defined earlier herein, preferably a myoglobin comprising at least 70% sequence identity with SEQ ID NO: 4, more preferably a myoglobin comprising at least 70% sequence identity with SEQ ID NO: 4 in combination with Q or H at position 65 and H at position 94 and optionally having at least one of the following amino acids at the following places within SEQ ID NO: 4: N at position 13, Q at position 27, I at position 31, N at position 67, A at position 128, S at position 133, A at position 145 and/or L at position 150, comprising culturing the host cell of the invention in a suitable medium and optionally recovering the host cell and/or myoglobin.

In an embodiment, the invention provides a method for the production of a myoglobin from cow as defined earlier herein, preferably a myoglobin comprising at least 70% sequence identity with SEQ ID NO: 5, more preferably a myoglobin comprising at least 70% sequence identity with SEQ ID NO: 5 in combination with Q or H at position 65 and H at position 94 and optionally having at least one of the following amino acids at the following places within SEQ ID NO: 5: N at position 13, Q at position 27, I at position 31, N at position 67, A at position 128, S at position 133, A at position 145 and/or L at position 150, comprising culturing the host cell of the invention in a suitable medium and optionally recovering the host cell and/or myoglobin.

In an embodiment, the invention provides a method for the production of a myoglobin from pig as defined earlier herein, preferably a myoglobin comprising at least 70% sequence identity with SEQ ID NO: 6, more preferably a myoglobin comprising at least 70% sequence identity with SEQ ID NO: 6 in combination with Q or H at position 65 and H at position 94 and optionally having at least one of the following amino acids at the following places within SEQ ID NO:6: N at position 13, Q at position 27, I at position 31, N at position 67, A at position 128, S at position 133, A at position 145 and/or L at position 150, comprising culturing the host cell of the invention in a suitable medium and optionally recovering the host cell and/or myoglobin.

In an embodiment, the invention provides a method for the production of a myoglobin from chicken as defined earlier herein, preferably a myoglobin comprising at least 70% sequence identity with SEQ ID NO: 7, more preferably a myoglobin comprising at least 70% sequence identity with SEQ ID NO: 7 in combination with Q or H at position 65 and H at position 94 and optionally having at least one of the following amino acids at the following places within SEQ ID NO: 7: Q at position 6, Q at position 10, T at position 13, I at position 14, H at position 27, M at position 31, H at position 35, D at position 36, D at position 42, R at position 43, G at position 49, P at position 53, Q at position 55, G at position 58, A at position 67, Q at position 72, K at position 75, Q at position 79, N at position 82, S at position 85, T at position 93, V at position 111, I at position 116, A at position 117, E at position 118, A at position 121, S at position 128, K at position 133, S at position 145 and/or F at position 150, comprising culturing the host cell of the invention in a suitable medium and optionally recovering the host cell and/or myoglobin.

In an embodiment, the invention provides a method for the production of a myoglobin from tuna as defined earlier herein, preferably a myoglobin comprising at least 70% sequence identity with SEQ ID NO: 8, more preferably a myoglobin comprising at least 70% sequence identity with SEQ ID NO: 8 in combination with Q or H at position 65 and H at position 94.

The features of this aspect are preferably as described elsewhere herein. This method may hereinafter alternatively be referred to as a process according to the invention. Suitable cell culturing methods for use in a process according to the invention are known to the skilled person and are discussed, for example, in van't Riet, K. and Tramper, J., 1st edition, Basic Bioreactor Design, CRC Press, NY, 1991. Such methods include, but are not limited to, submerged fermentation in liquid media, surface fermentation on liquid media and solid-state fermentations. Cell culturing may, for example, be performed by cultivation in micro-titer plates, shake-flasks, small-scale benchtop bioreactors, medium-scale bioreactors and/or large-scale bioreactors in a laboratory and/or an industrial setting. Suitable cell culturing modes include, but are not limited to, continuous, batch and/or fed-batch fermentation as well as their combinations. In an embodiment, cell culturing is performed using continuous fermentation. In a preferred embodiment, cell culturing is performed using batch fermentation. In a more preferred embodiment, cell culturing is performed using fed batch fermentation.

In the context of the invention, "culture medium", hereinafter alternately referred to as "growth medium", can be interpreted to encompass cases wherein the cultured cells are absent as well as cases wherein the cultured cells are present in the culture medium. "Culture broth" refers to the culture medium wherein the cultured cells are present. "Culture supernatant" refers to the culture medium wherein the cultured cells are absent. "Cell-free extract" refers to a cell lysate not comprising the cellular debris. Cell culturing as part of the process of the invention can be performed under conditions conducive to the production of the introduced myoglobins, which are known to the skilled person. Such conditions depend not only on the chemical composition of the culture medium but also on other process parameters including culture duration, temperature, $O_2$ levels in the culture broth and/or headspace, $CO_2$ levels in the culture broth and/or headspace, pH, ionic strength, agitation speed, hydrostatic pressure and the like. Cell culturing can take place using a culture medium comprising suitable nutrients, such as carbon and nitrogen sources and additional compounds such as inorganic salts and vitamins, using procedures known in the art (see, e. g. Bennett, W. and Lasure, L., 1st edition, More Gene Manipulations in Fungi, Academic Press, CA, 1991). Suitable growth media are available from commercial suppliers or may be prepared using published compositions that are suitable for the respective hosts (e.g. in catalogues of the Centraalbureau Voor Schimmelcultures collection (CBS) or of the American Type Culture Collection (ATCC)).

The exact composition of the growth medium and the values of culture process parameters are not critical features of the invention. Any growth medium composition may be contemplated, as long as it allows for growth of the host cell and production of the introduced myoglobins. The growth medium will typically comprise a carbon source to be used for the growth of the cultured cell. The skilled person understands that suitable carbon sources may be added externally to the growth medium or may already be present in said medium. Carbon sources may be present or added individually or in mixtures of multiple carbon sources. Examples of suitable carbon sources known in the art include simple sugars such as glucose, maltose, sucrose, xylose, arabinose, complex sugars such as maltodextrins, hydrolysed starch, starch, molasses, and second-generation feedstocks. Second-generation feedstocks can be particularly attractive because of their lower carbon footprint. Second-generation feedstocks will typically comprise lignocellulosic material. Such material includes any lignocellulose and/or hemicellulose-based materials. Such material may be sourced from agricultural, industrial or municipal, preferably agricultural, waste streams. Examples of suitable materials include (agricultural) biomass, commercial organic matter, municipal solid waste, virgin biomass such as waste paper and garden waste, or non-virgin biomass. General forms of biomass include trees, shrubs and pastures, wheat, wheat straw, sugarcane bagasse, switchgrass, Japanese pampas grass, corn, corn stover, corn cob, canola stalk, soybean stalk, sweet corn, corn kernels, products and by-products from cereal milling (including wet milling and dry milling), such as corn, wheat, and barley, often referred to as "bran or fiber", and municipal solids. Biomass can also be grassy materials, agricultural residues, forestry residues, municipal solid waste, waste paper, and pulp and paper mill residues. Agricultural biomass includes branches, shrubs, tows, corn and corn straw, energy crops, forests, fruits, flowers, cereals, pastures, herbaceous crops, leaves, bark, needles, logs, roots, young trees, short term rotating woody crops, shrubs, switch herbs, trees, vegetables, fruits, vines, sugar beet pulp, wheat middlings, oat hulls, and hard and soft timber (not including toxic wood), and organic waste materials resulting from agricultural processes including agriculture and forestry activities, particularly forestry wood waste. Agricultural biomass may be any of the foregoing alone, or any combination or mixture thereof. Carbon sources such as organic acids, aldehydes, ketones, esters and alcohols may also be contemplated. The use of growth media comprising combinations of multiple different carbon sources may also be contemplated in the process of the invention. Such media could, as a non-limiting example, combine more oxidized carbon sources such as organic acids with more reduced carbon sources such as alcohols. Examples of suitable nitrogen sources known in the art include soy bean meal, corn steep liquor, yeast extract, whey protein, egg protein, casein hydrolysate, urea, ammonia, ammonium salts and nitrate salts. Examples of additional suitable compounds known in the art include phosphate, sulphate, metals such as magnesium, trace elements and vitamins. The exact growth medium requirements will vary based on the host cell, e.g. between yeasts, bacteria and filamentous fungi, said requirements will be known to the skilled person. Accordingly, the growth medium may be a complete (rich) medium or a minimal medium, i.e. a medium comprising only the absolutely necessary components for growth depending on the cultured host cell.

Similar to the composition of the growth medium, process parameters can be assigned any value, as long as they allow for growth of the host cell and production of the introduced myoglobins. Typically, said values will differ based on the host cell that is being cultured and will be known to the skilled person. Preferably, the process according to the invention is an oxygen-limited or aerobic process, meaning that cell culturing is performed under oxygen-limited or aerobic conditions, more preferably the process is oxygen-limited. Oxygen-limited conditions, also known as a micro-aerobic conditions, are culture conditions in which the oxygen consumption is limited by the availability of oxygen. The degree of oxygen limitation is determined by the amount and composition of the ingoing gas flow as well as the actual mixing/mass transfer properties of the fermentation equipment used. Preferably, under oxygen-limited conditions in a liquid culture, the rate of oxygen consumption is at least about 5.5 mmol/L/h, more preferably at least about 6 mmol/L/h and even more preferably at least about 7 mmol/L/h. Aerobic conditions are culture conditions in which the oxygen consumption is not limited by the availability of oxygen.

Cell culturing may be performed at a temperature value that is optimal for the cell, typically at a temperature range of 16-42 °C. In some embodiments, the temperature ranges between 20-40 °C, more preferably between 25-38 °C, most preferably between 28-36 °C. In some most preferred embodiments, a temperature value of about 30 or 36 °C is used.

Cell culturing may be performed at a pH value that is optimal for the cell. In some embodiments, the culture pH value is about pH 2.5, about pH 3.0, about pH 3.5, about pH 4.0, about pH 4.5, about pH 5, about pH 5.5, about pH 6, about pH 6.5, about pH 7, about pH 7.5, about pH 8.0, about pH 8.5, about pH 9. In preferred embodiments, the pH ranges from about pH 3.0 to about pH 9, more preferably from about pH 3.5 to pH 7. In some most preferred embodiments, a pH value of about 6 is used.

Cell culturing may be performed at an ionic strength value of the culture medium that is optimal for the cell, typically at a range between 50 mM - 2 M. In some embodiments, the ionic strength of the culture medium ranges between 75 mM - 1 M, more preferably between 100 mM - 750 mM. In some most preferred embodiments, an ionic strength value of about 100 mM is used.

Cell culturing may be performed for a duration of 14, 13.5, 13, 12.5, 12, 11.5, 11, 10.5, 10, 9.5, 9, 8.5, 8, 7.5, 7, 6.5, 6, 5.5, 5, 4.5, 4, 3.5, 3, 2.5, 2, 1.5 or 1 days, wherein said duration may deviate by 20%. Preferably, cell culturing is performed for a duration of 14, 13.5, 13, 12.5, 12, 11.5, 11, 10.5, 10, 9.5, 9, 8.5, 8, 7.5, 7, 6.5, 6, 5.5, 5, 4.5, 4, 3.5, 3, 2.5, 2, 1.5 or 1 days, wherein said duration may deviate by 10%. More preferably, the cell culturing is performed for 5 days, wherein said duration may deviate by 20%, most preferably by 10%.

The process according to the invention will typically result in the production of at least 100 mg/L, 200 mg/L, 300 mg/L, 400 mg/L, 500 mg/L, 600 mg/L, 700 mg/L, 800 mg/L, 900 mg/L, 1 g/L, 2 g/L, 3 g/L, 4 g/L, 5 g/L, 6 g/L, 7 g/L, 8 g/L, 9 g/L, 10 g/L, 11 g/L, 12 g/L, 13 g/L, 14 g/L, 15 g/L, 16 g/L, 17 g/L, 18 g/L, 19 g/L, 20 g/L, 21 g/L, 22 g/L, 23 g/L, 24 g/L, 25 g/L, 50 g/L, 75 g/L, 100 g/L, 200 g/L or 300 g/L of a myoglobin.

In the process according to the invention, typically at least 10%, 20%, 25%, 30%, 35%, 40%, 45%, 50%, 55%, 60%, 65%, 70%, 75%, 80%, 85%, 90%, 95%, or 100%, preferably at least 20%, more preferably at least 40%, most preferably at least 60% of the carbon source in the growth medium will be converted to a myoglobin.

Cell culturing may also be performed by implementation of a multiple step, preferably a two-step, culture method. For example, a production step of a myoglobin may be preceded by a cellular biomass growth step, wherein only limited production or no production is taking place. The different steps may be carried out using different culture modes and/or different growth media and/or different culture process parameter values, depending on the goal of each step and/or the cultured cell. The biomass during the production step may or may not be actively growing.

In the context of the process of the invention, the host cells and/or myoglobin may optionally be recovered from the culture medium. When present intracellularly, the myoglobin may optionally be recovered from the recovered cellular biomass. Optionally, the recovered myoglobin is purified. Preferably, purification of the myoglobin will result in a purity of at least 70%, more preferably at least 75%, 80%, 85%, 90%, 95%, 96%, 97%, 98%, 99%, most preferably in a myoglobin that is substantially pure.

In an embodiment, the host cell produces the myoglobin extracellularly in the process according to the invention. In a process according to this embodiment, the myoglobin is transported out of the host cell after it is synthesized in the host cell. A process according to this embodiment may be called an extracellular process according to the invention. In this context, both secretory and extracellular fermentations are considered to be extracellular productions.

Without being bound to this theory, an extracellular process according to the invention has the advantage that the downstream processing to recover the produced myoglobin is more convenient, efficient and/or effective. Furthermore, a process according to this product may result in a composition comprising the myoglobin with a high purity such as at least 60%, 65%, 70%, 75%, 80%, 85%, 90%, 95%, 96%, 97%, 98%, or 99% with minimal downstream processing compared to intracellular methods wherein the myoglobin is not transported out of the host cell after its synthesis.

Without being bound to this theory, an extracellular process according to the invention has the advantage that the optional step of recovering myoglobin does not comprise lysing the host cell. As a result, an extracellular process according to the invention may result in a composition having a low concentration of nucleic acids originating from the host cell, as described in an embodiment below.

Accordingly, in a further aspect, the invention provides a method for the production of a myoglobin as defined earlier herein, comprising culturing the host cell of the invention in a suitable medium and optionally recovering the host cell and/or myoglobin.

In an embodiment, an extracellular process according to the invention results in a composition with a purity of myoglobin of at least 60%, 65%, 70%, 75%, 80%, 85%, 90%, 95%, 96%, 97%, 98%, or 99%, most preferably in myoglobin that is substantially pure.

Preferably, purity is measured as the weight percentage of the total protein fraction in the cell-free supernatant obtained at the end of a process or extracellular process according to the invention.

In an embodiment, an extracellular process according to the invention results in a composition comprising less than or equal to 5%, 4.9%, 4.8%, 4.7%, 4.6%, 4.5%, 4.4%, 4.3%, 4.2%, 4.1%, 4%, 3.9%, 3.8%, 3.7%, 3.6%, 3.5%, 3.4%, 3.3%, 3.2%, 3.1%, 3%, 2.9%, 2.8%, 2.7%, 2.6%, 2.5%, 2.4%, 2.3%, 2.2%, 2.1%, 2%, 1.9%, 1.8%, 1.7%, 1.6%, 1.5%, 1.4%, 1.3%, 1.2%, 1.1%, 1%, 0.9%, 0.8%, 0.7%, 0.6%, 0.5%, 0.4%, 0.3%, 0.2%, or 0.1% of nucleic acids originating from the host cell.

In an embodiment, the invention provides an extracellular process according to the invention for the production of a myoglobin from a woolly mammoth as defined earlier herein, preferably a myoglobin comprising at least 70% sequence identity with SEQ ID NO: 2, more preferably a myoglobin comprising at least 70% sequence identity with SEQ ID NO: 2 in combination with Q or H at position 65, Q at position 92 and H at position 94 and optionally having at least one of the following amino acids at the following places within SEQ ID NO: 2: E at position 9, K at position 13, T as position 14, P at position 23, L at position 27, V at position 31, G at position 54, Q at position 65, V at position 67, Q at position 84, Q at position 88, I at position 102, E at position 123 and/or I at position 143 comprising culturing the host cell of the invention in a suitable medium and optionally recovering the host cell and/or myoglobin.

In an embodiment, the invention provides an extracellular process according to the invention for the production of a myoglobin from a steppe mammoth as defined earlier herein, preferably a myoglobin comprising at least 70% sequence identity with SEQ ID NO: 3, more preferably a myoglobin comprising at least 70% sequence identity with SEQ ID NO: 3 in combination with at least one of the following amino acid combinations:

- F at position 30, and/or
- Q at position 65, and/or
- H at position 92, and/or
- H at position 94, and/or
- F at position 30 and Q at position 65, and/or
- F at position 30 and H at position 92, and/or
- F at position 30 and H at position 94, and/or
- Q at position 65 and H at position 92, and/or
- Q at position 65 and H at position 94, and/or
- H at position 92 and H at position 94, and/or
- F at position 30 and Q at position 65 and H at position 92, and/or
- F at position 30 and Q at position 65 and H at position 94, and/or
- F at position 30 and H at position 92 and H at position 94, and/or
- Q at position 65 and H at position 92 and H at position 94, and/or
- F at position 30 and Q at position 65 and H at position 92 and H at position 94;

comprising culturing the host cell of the invention in a suitable medium and optionally recovering the host cell and/or myoglobin.

Example 2.3 provides an extracellular production of steppe mammoth myoglobin.

In an embodiment, the invention provides an extracellular process according to the invention for the production of a myoglobin from sheep as defined earlier herein, preferably a myoglobin comprising at least 70% sequence identity with SEQ ID NO: 4, more preferably a myoglobin comprising at least 70% sequence identity with SEQ ID NO: 4 in combination with Q or H at position 65 and H at position 94 and optionally having at least one of the following amino acids at the following places within SEQ ID NO: 4: N at position 13, Q at position 27, I at position 31, N at position 67, A at position 128, S at position 133, A at position 145 and/or L at position 150, comprising culturing the host cell of the invention in a suitable medium and optionally recovering the host cell and/or myoglobin.

In an embodiment, an extracellular process according to the invention for the production of a myoglobin from cow as defined earlier herein, preferably a myoglobin comprising at least 70% sequence identity with SEQ ID NO: 5, more preferably a myoglobin comprising at least 70% sequence identity with SEQ ID NO: 5 in combination with Q or H at position 65 and H at position 94 and optionally having at least one of the following amino acids at the following places within SEQ ID NO: 5: N at position 13, Q at position 27, I at position 31, N at position 67, A at position 128, S at position 133, A at position 145 and/or L at position 150, comprising culturing the host cell of the invention in a suitable medium and optionally recovering the host cell and/or myoglobin.

In an embodiment, the invention provides an extracellular process according to the invention for the production of a myoglobin from pig as defined earlier herein, preferably a myoglobin comprising at least 70% sequence identity with SEQ ID NO: 6, more preferably a myoglobin comprising at least 70% sequence identity with SEQ ID NO: 6 in combination with Q or H at position 65 and H at position 94 and optionally having at least one of the following amino acids at the following places within SEQ ID NO:6: N at position 13, Q at position 27, I at position 31, N at position 67, A at position 128, S at position 133, A at position 145 and/or L at position 150, comprising culturing the host cell of the invention in a suitable medium and optionally recovering the host cell and/or myoglobin.

In an embodiment, the invention provides an extracellular process according to the invention for the production of a myoglobin from chicken as defined earlier herein, preferably a myoglobin comprising at least 70% sequence identity with SEQ ID NO: 7, more preferably a myoglobin comprising at least 70% sequence identity with SEQ ID NO: 7 in combination with Q or H at position 65 and H at position 94 and optionally having at least one of the following amino acids at the following places within SEQ ID NO: 7: Q at position 6, Q at position 10, T at position 13, I at position 14, H at position 27, M at position 31, H at position 35, D at position 36, D at position 42, R at position 43, G at position 49, P at position 53, Q at position 55, G at position 58, A at position 67, Q at position 72, K at position 75, Q at position 79, N at position 82, S at position 85, T at position 93, V at position 111, I at position 116, A at position 117, E at position 118, A at position 121, S at position 128, K at position 133, S at position 145 and/or F at position 150, comprising culturing the host cell of the invention in a suitable medium and optionally recovering the host cell and/or myoglobin.

In an embodiment, the invention provides an extracellular process according to the invention for the production of a myoglobin from tuna as defined earlier herein, preferably a myoglobin comprising at least 70% sequence identity with SEQ ID NO: 8, more preferably a myoglobin comprising at least 70% sequence identity with SEQ ID NO: 8 in combination with Q or H at position 65 and H at position 94.

The relevant downstream processing technology that may be suitable for recovery and/or purification is not a critical feature of the invention and will depend on whether the myoglobin is accumulated within the cultured cells or excreted. Said processing technology and the associated choice will be known to the skilled person and is discussed, for example, in Wesselingh, J.A and Krijgsman, J., 1st edition, Downstream Processing in Biotechnology, Delft Academic Press, NL, 2013. In a non-limiting example of a recovery process, the biomass is recovered from the culture medium using e.g. centrifugation or filtration. If the produced myoglobin is accumulated within the cells, it can then be recovered and/or purified from the biomass. If it is excreted, it can be recovered from the cell-free medium or, if the biomass separation step is skipped, directly from the culture broth. Recovery and/or purification may be performed according to any conventional recovery or purification methodology known in the art. Methods for recovery and/or purification of proteins are known to the skilled person and are discussed in standard handbooks, such as Sambrook and Russel, Molecular Cloning: A Laboratory Manual, 3rd edition, Cold Spring Harbor Laboratory, Cold Spring Harbor Laboratory Press, NY, 2001 or Ausubel F. et al, eds., Current protocols in molecular biology, Green Publishing and Wiley Interscience, NY, 2003. Examples of widely used recovery and/or purification methods include chromatographic methods such as gel-filtration chromatography, ion-exchange chromatography, immunoaffinity chromatography, metal affinity chromatography, gel-filtration chromatography, fractionation with precipitants such as ammonium sulfate and polyethylene glycol, gel electrophoresis and salting out and dialysis. Preferably, metal affinity chromatography or size-exclusion chromatography is used. Recovery and/or purification may optionally be enhanced by linking the enzyme polypeptide to a sequence that facilitates purification, such as with a GST domain, using well-known molecular toolbox techniques. Optionally, the sequence that facilitates purification and/or signal peptide that facilitates excretion of the myoglobin is removed from the final product using techniques known in the art, for example proteolysis by endopeptidases targeting a linker between the sequence that facilitates purification and/or the signal peptide and the myoglobin. In some embodiments, the enzyme polypeptide is linked (fused) to a hexa-histidine peptide, such as the tag provided in a pET23a(+) vector (Genescript Biotech, Leiden, The Netherlands), among others, many of which are commercially available. As, for example, described in Gentz et al., Proc. Natl. Acad. Sci. USA 86:821-824 (1989), hexa-histidine peptide provides for convenient purificaton of the fusion protein.

In a preferred embodiment, the produced myoglobin is recovered and/or purified from the culture medium. This may be realized continuously with the production process or subsequently to it.

In a preferred embodiment, the produced myoglobin is recovered and/or purified from the cultured cells. This may be realized continuously with the production process, by harvesting fractions of growing cells, or subsequently to it.

In a preferred embodiment, the cultured host cells used in the method of the invention are immobilized. Immobilization of cells may be achieved by any means known to the skill person as discussed in standard handbooks such as Guisan, J.M., Bolivar, J.M., López-Gallego, F., Rocha-Martin, J. (Eds.), Immobilization of Enzymes and Cells: Methods and Protocols, Springer US, USA, 2020. Typically, the host cells can be immobilized to a semi-solid or solid support by three different methods. The first method involves polymerizing or solidifying a spore- or cell-containing solution. Examples of polymerizable or solidifiable solutions include alginate, A-carrageenan, chitosan, polyacrylamide, polyacrylamide-hydrazide, agarose, polypropylene, polyethylene glycol, dimethyl acrylate, polystyrene divinyle benzene, polyvinyl benzene, polyvinyl alcohol, epoxy carrier, cellulose, cellulose acetate, photocrosslinkable resin, prepolymers, urethane, and gelatin. The second method involves cell adsorption onto a support. Examples of such supports include bone char, cork, clay, resin, sand porous alumina beads, porous brick, porous silica, celite, or wood chips. The host cells can colonize the support and form a biofilm. The third method involves the covalent coupling of the host cells to a support using chemical agents like glutaraldehyde, o-dianisidine (U.S. Pat. No. 3,983,000), polymeric isocyanates (U.S. Pat. No. 4,071,409), silanes (U.S. Pat. Nos. 3,519,538 and 3,652,761), hydroxyethyl acrylate, transition metal-activated supports, cyanuric chloride, sodium periodate, toluene, and the like. Cultured host cells can be immobilized in any phase of their growth, for example after a desired cell density in the culture has been reached. Suitable culture modes and/or different culture process parameter values will be known to the skilled person and are discussed in standard handbooks, such as Colin R. Phillips C.R., Poon Y. C., Immobilization of Cells: In Biotechnology Monographs book series (Biotechnology, volume 5), Springer, Berlin, Germany, 1988; Tampion J., Tampion M. D., Immobilized Cells: Principles and Applications, Cambridge University Press, UK, 1987. Preferably, immobilized cells are cultured in packed bed bioreactors, also known as plug-flow bioreactors, or expanded (fluidized) bed bioreactors. Suitable growth media and recovery and/or purification methods are further discussed elsewhere herein.

In a further aspect, there is provided a method for producing a meat substitute as earlier defined in comprising formulating the myoglobin obtainable from the method defined earlier herein into a meat substitute. Depending on the meat substitute and/or the food ingredient envisaged, the skilled person would know which formulations are the most suitable.

General information

Unless stated otherwise, all technical and scientific terms used herein have the same meaning as customarily and ordinarily understood by a person of ordinary skill in the art to which this invention belongs, and read in view of this disclosure.

As used herein, the term "promoter" or "regulatory sequence" refers to a nucleic acid fragment that functions to control the transcription of one or more coding sequences, and is located upstream with respect to the direction of transcription of the transcription initiation site of the coding sequence, and is structurally identified by the presence of a binding site for DNA-dependent RNA polymerase, transcription initiation sites and any other DNA sequences, including, but not limited to transcription factor binding sites, repressor and activator protein binding sites, and any other sequences of nucleotides known to one of skill in the art to act directly or indirectly to regulate the amount of transcription from the promoter. A "constitutive" promoter is a promoter that is active under most physiological and developmental conditions. An "inducible" and/or "repressible" promoter is a promoter that is physiologically or developmentally regulated to be induced and/or repressed, e.g. by the application of a chemical inducer or repressing signal.

As used herein, the term "operably linked" refers to a linkage of polynucleotide elements in a functional relationship. A nucleic acid is "operably linked" when it is placed into a functional relationship with another nucleic acid sequence. For instance, a transcription regulatory sequence such as a promoter is operably linked to a coding sequence if it affects the transcription of the coding sequence. Operably linked means that the DNA sequences being linked are typically contiguous and, where necessary to join two protein encoding regions, contiguous and in reading frame.

As used herein, a "regulator" or "transcriptional regulator" is a protein that controls the rate of transcription of genetic information from DNA to messenger RNA, by binding to a specific DNA sequence.

The terms "protein" or "polypeptide" are used interchangeably and refer to molecules consisting of a chain of amino acids, without reference to a specific mode of action, size, 3-dimensional structure or origin.

The term "gene" means a DNA fragment comprising a region (transcribed region), which is transcribed into an RNA molecule (e.g. an mRNA) in a cell, operably linked to suitable regulatory regions (e.g. a promoter). A gene will usually comprise several operably linked fragments, such as a promoter, a 5' leader sequence, a coding region and a 3'-nontranslated sequence (3'-end) e.g. comprising a polyadenylation- and/or transcription termination site.

"Expression of a gene" refers to the process wherein a DNA region which is operably linked to appropriate regulatory regions, particularly a promoter, is transcribed into an RNA, which is biologically active, i.e. which is capable of being translated into a biologically active protein or peptide.

In amino acid sequences as described herein, amino acids or "residues" are denoted by three-letter symbols. These three-letter symbols as well as the corresponding one-letter symbols are well known to the person skilled in the art and have the following meaning: A (Ala) is alanine, C (Cys) is cysteine, D (Asp) is aspartic acid, E (Glu) is glutamic acid, F (Phe) is phenylalanine, G (Gly) is glycine, H (His) is histidine, I (Ile) is isoleucine, K (Lys) is lysine, L (Leu) is leucine, M (Met) is methionine, N (Asn) is asparagine, P (Pro) is proline, Q (Gln) is glutamine, R (Arg) is arginine, S (Ser) is serine, T (Thr) is threonine, V (Val) is valine, W (Trp) is tryptophan, Y (Tyr) is tyrosine. A residue may be any proteinogenic amino acid, but also any non-proteinogenic amino acid such as D-amino acids and modified amino acids formed by post-translational modifications, and also any non-natural amino acid, as described herein.

In the context of this application, all percentages in the context of a concentration or composition refer to weight percentages, unless defined otherwise.

In the context of this application, expressions such as "a parameter having a value of at least X, Y or Z" should be interpreted as said parameter having a value of at least X, of at least Y, or of at least Z.

*Sequence identity*

In the context of the invention, a nucleic acid molecule such as a nucleic acid molecule encoding an animal myoglobin is represented by a nucleic acid or nucleotide sequence which encodes a protein fragment or a polypeptide or a peptide or a derived peptide.

It is to be understood that each nucleic acid molecule or protein fragment or polypeptide or peptide or derived peptide or construct as identified herein by a given sequence identity number (SEQ ID NO) is not limited to this specific sequence as disclosed. Each coding sequence as identified herein encodes a given protein fragment or polypeptide or peptide or derived peptide or construct or is itself a protein fragment or polypeptide or construct or peptide or derived peptide.

Throughout this application, each time one refers to a specific nucleotide sequence SEQ ID NO (take SEQ ID NO: X as example) encoding a given protein fragment or polypeptide or peptide or derived peptide, one may replace it by:

 i. a nucleotide sequence comprising a nucleotide sequence that has at least 60% sequence identity with SEQ ID NO: X;
 ii. a nucleotide sequence the sequence of which differs from the sequence of a nucleic acid molecule of (i) due to the degeneracy of the genetic code; or
 iii. a nucleotide sequence that encodes an amino acid sequence that has at least 60% amino acid identity or similarity with an amino acid sequence encoded by a nucleotide sequence SEQ ID NO: X.

Another preferred level of sequence identity or similarity is 70%. Another preferred level of sequence identity or similarity is 80%. Another preferred level of sequence identity or similarity is 90%. Another preferred level of sequence identity or similarity is 95%. Another preferred level of sequence identity or similarity is 99%.

Throughout this application, each time one refers to a specific amino acid sequence SEQ ID NO (take SEQ ID NO: Y as example), one may replace it by: a polypeptide represented by an amino acid sequence comprising a sequence that has at least 60% sequence identity or similarity with amino acid sequence SEQ ID NO: Y. Another preferred level of sequence identity or similarity is 70%. Another preferred level of sequence identity or similarity is 80%. Another preferred level of sequence identity or similarity is 90%. Another preferred level of sequence identity or similarity is 95%. Another preferred level of sequence identity or similarity is 99%.

Each nucleotide sequence or amino acid sequence described herein by virtue of its identity or similarity percentage with a given nucleotide sequence or amino acid sequence respectively has in a further preferred embodiment an identity or a similarity of at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% with the given nucleotide or amino acid sequence, respectively.

The terms "homology", "sequence identity" and the like are used interchangeably herein. Sequence identity is described herein as a relationship between two or more amino acid (polypeptide or protein) sequences or two or more nucleic acid (polynucleotide) sequences, as determined by comparing the sequences. In a preferred embodiment, sequence identity is calculated based on the full length of two given SEQ ID NO's or on a part thereof. Part thereof preferably means at least 50%, 60%, 70%, 80%, 90%, or 100% of both SEQ ID NO's. In the art, "identity" also refers to the degree of sequence relatedness between amino acid or nucleic acid sequences, as the case may be, as determined by the match between strings of such sequences. "Similarity" between two amino acid sequences is determined by comparing the amino acid sequence and its conserved amino acid substitutes of one polypeptide to the sequence of a second polypeptide. "Identity" and "similarity" can be readily calculated by known methods, including but not limited to those described in Bioinformatics and the Cell: Modern Computational Approaches in Genomics, Proteomics and transcriptomics, Xia X., Springer International Publishing, New York, 2018; and Bioinformatics: Sequence and Genome Analysis, Mount D., Cold Spring Harbor Laboratory Press, New York, 2004, each incorporated herein by reference.

"Sequence identity" and "sequence similarity" can be determined by alignment of two peptide or two nucleotide sequences using global or local alignment algorithms, depending on the length of the two sequences. Sequences of similar lengths are preferably aligned using a global alignment algorithms (e.g. Needleman-Wunsch) which aligns the sequences optimally over the entire length, while sequences of substantially different lengths are preferably aligned using a local alignment algorithm (e.g. Smith-Waterman). Sequences may then be referred to as "substantially identical" or "essentially similar" when they (when optimally aligned by for example the program EMBOSS needle or EMBOSS water using default parameters) share at least a certain minimal percentage of sequence identity (as described below).

A global alignment is suitably used to determine sequence identity when the two sequences have similar lengths. When sequences have a substantially different overall length, local alignments, such as those using the Smith-Waterman algorithm, are preferred. EMBOSS needle uses the Needleman-Wunsch global alignment algorithm to align two sequences over their entire length (full length), maximizing the number of matches and minimizing the number of gaps. EMBOSS water uses the Smith-Waterman local alignment algorithm. Generally, the EMBOSS needle and EMBOSS water default parameters are used, with a gap open penalty = 10 (nucleotide sequences) / 10 (proteins) and gap extension penalty = 0.5 (nucleotide sequences) / 0.5 (proteins). For nucleotide sequences the default scoring matrix used is DNAfull and for proteins the default scoring matrix is Blosum62 (Henikoff & Henikoff, 1992, PNAS 89, 915-919, incorporated herein by reference). Alternatively, percentage similarity or identity may be determined by searching against public databases, using algorithms such as FASTA, BLAST, etc. Thus, the nucleic acid and protein sequences of some embodiments of the present invention can further be used as a "query sequence" to perform a search against public databases to, for example, identify other family members or related sequences. Such searches can be performed using the BLASTn and BLASTx programs (version 2.0) of Altschul, et al. (1990) J. Mol. Biol. 215:403-10, incorporated herein by reference. BLAST nucleotide searches can be performed with the NBLAST program, score = 100, wordlength = 12 to obtain nucleotide sequences homologous to oxidoreductase nucleic acid molecules of the invention. BLAST protein searches can be performed with the BLASTx program, score = 50, wordlength = 3 to obtain amino acid sequences homologous to protein molecules of the invention. To obtain gapped alignments for comparison purposes, Gapped BLAST can be utilized as described in Altschul et al., (1997) Nucleic Acids Res. 25(17): 3389-3402, incorporated herein by reference. When utilizing BLAST and Gapped BLAST programs, the default parameters of the respective programs (e.g., BLASTx and BLASTn) can be used. See the homepage of the National Center for Biotechnology Information accessible on the world wide web at www.ncbi.nlm.nih.gov/. Optionally, in determining the degree of amino acid similarity, the skilled person may also take into account so-called conservative amino acid substitutions. As used herein, "conservative" amino acid substitutions refer to the interchangeability of residues having similar side chains. Examples of classes of amino acid residues for conservative substitutions are given in the Tables below.

| Acidic Residues | Asp (D) and Glu (E) |
|---|---|
| Basic Residues | Lys (K), Arg (R), and His (H) |
| Hydrophilic Uncharged Residues | Ser (S), Thr (T), Asn (N), and Gln (Q) |
| Aliphatic Uncharged Residues | Gly (G), Ala (A), Val (V), Leu (L), and Ile (I) |
| Non-polar Uncharged Residues | Cys (C), Met (M), and Pro (P) |
| Aromatic Residues | Phe (F), Tyr (Y), and Trp (W) |

Alternative conservative amino acid residue substitution classes :

| 1 | A | S | T |
|---|---|---|---|
| 2 | D | E | |
| 3 | N | Q | |
| 4 | R | K | |
| 5 | I | L | M |
| 6 | F | Y | W |

Alternative physical and functional classifications of amino acid residues:

| Alcohol group-containing residues | S and T |
|---|---|
| Aliphatic residues | I, L, V, and M |
| Cycloalkenyl-associated residues | F, H, W, and Y |
| Hydrophobic residues | A, C, F, G, H, I, L, M, R, T, V, W, and Y |
| Negatively charged residues | D and E |
| Polar residues | C, D, E, H, K, N, Q, R, S, and T |
| Positively charged residues | H, K, and R |
| Small residues | A, C, D, G, N, P, S, T, and V |
| Very small residues | A, G, and S |
| Residues involved in turn formation | A, C, D, E, G, H, K, N, Q, R, S, P and T |
| Flexible residues | Q, T, K, S, G, P, D, E, and R |

For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine;

and a group of amino acids having sulphur-containing side chains is cysteine and methionine. Preferred conservative amino acids substitution groups are: valine-leucine-isoleucine, phenylalanine-tyrosine, lysine-arginine, alanine-valine, and asparagine-glutamine. Substitutional variants of the amino acid sequence disclosed herein are those in which at least one residue in the disclosed sequences has been removed and a different residue inserted in its place. Preferably, the amino acid change is conservative. Preferred conservative substitutions for each of the naturally occurring amino acids are as follows: Ala to Ser; Arg to Lys; Asn to Gln or His; Asp to Glu; Cys to Ser or Ala; Gln to Asn; Glu to Asp; Gly to Pro; His to Asn or Gln; Ile to Leu or Val; Leu to Ile or Val; Lys to Arg; Gln or Glu; Met to Leu or Ile; Phe to Met, Leu or Tyr; Ser to Thr; Thr to Ser; Trp to Tyr; Tyr to Trp or Phe; and, Val to Ile or Leu.

*Gene or coding sequence*

The term "gene" means a DNA fragment comprising a region (transcribed region), which is transcribed into an RNA molecule (e.g. an mRNA) in a cell, operably linked to suitable regulatory regions (e.g. a promoter). A gene will usually comprise several operably linked fragments, such as a promoter, a 5' leader sequence, a coding region and a 3'-nontranslated sequence (3'-end) e.g. comprising a polyadenylation- and/or transcription termination site. "Expression of a gene" refers to the process wherein a DNA region which is operably linked to appropriate regulatory regions, particularly a promoter, is transcribed into an RNA, which is biologically active, i.e. which is capable of being translated into a biologically active protein or peptide.

*Promoter*

As used herein, the term "promoter" or "transcription regulatory sequence" refers to a nucleic acid fragment that functions to control the transcription of one or more coding sequences, and is located upstream with respect to the direction of transcription of the transcription initiation site of the coding sequence, and is structurally identified by the presence of a binding site for DNA-dependent RNA polymerase, transcription initiation sites and any other DNA sequences, including, but not limited to transcription factor binding sites, repressor and activator protein binding sites, and any other sequences of nucleotides known to one of skill in the art to act directly or indirectly to regulate the amount of transcription from the promoter. A "constitutive" promoter is a promoter that is active under most physiological and developmental conditions. An "inducible" promoter is a promoter that is physiologically or developmentally regulated, e.g. by the application of a chemical inducer.

*Operably linked*

As used herein, the term "operably linked" refers to a linkage of polynucleotide elements in a functional relationship. A nucleic acid is "operably linked" when it is placed into a functional relationship with another nucleic acid sequence. For instance, a transcription regulatory sequence is operably linked to a coding sequence if it affects the transcription of the coding sequence. Operably linked means that the DNA sequences being linked are typically contiguous and, where necessary to join two protein encoding regions, contiguous and in reading frame. Linking can be accomplished by ligation at convenient restriction sites or at adapters or linkers inserted in lieu thereof, or by gene synthesis.

*Proteins and amino acids*

The terms "protein" or "polypeptide" or "amino acid sequence" are used interchangeably and refer to molecules consisting of a chain of amino acids, without reference to a specific mode of action, size, 3-dimensional structure or origin. In amino acid sequences as described herein, amino acids or "residues" are denoted by three-letter symbols. These three-letter symbols as well as the corresponding one-letter symbols are well known to a person of skill in the art and have the following meaning: A (Ala) is alanine, C (Cys) is cysteine, D (Asp) is aspartic acid, E (Glu) is glutamic acid, F (Phe) is phenylalanine, G (Gly) is glycine, H (His) is histidine, I (Ile) is isoleucine, K (Lys) is lysine, L (Leu) is leucine, M (Met) is methionine, N (Asn) is asparagine, P (Pro) is proline, Q (Gln) is glutamine, R (Arg) is arginine, S (Ser) is serine, T (Thr) is threonine, V (Val) is valine, W (Trp) is tryptophan, Y (Tyr) is tyrosine. A residue may be any proteinogenic amino acid, but also any non-proteinogenic amino acid such as D-amino acids and modified amino acids formed by post-translational modifications, and also any non-natural amino acid.

*Gene constructs*

Gene constructs as described herein could be prepared using any cloning and/or recombinant DNA techniques, as known to a person of skill in the art, in which a nucleotide sequence encoding said myoglobin is expressed in a suitable cell, e.g. cultured cells or cells of a multicellular organism, such as described in Ausubel et al., "Current Protocols in Molecular Biology", Greene Publishing and Wiley-Interscience, New York (1987) and in Sambrook and Russell (2001, *supra*); both of which are incorporated herein by reference in their entirety. Also see, Kunkel (1985) Proc. Natl. Acad. Sci. 82:488 (describing site directed mutagenesis) and Roberts et al. (1987) Nature 328:731-734 or Wells, J.A., et al. (1985) Gene 34: 315 (describing cassette mutagenesis).

*Expression vectors*

The phrase "expression vector" or "vector" generally refers to a tool in molecular biology used to obtain gene expression in a cell, for example by introducing a nucleotide sequence that is capable of effecting expression of a gene or a coding sequence in a host compatible with such sequences. An expression vector carries a genome that is able to stabilize and remain episomal in a cell. Within the context of the invention, a cell may mean to encompass a cell used to make the construct or a cell wherein the construct will be administered. Alternatively, a vector is capable of integrating into a cell's genome, for example through homologous recombination or otherwise.

These expression vectors typically include at least suitable promoter sequences and optionally, transcription termination signals. An additional factor necessary or helpful in effecting expression can also be used as described herein. A nucleic acid or DNA or nucleotide sequence encoding a myoglobin is incorporated into a DNA construct capable of introduction into and expression in an *in vitro* cell culture. Specifically, a DNA construct is suitable for replication in a prokaryotic host, such as bacteria, *e.g., E. coli,* or can be introduced into a cultured mammalian, plant, insect, (e.g., Sf9), yeast, fungi or other eukaryotic cell lines.

A DNA construct prepared for introduction into a particular host may include a replication system recognized by the host, an intended DNA segment encoding a desired polypeptide, and transcriptional and translational initiation and termination regulatory sequences operably linked to the polypeptide-encoding segment. The term "operably linked" has already been described herein. For example, a promoter or enhancer is operably linked to a coding sequence if it stimulates the transcription of the sequence. DNA for a signal sequence is operably linked to DNA encoding a polypeptide if it is expressed as a preprotein that participates in the secretion of a polypeptide. Generally, a DNA sequence that is operably linked are contiguous, and, in the case of a signal sequence, both contiguous and in reading frame. However, enhancers need not be contiguous with a coding sequence whose transcription they control. Linking is accomplished by ligation at convenient restriction sites or at adapters or linkers inserted in lieu thereof, or by gene synthesis. The selection of an appropriate promoter sequence generally depends upon the host cell selected for the expression of a DNA segment. Examples of suitable promoter sequences include prokaryotic, and eukaryotic promoters well known in the art (see, *e.g.* Sambrook and Russell, 2001, *supra*). A transcriptional regulatory sequence typically includes a heterologous enhancer or promoter that is recognised by the host. The selection of an appropriate promoter depends upon the host, but promoters such as the trp, lac and phage promoters, tRNA promoters and glycolytic enzyme promoters are known and available (see, *e.g.* Sambrook and Russell, 2001, *supra*). An expression vector includes the replication system and transcriptional and translational regulatory sequences together with the insertion site for the polypeptide encoding segment. In most cases, the replication system is only functional in the cell that is used to make the vector (bacterial cell as *E. Coli*). Most plasmids and vectors do not replicate in the cells infected with the vector. Examples of workable combinations of cell lines and expression vectors are described in Sambrook and Russell (2001, *supra)* and in Metzger et al. (1988) Nature 334: 31-36. For example, suitable expression vectors can be expressed in, yeast, *e.g. S. cerevisiae, e.g.,* insect cells, *e.g.,* Sf9 cells, mammalian cells, *e.g.,* CHO cells and bacterial cells, *e.g., E. coli.* A cell may thus be a prokaryotic or eukaryotic host cell. A cell may be a cell that is suitable for culture in liquid or on solid media. Alternatively, a host cell is a cell that is part of a multicellular organism such as a transgenic plant or animal.

The selection of an appropriate promoter sequence generally depends upon the host cell selected for the expression of a DNA segment. Examples of suitable promoter sequences include prokaryotic, and eukaryotic promoters well known in the art (see, *e.g.* Sambrook and Russell, 2001, *supra).* A transcriptional regulatory sequence typically includes a heterologous enhancer or promoter that is recognised by the host. The selection of an appropriate promoter depends upon the host, but promoters such as the trp, lac and phage promoters, tRNA promoters and glycolytic enzyme promoters are known and available (see, *e.g.* Sambrook and Russell, 2001, *supra).* An expression vector includes the replication system and transcriptional and translational regulatory sequences together with the insertion site for the polypeptide encoding segment. In most cases, the replication system is only functional in the cell that is used to make the vector (bacterial cell as *E. Coli*). Most plasmids and vectors do not replicate in the cells infected with the vector. Examples of workable combinations of cell lines and expression vectors are described in Sambrook and Russell (2001, *supra)* and in Metzger et al. (1988) Nature 334: 31-36. For example, suitable expression vectors can be expressed in yeast, *e.g. S. cerevisiae, e.g.,* and bacterial cells, *e.g., E. coli.* A cell may thus be a prokaryotic or eukaryotic host cell. A cell may be a cell that is suitable for culture in liquid or on solid media.

*Expression*

Expression may be assessed by any method known to a person of skill in the art. For example, expression may be assessed by measuring the levels of transgene expression in the transduced tissue on the level of the mRNA or the protein by standard assays known to a person of skill in the art, such as qPCR, RNA sequencing, Northern blot analysis, Western blot analysis, mass spectrometry analysis of protein-derived peptides or ELISA.

Expression may be assessed at any time after administration of the gene construct, expression vector or composition as described herein. In some embodiments herein, expression may be assessed after 1 week, 2 weeks, 3 weeks, 4, weeks, 5 weeks, 6 weeks, 7 weeks, 8 weeks, 9, weeks, 10 weeks, 11 weeks, 12 weeks, 14 weeks, 16 weeks, 18 weeks, 20 weeks, 22 weeks, 24 weeks, 28 weeks, 32 weeks, 36 weeks, 40 weeks, or more.

In this document and in its claims, the verb "to comprise" and its conjugations is used in its non-limiting sense to mean that items following the word are included, but items not specifically mentioned are not excluded. In addition, the verb "to consist" may be replaced by "to consist essentially of" meaning that a meat substitute, a gene construct, a host cell (or methods) as described herein may comprise additional component(s) (or additional steps) than the ones specifically identified, said additional component(s) not altering the unique characteristic of the invention. In addition, the verb "to consist" may be replaced by "to consist essentially of" meaning that a method as described herein may comprise additional step(s) than the ones specifically identified, said additional step(s) not altering the unique characteristic of the invention.

Reference to an element by the indefinite article "a" or "an" does not exclude the possibility that more than one of the elements is present, unless the context clearly requires that there be one and only one of the elements. The indefinite article "a" or "an" thus usually means "at least one".

As used herein, with "at least" a particular value means that particular value or more. For example, "at least 2" is understood to be the same as "2 or more" i.e., 2, 3, 4, 5, 6, 7, 8, 9, 10, 11, 12, 13, 14, 15, ..., etc.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The word "about" or "approximately" when used in association with a numerical value (e.g. about 10) preferably means that the value may be the given value (of 10) more or less 0.1% of the value. As used herein, the term "and/or" indicates that one or more of the stated cases may occur, alone or in combination with at least one of the stated cases, up to with all of the stated cases.

Various embodiments are described herein. Each embodiment as identified herein may be combined together unless otherwise indicated.

All patent applications, patents, and printed publications cited herein are incorporated herein by reference in the entireties, except for any definitions, subject matter disclaimers or disavowals, and except to the extent that the incorporated material is inconsistent with the express disclosure herein, in which case the language in this disclosure controls.

One skilled in the art will recognize many methods and materials similar or equivalent to those described herein, which could be used in the practice of the present invention. Indeed, the present invention is in no way limited to the methods and materials described.

The present invention is further described by the following examples which should not be construed as limiting the scope of the invention.

**Legend to the figures**

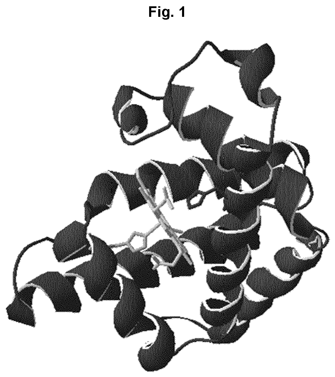

**Figure 1.** Tridimensional structure of bovine myoglobin. Structure of wildtype pork Mb, solved by X-ray diffraction at a resolution of 1.8 Å (Krzywda et al. 1998). Shown are the heme,, the proximal histidine (His94) and the distal histidine (His65).

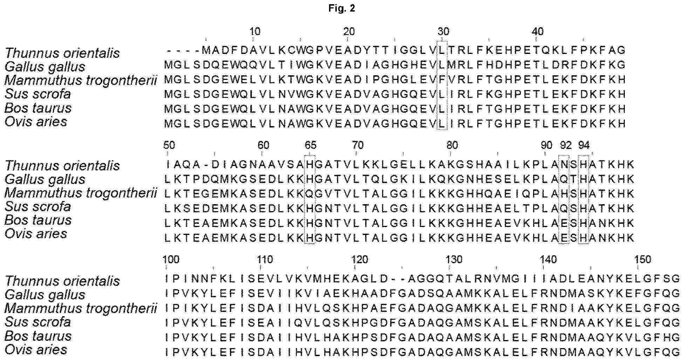

**Figure 2.** Comparison of the myoglobin protein sequence from six animal species. The sequence alignment shows sequences from tuna (*Thunnus orientalis*), chicken (*Gallus gallus*), the steppe mammoth *(Mammuthus trogontherii)*, pork (*Sus scrofa*), cattle (*Bos taurus*) and sheep (*Ovis aries*). The level of amino acid conservation is indicated by blue shading, using the BLOSUM62 score. The proximal histidine (His94, boxed) is present in all six species. The steppe mammoth carries a glutamine (Gln65, boxed) at the position normally occupied by the distal histidine (His65, boxed), a phenylalanine at position 30 (Phe30, boxed), and a histidine at position 92 (His92, boxed).

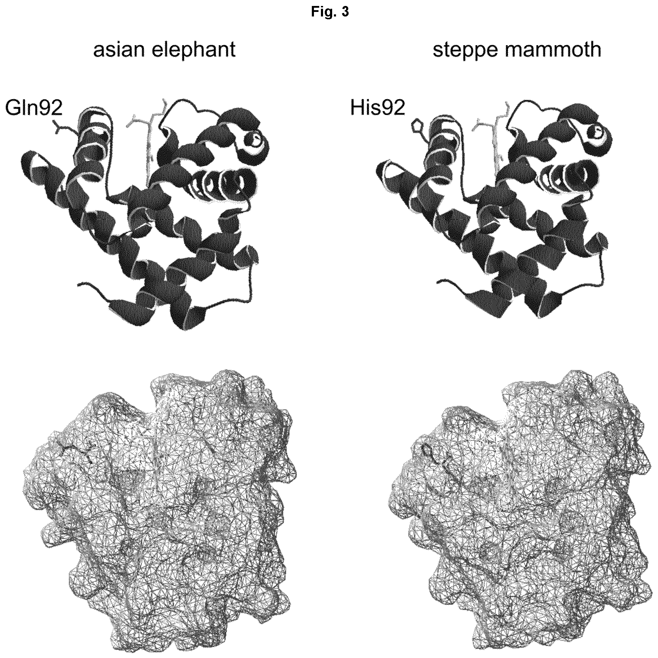

**Figure 3.** Model of the tridimensional structure of myoglobin from the asian elephant (left) and the steppe mammoth (right). The ribbon (top) and surface (bottom) views are shown. The heme molecule is depicted , and so are the residue at position 92 (glutamine in the elephant, histidine in the mammoth) . In the surface view, positive charges are shown in darker grey.

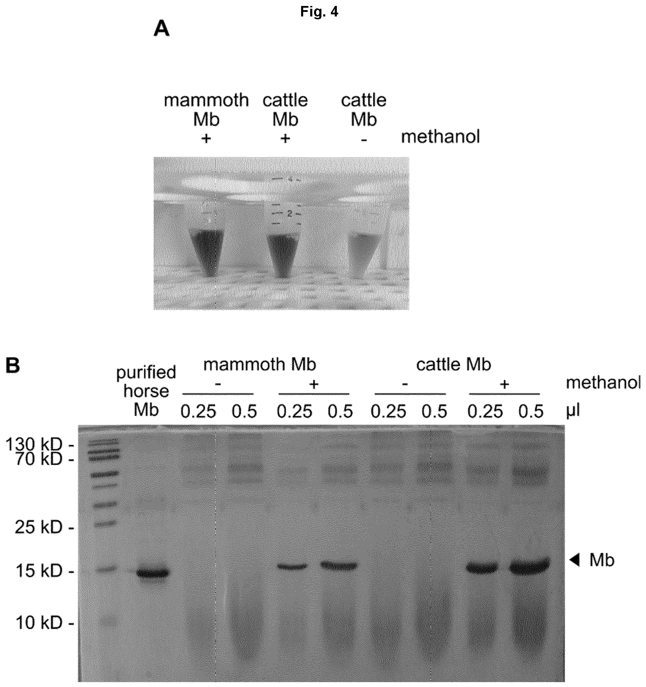

**Figure 4.** Production of extracellular animal Mb in *Pichia pastoris.* Cell-free supernatants were collected after fermentation with (+) or without (-) methanol induction, and concentrated 10-fold by ultrafiltration. Concentrated samples obtained after methanol induction showed a dark-red color (A) and, after analysis by SDS-PAGE, revealed a protein of the expected molecular weight for Mb (B).

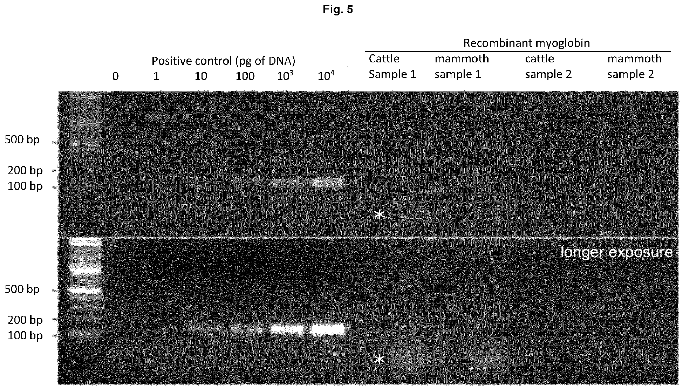

**Figure 5.** Absence of recombinant DNA in the myoglobin preparations obtained by fermentation. The same gel is shown with two different exposure times (upper and bottom panel). The asterisk indicates the primers that remain after the PCR reaction.

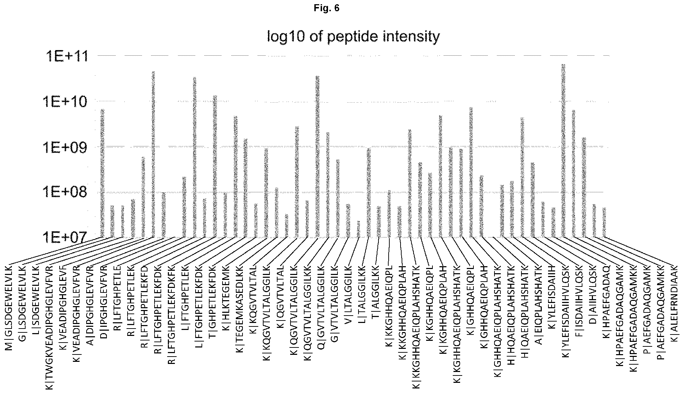

**Figure 6.** Sequence coverage of mammoth myoglobin identification by mass spectrometry. A bar graph (lower panel) shows the intensity of the different peptides in logarithmic scale. The sequences of the peptides at the bottom of the graph correspond to SEQ ID NOs: 31-80.

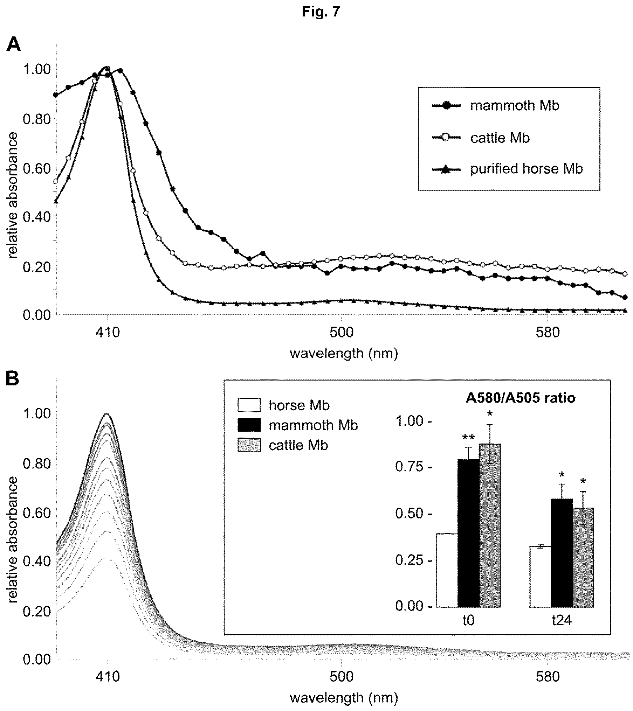

**Figure 7.** Absorption spectra of mammoth and cattle myoglobin solutions. (A) The absorbance spectrum of recombinant steppe mammoth Mb (black circles), cattle Mb (white circles) and commercially available Mb, purified from equine muscle (triangles) was recorded at room temperature. (B) Absorbance spectra of horse Mb during a 24h incubation at pH5.6 and 25°C. The insert shows the ratio between absorbance at 580 nm (MbO2) and at 505 nm (MetMb) for the recombinant mammoth Mb (black bars), cattle Mb (grey bars) and commercially available horse Mb (white bar). Asterisks represent statistically significant differences between the recombinant Mb and horse Mb: (*) = $p < 0.05$, (**) = $p < 0.01$.

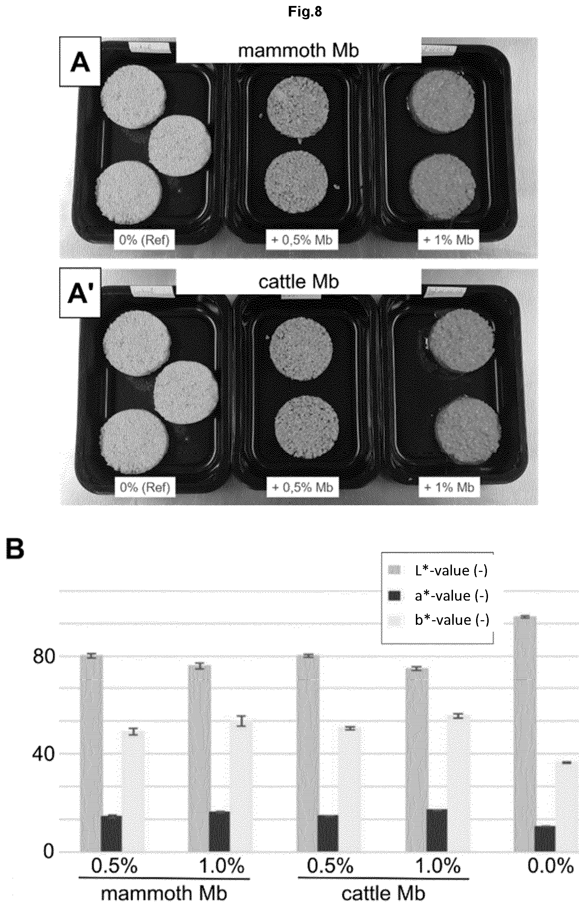

**Figure 8.** Color of lab-made meat analogues containing myoglobin. Pictures (A) and quantifications (B) showing the effect of myoglobin addition on the color of plant-based burgers.

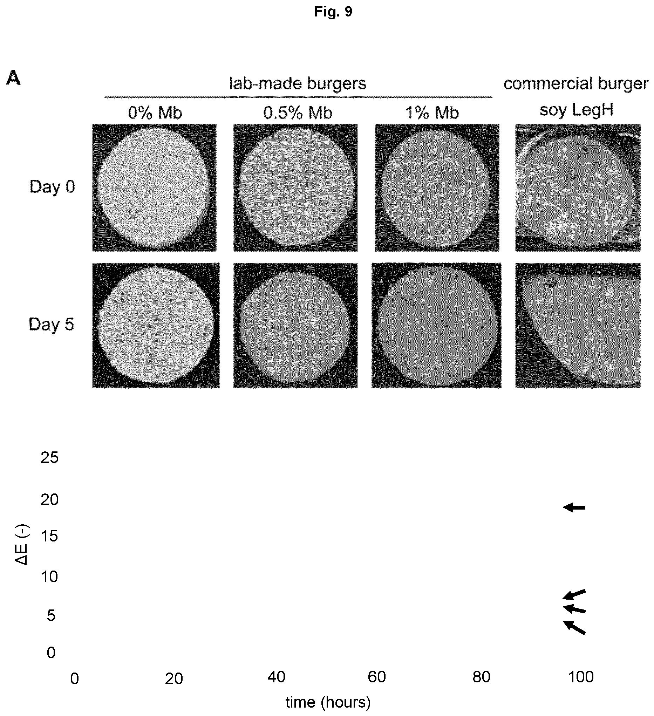

**Figure 9.** Color stability of meat analogues containing myoglobin. Pictures (A) and quantifications (B) showing color changes observed over time in plant-based burgers containing various concentrations of purified horse Mb, exposed to constant light at 4°C. A commercial burger containing soy leghemoglobin was included for comparison.

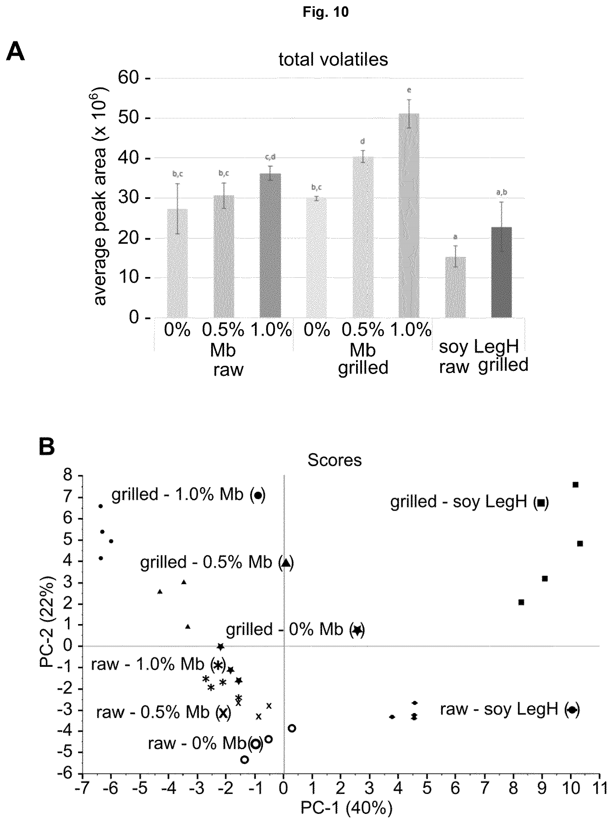

**Figure 10.** Volatile compounds extracted from lab-made meat analogues containing recombinant heme proteins. (A) Quantification of the number of volatile compounds retrieved from plant-based burgers by HS-SPME GC-MS. Values not sharing the same subscript were found significantly different from each other at $p < 0,05$ according to Tukey's HSD test. (B) Principal Component Analysis (PCA) of volatiles from lab-made plant-based burgers, either raw (shown in green) or gilled (shown in blue). A commercially available plant-based burger, containing a recombinant soy leghemoglobin (LegH), was used as reference.

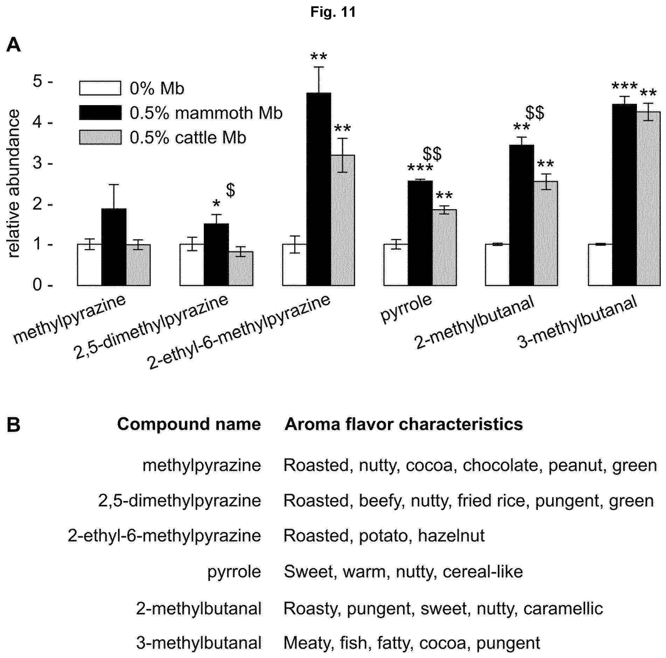

**Figure 11.** Figure 11. Volatile compounds associated with the addition of recombinant myoglobin to lab-made meat analogues. (A) Relative amounts of volatile flavors active-compounds detected in grilled plant-based burgers, normalized to the levels observed in plant-based burgers without Mb. Asterisks represent statistically significant differences between the burgers without and with Mb: (*) = $p < 0.5$, (**) = $p < 0.01$, (***) = $p < 0.001$. $ represent statistically significant differences between the mammoth and cattle Mb: ($) = $p < 0.05$, ($$) = $p < 0.05$. (B) Aroma description of the volatile compounds, which are also found in cooked meat (van Ba et al. 2021, The Good Scent Company information system).

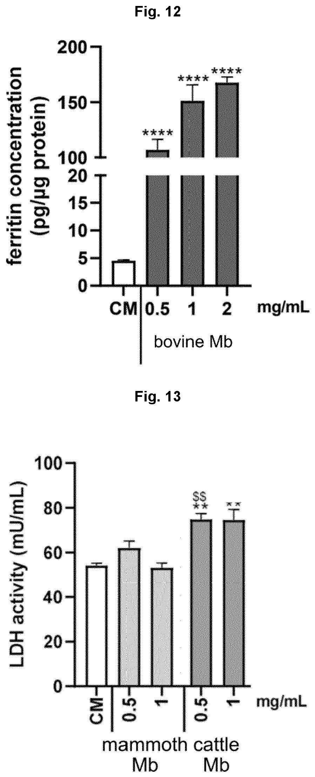

**Figure 12.** Iron bioavailability from myoglobin. Iron uptake (content of ferritin in ng, normalized to the total protein content in mg) by human Caco-2 intestinal cells exposed to purified bovine Mb at 0.5 mg/ml, 1.0 mg/ml or 2.0 mg/ml, compared to the control medium alone (CM). Asterisks represent statistically significant differences between CM and treatments: (****) = $p < 0.0001$.

**Figure 13.** Effect of recombinant myoglobin on differentiated Caco-2 monolayers. Cytotoxicity was measured after 24h of incubation in the presence of recombinant mammoth or cattle Mb, or the complete medium (CM) as control. Asterisks represent statistically significant differences between CM and treatments: (*) = $p < 0.05$; (**) = $p < 0.01$.

**Examples**

Example 1: production method A

*Example 1.1: General construction of gene constructs and vectors*

In some instances, complete vectors and/or genomic integration cassettes suitable for expression in *Pichia Pastoris, Escherichia coli* and *Aspergillus niger* are synthetically made by commercial suppliers. In some instances, the construction of vector fragments and/or genomic integration cassettes is performed using PCR with primers designed for the introduction of compatible overhangs between fragments and commercially available polymerases, according to the manufacturer's protocol, followed by complete vector and/or genomic integration cassette assembly performed in vitro using commercially available kits, according to the manufacturers' protocols. In some instances, the complete vector assembly is performed in vivo by transformation of vector fragments into yeast cells as described previously in the art (Kuijpers et al. 2013) followed by isolation of the complete vectors using commercially available kits.

Complete vectors comprise the known necessary origin of replication sequences for maintenance of said vectors in the respective organism, the nucleotide sequence to be expressed (selected from: SEQ ID NO: , 9, 10, 11, 12, 13, 14, 15, 16), suitable regulatory elements for expression in the respective organism (at least a promoter and a terminator) and a selection marker allowing the screening of correct transformants and maintenance of the vector in transformed strains. Genomic integration cassettes comprise the nucleotide sequence to be expressed (selected from: SEQ ID NO: 9, 10, 11, 12, 13, 14, 15, 16), suitable regulatory elements for expression in the respective organism (at least a promoter and a terminator), and, optionally, 5' and 3' regions with suitable length, such as from 30-3000 bp, which are homologous to the respective genomic integration site to facilitate homologous recombination. In some instances, the genomic integration cassettes are fused and/or co-transformed with suitable selection markers to facilitate screening. In some instances, the suitable selection markers fused with the genomic integration cassette are removed from the final production strain after transformation using methods generally known in the art such as counter-selection in suitable media, use of the Cre-Lox recombinase system or use of CRISPR/Cas methods, such as to create a marker-free strain. In some instances, the nucleotide sequences to be expressed from the vectors and/or genomic integration cassettes are operably linked to sequences encoding for suitable signal peptides to facilitate excretion of the expressed fused protein and/or sequence tags to facilitate the purification of the expressed fused protein after its excretion. In some instances, the operably linked sequences are such as to resulting in the expressed peptides being fused to the N-terminus of the fusion protein. In some instances, the fused sequence comprises recognition sites for native or synthetic peptidases which facilitate cleavage of the signal peptide and/or tag from the fused protein upon or after excretion of the expressed fused protein by the cell.

*Example 1.2: General strain construction*

*Pichia pastoris, Escherichia coli* and *Aspergillus niger* strains are transformed using molecular toolbox techniques known in the art. Selection of correct transformants is performed under known suitable growth conditions for each organism, in suitable known selective growth media such as comprising antibiotics, depending on whether a selection marker is used and its type (dominant/auxotrophic), after allowing for sufficient time for colony growth to be observed. Correct transformants in each case are confirmed by diagnostic PCR or other suitable known molecular toolbox methods such as Southern blotting. In some instances wherein genomic integration cassettes are used for transformation, the strains to be transformed are deficient in non-homologous end-joining repair machinery to facilitate homologous recombination. In some instances wherein a selection marker was used for transformation, the selection marker is removed as described in Example 1, such as to generate a marker-free strain. Correctly transformed strains are preserved in glycerol stocks at -80 °C for further use.

*Example 1.3: Production of myoglobin by transformed strains*

Transformed strains obtained in Example 2 are tested for production of extracellular myoglobin. Cell culturing is performed by cultivation in shake-flasks, under conditions conducive to the production of the protein suitable for each host which are generally known, such as temperature, pH and ionic strength of the growth medium, and agitation speed. In some instances, the temperature value ranges from 16-40 °C, the pH value ranges from 3-7, the ionic strength value ranges from 100-1000 mM and the agitation speed ranges from 100-300 rpm. The growth medium suitable for cultivation of each host is generally known and comprises carbon and nitrogen sources, as well as additional nutrients such as inorganic salts and vitamins. Cultures are inoculated from frozen stocks of the transformed strains. After 12-24 h of culture, the culture broth is collected and the biomass is removed via centrifugation. Culture supernatant is then tested for the presence of the produced myoglobin using known techniques such as SDS-PAGE electrophoresis by identification of the correct size band or by Western blotting using commercially available antibodies. The results confirm the production of extracellular myoglobin. The produced myoglobin is subsequently purified from the culture supernatant and purified by chromatographic techniques according to standard protocols. The absence of the excretion peptide (cleaved during excretion) is confirmed via mass spectrometry analysis according to standard protein sequencing protocols. The purified myoglobin is stored at -20 °C for later use.

*Example 1.4: Production of a meat substitute*

Purified myoglobin produced in Example 3 is used for the production of muscle tissue and fat (adipose) tissue according to known procedures. To produce a muscle tissue substitute, purified myoglobin is cross-linked with pea vicilin protein via transglutaminase. A muscle tissue substitute is also produced by forming a heated gel of pea vicilin protein, to which purified myoglobin is added and thoroughly mixed during cooling down of the heated gel to room temperature. A muscle tissue substitute is also produced by co-extrusion of purified myoglobin with pea vicilin protein. A fat tissue substitute is produced by forming a heated gel of pea albumin protein, oil and lecithin following high pressure homogenization, to which the purified myoglobin is added and thoroughly mixed during cooling down of the heated gel to room temperature. A connective tissue substitute is produced using a zein protein source by extrusion according to known procedures. The muscle, fat and connective tissue substitutes are combined in desired ratios in a meat grinder to produce the meat substitute. In some instances, the meat substitute is then cooked.

*Example 1.5: Production of purified myoglobin by Escherichia coli*

Full length myoglobin genes from woolly mammoth, steppe mammoth, sheep, cow, pig, chicken and tuna (SEQ ID NOs 9, 10, 11, 12, 13, 14, 15, 16) are synthesized and cloned in a modified pET-23a(+) vector, comprising the T7 promoter and terminator and a C-terminal hexa His-tag (Genscript Biotech, Leiden, the Netherlands). Herein, it is understood that SEQ ID NO: 9 codes for only part of a myoglobin, but may nevertheless be synthesized and cloned in said vector. The *ampR* marker gene originally present in said vector is replaced by the *proBA* operon from *E. coli* strain K12, including its original transcription regulatory elements, to facilitate selection without antibiotics. The correctly assembled plasmids are confirmed by PCR and used to transform a proline auxotrophic *E. coli* protein production strain (*E. coli* K12 *ΔproBA*). Transformed strains are incubated overnight in shake-flasks containing minimal medium (10.5 g/L $K_2HPO_4$, 4.5 g/L $KH_2PO_4$, 1.0 g/L $(NH_4)_2SO_4$, 0.12 g/L MgSO4, 0.5 g/l Nacitrate, 2 g/L glucose, and 5.0 mg/L thiamine·HClat 37°C and 150 rpm **(pH 6).** 500 µl of the overnight culture is transferred to a 1 L shake-flask containing 500 mL minimal medium and incubated at 37°C and 150 rpm, until an OD600 of 0.4-1 is reached. IPTG (isopropyl-β-D-thiogalactoside) at a concentration of 100 µM is added to the culture, after which the culture is incubated for 24 h at 16°C and 150 rpm. The culture is harvested and centrifuged at 3500 x g (4°C) for 15 min. The supernatant is discarded and the pellet is dissolved in 50 mL BugBuster Protein Extraction Reagent (Novagen), containing 1 KU Lysozyme/ml (Sigma-Aldrich), 25 U Benzonase® Nuclease and cOmplete™, EDTA-free Protease Inhibitor Cocktail (Roche). The dissolved pellet is incubated for 30 min at 4°C in a shaker. The centrifugation step is repeated and the cell-free extract (supernatant) is collected and assayed by SDS-PAGE to confirm the production of myoglobins. The production of woolly mammoth, steppe mammoth, sheep, cow, pig, chicken and tuna myoglobins by the respective transformed strains is confirmed by the presence of protein bands of the correct size.

For purification, the cell-free extracts containing the soluble fraction of proteins is loaded to a HisTrap FF 1mL column (Cytiva, MA, USA), coupled with an AKTA start system. The column is equilibrated with 20 mM HEPES, 0.4 M NaCl, and 20 mM imidazole, pH 7.5, 1 mL/min flow rate. The protein is eluted with 20 mM HEPES, 0.4 M NaCl, and 400 mM imidazole, pH 7.5. The fractions containing the myoglobins are pooled, concentrated and confirmed by SDS-PAGE and Western Blotting using an anti-histidine tag antibody (Bio-Rad), which confirm the successful purification of all myoglobins. The purified myoglobins are stored at -20°C for later use.

*Example 1.6: Production of purified myoglobin by Aspergillus niger*

Full length myoglobin expression cassettes comprising the genes from woolly mammoth, steppe mammoth, sheep, cow, pig, chicken and tuna (SEQ ID NOs 9, 10, 11, 12, 13, 14, 15, 16), under the control of *A. niger glaA* promoter and terminator, operably linked to the excretion signal sequence of pectinmethylesterase of *A. niger* to facilitate myoglobin excretion, are synthesized. Herein, it is understood that SEQ ID NO: 9 codes for only part of a myoglobin, but may nevertheless be comprised in said expression cassette Genomic integration cassettes are assembled by fusion PCR of myoglobin expression cassettes with the orotidine 5'-phosphate decarboxylase gene sequence (*pyrG*) from *Aspergillus oryzae,* as well as 1000 bp at the 5'and 3' ends which are homologous to the native upstream and downstream sequences of the *A. niger pyrG* gene.

The genomic integration cassettes are transformed to protoplasts of *A. niger* CBS 120.49 *ΔkusA ΔpyrG.* Prior to transformation, protoplasts are prepared by overnight shake-flask cultivation in complete medium (2% (mass/vol) glucose, 6 g/L NaNO$_3$, 1.5g g/L $KH_2PO_4$, 0.5 g/L KCl, 0.5 g/L $MgSO_4 \cdot 7H_2O$, 0.2% (mass/vol) tryptone, 0.1% (mass/vol) yeast extract, 0.1% (mass/vol) casamino acids, 0.05% (mass/vol) yeast RNA, and trace elements according to Vishniac (1957)) at 30°C and 250 rpm. Mycelia are harvested by filtration and dissolved in PS buffer (0.2 M sodium phosphate buffer, 0.8 M L-sorbitol, pH 6), to which 0.5 g VinoTaste® Pro lysing enzyme per g of mycelia is added, followed by incubation at 30°C and 100 rpm. Undigested mycelia are removed via filtration and the protoplasts are collected by mild centrifugation (1500 x g, 3°C), washed with SC solution (182.2 g L$^{-1}$ sorbitol, 7.35 g L$^{-1}$ CaCl$_2$* 2H$_2$O) and resuspended in the same solution to a concentration of 10$^8$ protoplasts/mL. Fresh protoplasts are transformed by mixing 200 µL of protoplast suspension with 5 µg of genomic integration cassette in a mixture additionally containing 20 µL of 0.4 M ATA (aurintricarboxylic acid ammonium salt) and 100 µL of 20% PEG-4000, followed by incubation for 10 min. Then, 5 mL of 1.2 M sorbitol solution are added and the mixture is incubated for another 10 min. The transformed protoplasts are collected by mild centrifugation and resuspended in 1 mL of the same sorbitol solution. Transformed protoplasts are plated in minimal medium agar plates (1.5% (mass/vol) agar, 2% (mass/vol) glucose, 6 g/L NaNO$_3$, 1.5 g/L KH$_2$PO$_4$, 0.5 g/L KCl, 0.5 g/L MgSO$_4$*7H$_2$O, and trace elements according to Vishniac (1957)) and incubated at 30°C for 4 days (pH 5). DNA from selected transformants is extracted using standard phenol/chloroform extraction. Correct integration of genomic integration cassettes comprising the myoglobin genes from woolly mammoth, steppe mammoth, sheep, cow, pig, chicken and tuna are confirmed by Southern blotting.

Spores of correct transformants are obtained by incubation on complete medium agar plates for four days at 30°C and harvested with 10 mL N-(2-acetamido)-2-aminoethanesulfonic acid (ACES) buffer. 2 x 10$^8$ spores are used to inoculate shake-flask cultures containing 400 mL minimal medium and incubated overnight at 30°C, 250 rpm. Mycelia are transferred to fresh shake-flask cultures containing 400 mL minimal medium and at 30°C, 250 rpm for 24 h. The cells are removed by culture harvesting followed by centrifugation at 3200 x g at 4°C for 10 min. The supernatant is assayed by SDS-PAGE to confirm the production of myoglobins. The expression of woolly mammoth, steppe mammoth, sheep, cow, pig, chicken and tuna myoglobins by the respective transformed strains is confirmed by the presence of protein bands of the correct size.

For purification, culture supernatants containing the soluble fraction of proteins are loaded to a HiLoad 16/600 Superdex 75 pg column (10 mm x 300 mm) (Cytiva, MA, USA). The column is equilibrated with 0.15 M ammonium acetate, pH 6.0, 0.75 mL/min flow rate. The fractions containing the myoglobins are pooled, concentrated and confirmed by SDS-PAGE and LC-MS according to standard protocols. The results confirm the presence of the purified myoglobins without the excretion signal peptide.

*Example 1.7. Production of purified myoglobin by Pichia pastoris*

Full length myoglobin expression cassettes comprising the genes from woolly mammoth, steppe mammoth, sheep, cow, pig, chicken and tuna (SEQ ID NOs 9, 10, 11, 12, 13, 14, 15, 16), under the control of *P. pastoris* native *pgk1* promoter and terminator, are synthesized. Genomic integration cassettes are assembled by fusion PCR of myoglobin expression cassettes with the gene encoding the native *P. pastoris* histidine biosynthesis trifunctional protein gene (*his4*) under control of its own promoter and terminator, as well as 1000 bp at the 5'and 3' ends which are homologous to the native upstream and downstream sequences of the *P. pastoris his4* locus. The genomic integration cassettes are transformed to the antibiotics-resistance marker free, histidine auxotrophic, *P. pastoris* strain Bg12 (BioGrammatics Inc. Carlsbad, CA), using the lithium-acetate transformation protocol as previously described in Gietz and Woods (2002) (Gietz RD et al., (2002), Methods Enzymol., 350: 87-96). Correct transformants are obtained by incubation on 2% w/v agar plates containing synthetic medium (without histidine to facilitate selection) as previously described in Verduyn et al. (1992) (Verduyn C., et al (1992), Yeast, 8: 501-517): 5 g/L (NH$_4$)$_2$SO$_4$, 3 g/L KH$_2$PO$_4$, 0.5 g/L MgSO$_4$.7H$_2$O, 4.5 mg/L ZnSO$_4$.7H$_2$0, 0.3 mg/L CoCl$_2$.6H$_2$O, 1 mg/L MnCl$_2$.4H$_2$O, 0.3 mg/L CuSO$_4$.5H$_2$O, 4.5 mg/L CaCl$_2$.H$_2$O, 3 mg/L FeSO$_4$.7H$_2$O, 0.4 mg/L NaMoO$_4$.2H$_2$O, 1 mg/L H$_3$BO$_3$, 0.1 mg/L KI, 0.05 g/L biotin, 1 mg/L calcium pantothenate, 1 mg/L nicotinic acid, 25 mg/L inositol, 1 mg/L thiamine.HCl, 1 mg/L pyridoxine.HCl, 0.2 mg/L para-aminobenzoic acid and 2% w/v glucose as carbon source (pH 5). Plates are incubated at 30°C for 4 days. Correct integration of genomic integration cassettes comprising the myoglobin genes from woolly mammoth, steppe mammoth, sheep, cow, pig, chicken and tuna is confirmed by colony PCR, following DNA preparation using a Yeast Protein Kit (ZymoResearch, Irvine, CA), according to manufacturer's protocol. Glycerol stocks of correct transformants are prepared and stored at -80°C.

For myoglobin production, frozen glycerol stocks are used to inoculate 1L-pre-culture shake-flasks containing 500 mL of synthetic medium and incubated overnight at 30°C and 250 rpm. The pre-cultures are used to inoculate subsequent shake-flask cultures to a starting OD660 of 0.2. Cultures are incubated at 30°C and 250 rpm for 24h. The cultures are harvested and centrifuged at 3500 x g (4°C) for 15 min. The supernatant is discarded, the cell pellet is resuspended in ice-cold demineralized water and the centrifugation step is repeated. The supernatant is discarded and the cells are lysed using a Yeast Protein Kit (ZymoResearch, Irvine, CA), according to manufacturer's protocol, together with mechanical disruption. The mixture containing the cellular debris and the soluble proteins is centrifuged at 3500 x g (4°C) for 15 min. The supernatant (cell-free exract) containing the soluble proteins is collected and assayed by SDS-PAGE to confirm the production of myoglobins. The production of woolly mammoth, steppe mammoth, sheep, cow, pig, chicken and tuna myoglobins by the respective transformed strains is confirmed by the presence of protein bands of the correct size.

For purification, cell-free extracts containing the soluble fraction of proteins are loaded to a HiLoad 16/600 Superdex 75 pg column (10 mm x 300 mm) (Cytiva, MA, USA). The column is equilibrated with 0.15 M ammonium acetate, pH 6.0, 0.75 mL/min flow rate. The fractions containing the myoglobins are pooled, concentrated and confirmed by SDS-PAGE and LC-MS according to standard protocols. The results confirm the presence of the purified myoglobins.

Example 2: production method B

Unless explicitly mentioned otherwise, "mammoth" and "mammoth myoglobin" refer to "steppe mammoth" and "steppe mammoth myoglobin" in Example 2.

*Example 2.1. Materials and methods*

*Sequence analysis*

The sequence coding for myoglobin from *Bos taurus, Gallus gallus, Thunnus orientalis, Ovis aries* and *Sus scrofa* were obtained from Uniprot (accession numbers: P02192, P02197, P68190, P02190, P02189, respectively). The sequence coding for myoglobin from the steppe mammoth (*Mammuthus Trogontherii*), which was unknown at the time this application was filed, was obtained by the inventors after DNA extraction from a molar sample from the so-called Adycha specimen, Illumina DNA sequencing, merging the reads and mapping them against the African savannah elephant (*Loxodonta africana*) genome (van der Valk et al. 2021).
Multiple sequence alignments were performed with Clustal Omega (Sievers et al. 2011) and visualized using Jalview 2.11.1.4 (Waterhouse et al. 2009).

*Protein modelling*

The structure of wildtype deoxymyoglobin from *Sus scrofa* was retrieved from the RCSB Protein Data Bank (PDB accession number: 1MWD, chain A). The structure of the steppe mammoth myoglobin was modelled using the automated protein structure homology-modelling server SWISS-MODEL (Waterhouse et al. 2018), using the crystal structure of myoglobin from *Elephas maximus* (asian elephant) as template (PDB accession number: 1EMY). Net surface charge ($Z_{Mb}$) was calculated as the sum of the charge of all ionizable groups at pH 6.5 using published, site-specific ionization constants (Mirceta et al. 2013). All protein structures were visualized and figures generated using DeepView v4.1 (Guex and Peitsch 1997).

*Construction of expression plasmids*

Myoglobin coding sequences were codon optimized for expression in *Pichia pastoris* (*Komagataella phaffii*) (Love et al. 2016) (SEQ ID NO: 19-24). The optimized sequences, preceded by the coding sequence of the *Saccharomyces cerevisiae* mating factor alpha (SEQ ID NO: 25-30) were chemically synthetized by GenScript. Gene fragments were cloned into the pBDIPp5 vector, downstream of the AOX1 promoter and upstream of the AOX1 terminator, the *HIS4* selection marker and the *AOX1* 3' fragment. Vectors were amplified in *E. coli* (DH10B), purified with the Plasmid kit (Quiagen) and verified by Sanger sequencing. Expression cassettes were generated from the resulting vectors by restriction of the plasmids with BglII (New England Biolabs), which cuts upstream from the AOX1 promoter and downstream from the AOX1 3' fragment, and purified with the QIAquick Gel Extraction Kit (QUiagen).

*Strains engineering*

The *Pichia pastoris* (*Komagataella phaffii*) strain GS115 (*his4*) was obtained from Life Technologies. Cell transformation was performed using the electroporation method essentially as previously described (Cregg 2007). Briefly, cells of the GS115 strain were grown in YPD (1% yeast extract, 2% peptone and 2% D-glucose) medium. Cells in the exponentially growing phase were incubated for 30 min in YPD medium with 200 mM HEPES buffer (pH 8.0) and 25 mM dithiothreitol. Competent cells were then washed with ice-cold 1M sorbitol, and transferred to a sterile electroporation cuvette (Bio-Rad). Cells were then electroporated with 1-5 μg of linear expression cassette (see 3.3) using a Gene-Pulser (Bio-Rad) electroporator, and resuspended in 1 mL of YPD medium containing 1M sorbitol before transfer to a sterile 1.5-mL eppendorf tube. Cells were incubated at 28°C without agitation for 3 h, before plating onto agar plates containing solid MGY medium (Minimal Glycerol Medium: 1.34% Yeast Nitrogen Base with ammonium sulfate without amino acids, 2% D-glucose, 4 $10^{-5}$% biotin with 2% agar). Plates were incubated for up to 4 days at 28°C.
Transformants able to grow in the absence of histidine were screened for their ability to express Mb. Cells were grown in 50 mL Falcon tubes in BMGY medium (1% yeast extract, 2% peptone, 100 mm Potassium Phosphate buffer (pH6), 1.34% Yeast Nitrogen Base with ammonium sulfate without amino acids, 4 $10^{-5}$% biotin, 1% glycerol). 1% methanol was added to exponentially growing cells, to induce Mb expression. Samples were collected 24h, 48h, 72h and 96h after the start of the methanol induction, and analyzed by SDS-PAGE to assess Mb levels (see below).

The best producing strains were selected for further experiments, and were cryopreserved as a master cell bank at -80 °C prior to use. Results shown here were obtained using the PAL-02-02 (mammoth Mb) and PAL-02-03 (cattle Mb) strains.

*Recombinant protein production and purification*

Cells from the master cell bank were plated on a YPD agar (1% yeast extract, 2% peptone and 2% D-glucose, 2% agar) plate and incubated for 2 days at 28°C. A seed culture was then prepared in a baffled flask containing 100 mL of BMGY medium, and incubated for 24h at 28°C in an orbital shaker incubator. The seed culture was then used to inoculate a 3L spherical glass flask or a glass-vessel fermenter (Sartorius) containing 900 ml of BMGY medium with 0.03% FoamAway™ Irradiated AOF (ThermoFisher). The propagation phase was conducted for about 24h, until all the glycerol was consumed. The temperature was then lowered to 26°C, and methanol 1% was added every 12h to induce Mb expression. Dissolved oxygen was maintained above 20% during the whole fermentation, while the pH was kept at 6 during the propagation phase and 5 during the induction phase.

After 96h of methanol induction, cells were removed by centrifugation at 4,000 rpm at 4°C. Remaining cells were removed by microfiltration using nitrocellulose filters with a pore diameter of 0.45 $\mu$m (Millipore). The cell-free supernatants were stored frozen at -20°C. Where needed for the assays, cell-free supernatants were further concentrated by using disposable ultrafiltration centrifuge devices, with a polyethersulfone membrane having a molecular-weight cutoff of 10 kD (Thermo Scientific Pierce Protein Concentrators).

The presence of recombinant DNA in the cell-free supernatant was tested by PCR, using oligonucleotides (Sigma) designed to be complementary to the sequence encoding the signal peptide. A DNA fragment amplified from the genomic DNA of the PAL-02-02 strain (mammoth Mb) was used as positive control, next to 1 $\mu$l of concentrated cell-free supernatant containing the mammoth or cattle Mb. PCR reactions were conducted using the OneTaq® Quick-Load® 2X Master Mix with Standard Buffer (New England Biolabs) according to the manufacturer's instructions. PCR products were vizualized after migration at 60V for 60 min in a 2% agarose TAE gel with ethidium bromide. The the Quick-Load® Purple 1 kb Plus DNA Ladder (New England Biolabs) was used to control the size of the amplified fragment.

*Sodium dodecyl sulfate polyacrylamide gel electrophoresis (SDS-PAGE)*

For protein electrophoresis, 20 $\mu$l of concentrated cell-free supernatant were mixed with an equal volume of 2X protein loading buffer (100 mM Tris (pH6,8); 4 mM EDTA, 4% SDS, 20% glycerol; 0.02% bromophenol blue, 4% β-mercaptoethanol). Samples were incubated at 95°C for 5 minutes, and the appropriate volume loaded in a 15% polyacrylamide gel on a vertical mini-PROTEAN gel apparatus (Bio-Rad) at 200 V for 40 minutes. The molecular weight marker (PageRuler™ Prestained Protein Ladder) was purchased from Thermofisher. After electrophoresis, proteins in the gel were stained with Coomassie Brilliant Blue G-250 (Bio-Rad).

*Mass spectrometry*

For MS analysis, the SDS-PAGE gel band corresponding to Mb was excised from the Coomassie-stained gel using a scalpel knife. The sample was rinsed with sterile water and stored at -20°C until processed for MS analysis at the VIB Proteomics Core (Gent, Belgium). Peptides were generated by digestion with 1 $\mu$g trypsin (Promega) overnight at 37°C, and stored at -20°C until LC-MS/MS analysis. Peptides were re-dissolved in loading solvent and injected for LC-MS/MS analysis using a Q Exactive HF mass spectrometer (Thermo Fisher). Data analysis was performed with MaxQuant algorithm (version 2.0.1.0) with default search settings including a false discovery rate set at 1% on peptide-spectrum match (PSM), peptide and protein level. All raw spectral data files were searched together against the theoretical protein sequence of mammoth Mb and the following reference proteomes in the Uniprot database: *Komagataella phaffii* (database release version of 2021_11, containing 5,073 protein sequences), Bos taurus (taxid 9913, database release version of 2021_11, containing 37,513 protein sequences), and Sus scrofa (taxid 9823, database release version of 2021_11, containing 49,792 protein sequences).

*Plant-based meat analogues*

Plant-based burgers were prepared by mixing 25% texturized soy proteins, 15% sunflower oil, 1.5% NaCl, 1% methylcellulose and 57.5% water. Recombinant myoglobin or commercially available myoglobin (purified from equine muscle, Sigma) were added at 0.5% or 1% final concentration, similar to the myoglobin content of white and red meat, respectively. Recombinant myoglobin preparations were obtained by lyophilization of the cell-free supernatant. For the burgers containing 1% Mb, 13.8 g of additional water were added per 100g of burger. Commercially available plant-based burgers containing soy Leghemoglobin (Impossible Food) were included for comparison in some assays.

*Spectrometry and color analyses*

Absorbance spectra were recorded with a Synergy microplate reader (BioTek). Absorbance measurements for the determination of myoglobin auto-oxidation rate were performed in quartz cuvettes using an Ultrospec III spectrophotometer (Pharmacia). Color measurements were performed using a portable Miniscan EZ 4500L 45°/0° (Hunterlab, Murnau, Germany) with 8 mm viewing area size, illuminant D65 and 10° standard observer to register the L*, a*and b* values (based on the CIELAB color space). The difference in color over time (ΔE) was calculated according the CIE76 formula:

$$\Delta E\text{-value (-)}$$
$$\Delta E = \sqrt{(\Delta L^*)^2+(\Delta a^*)^2+(\Delta b^*)^2}$$

*Aromatic analyses*

Aromas from plant-based meat alternatives containing different concentrations of recombinant Mb were analyzed using gas chromatography-mass spectrometry (GC-MS) with headspace solid-phase microextraction (HS-SPME). Samples were analyzed raw or after grilling. The headspace volatiles were extracted by Divinylbenzene-Carboxen-Polydimethylsiloxane (DVB/CAR/PDMS) coated fibers, and separated by an HP-1ms column, before identification by mass spectrometry. For data analysis, the area under the peaks of the different aromatic compounds was evaluated by one-way ANOVA (analysis of variance), followed by Tukey's HSD (Honestly Significant Difference) posthoc test when statistically significant differences were found. Multivariate statistical analysis was performed using PCA (Principle Component Analysis).

*Iron bioavailability and bioaccessibility*

Assays were performed by ProDigest (Gent, Belgium). Caco-2 cells (HTB-37; American Type Culture Collection) were seeded at 5 x 105 cells in 12-wells coated with 0.1% gelatin. Cells were grown for 14 days in complete medium (Dulbecco's Modified Eagle Medium (DMEM) supplemented with 20% heat-inactivated fetal bovine serum (FBS), 10 mM HEPES and 1x antibiotic-antimycotic) with 3 medium changes/week. 24h prior to stimulation, cells were washed once with Minimum Essential Medium (MEM) supplemented with 10 mM HEPES, 2 mM L-Glutamine, 1x antibiotic-antimycotic, 11 μM hydrocortisone, 0.87 μM insulin, 0.02 μM sodium selenite, 0.05 μM 3,3',5-Triiodo-L-thyronine sodium salt, 20 μg/L epidermal growth factor and further incubated in this medium for 24h. Then, cells were incubated with bovine Mb (Tebu-bio N.V.) at three concentrations (0.5, 1 and 2 mg/mL) or with supplemented MEM as negative control (indicated as CM). After 24h of incubation at 37°C, cells were washed twice with ice-cold PBS and lysed with CelLytic™ (Sigma Aldrich). Human ferritin levels were determined using a human ferritin ELISA kit (ThermoScientific) according to the manufacturer's instructions. Finally, protein concentrations were determined using Pierce™ BCA Protein Assay kit (ThermoFisher Scientific) following the microplate procedure instructions. All assays were performed in triplicate.

*In vitro toxicity assay*

Assays were performed by ProDigest (Gent, Belgium). Caco-2 cells were seeded in 24-well plates coated with 0.1% gelatin an cultivated as for the ferritin assay. On the day of the toxicity test, cells were incubated with recombinant mammoth or cattle Mb at 0.5, 1.0 or 2.0 mg/ml. Cytotoxicity assays were performed after 24h of incubation with Mb at 37°C. To assess possible toxicity induced by the products on Caco-2 cells, a lactate dehydrogenase (LDH) cytotoxicity assay (Merck Life Science B.V.) was performed on the supernatants, according to the manufacturers' instructions. All assays were performed in triplicate. To assess differences between the complete medium control (CM) and the products, an ordinary one-way ANOVA with Dunnett's multiple comparisons test was performed for each timepoint separately. (*) represents statistically significant differences between CM and products. (*) = p<0.05; (**) = p<0.01; (***) = p<0.001 and (****) = p<0.0001. All statistics were performed using GraphPad Prism version 9.1.2 for Windows (GraphPad Software, San Diego, CA, USA).

*Bacterial Reverse Mutation Assay (AMES Test)*

The genotoxicity assay was performed according to the OECD Guideline for chemical testing n°471. The AMES FT Mutagenicity Test Kit (Moltox, Trinova) was used following the manufacturer's instructions. All assays were performed in triplicate. The mean revertant counts for each strain treated with the vehicle were within the expected range based on data provided by the manufacturer. Mutation rates were calculated as the ratio between the number of revertants observed with the test compound and with the vehicle alone.

*Example 2.2. Structural characteristics of myoglobin*

Myoglobin (Mb) is a relatively small globular protein of about 17 kD, found in heart and skeletal muscles. It carries a single heme group capable of reversible oxygen binding (Fig. 1), allowing myoglobin to transport oxygen from the cell surface to mitochondria. Mb is also the primary pigment responsible for meat color. In oxygenated Mb (oxymyoglobin), the iron atom of the heme group is coordinated by four nitrogen atoms of the porphyrin ring, the so-called "proximal" histidine (His94) in the Mb chain, and an oxygen molecule. This can be further stabilized by hydrogen bonding between the oxygen molecule and another histidine in the heme binding pocket of Mb, the "distal" histidine (His65). Under this form, Mb has a typical bright red color. In the absence of oxygen, Mb takes a darker red color (deoxymyoglobin). When the heme iron is oxidized from the ferrous (Fe(II)) to the ferric (Fe(III)) state, it is unable to bind oxygen and Mb displays a brown color (metmyoglobin), as seen in cooked meat. Heme iron oxidation also reduces the affinity of Mb for heme, leading to increased heme loss and subsequent unfolding of the protein.

The amino acid sequence of Mb has been strongly conserved during evolution, and shows relatively little variation between species (Fig. 2). In Proboscideans, Mb presents an atypical phenylalanine in position 30 (Phe30). Remarkably, the Mb sequence from a steppe mammoth (*Mammuthus trongotherii*), which we generated from the so-called Adycha specimen, dating between 1.2 and 1.0 Ma, is also included in Fig. 2.

In addition to the characteristic Phe30 substitution (Fig. 2), we found that Mb from steppe mammoth displayed a higher positive surface charge (net surface charge ($Z_{Mb}$) at pH 6.5 = 2.67) than Mb from e.g. the asian elephant ($Z_{Mb}$ at pH 6.5 = 2.11), due to the presence of a histidine residue instead of a glutamine at position 92 (His92) (Fig. 2, 3). This is reminiscent of adaptation of Mb to deep-sea diving in cetaceans, where increased positive surface charge led to decreased attractive interactions between Mb molecules at a contact distance, increasing Mb stability and preventing its aggregation. Overall, Mb from the steppe mammoth remarkably presents several characteristics that make it particularly attractive. Here, we evaluate the production and characteristics of recombinant Mb from the steppe mammoth, next to other extant species.

*Example 2.3. Extracellular production of myoglobin in Pichia pastoris*

The sequences coding for Mb from the steppe mammoth, pig, chicken, cattle, pork and tuna were cloned downstream of the methanol-inducible AOX1 promoter of *Pichia pastoris,* and upstream of the AOX1 terminator, a histidine prototrophy selection marker and the so-called 3' fragment of the *AOX1* gene. Myoglobin is naturally found in the cytoplasm of muscle cells. To facilitate recombinant protein purification, we fused a sequence encoding a signal peptide in frame with the myoglobin coding sequence, in order to target nascent Mb proteins to the secretory pathway for excretion outside the cell. These constructs were used to transform histidine-auxotroph *Pichia pastoris cells,* and transformants were selected for their ability to grow in the absence of histidine. For each construct, up to ten transformants were tested for their ability to produce extracellular animal myoglobin after methanol induction in microplates. The best producing clones were further tested in flasks. Methanol induction led to the cell-free supernatant displaying a dark red color, especially after 10-fold concentration by ultrafiltration, as expected for samples containing a heme protein (Fig. 4A). The presence of a methanol-inducible protein of the expected molecular weight, about 17 kD, was also confirmed after protein electrophoresis and staining with Coomassie blue (Fig. 4B). Recombinant Mb accounted for 75-82% of the total proteins in the concentrated cell-free supernatant, while the yield for Mb production varied between 0.420mg and 1.93g per liter of supernatant.

Since myoglobin is produced extracellularly, yeast cells do not need to be lyzed during the purification process. These cells are removed from the fermentation broth by centrifugation, followed by microfiltration (0.45 $\mu$m pores). The final product is therefore not expected to contain any recombinant genetic material. To verify this, we designed a PCR test based on the amplification of a short fragment (130 bp) of the recombinant gene, corresponding to the sequence encoding the signal peptide, from as little as 1pg of DNA (Fig. 5). Using this test, we failed to detect any recombinant DNA in the concentrated cell-free supernatant (Fig. 5).

In order to confirm identity of the recombinant Mb, and ensure that the signal peptide was correctly processed and removed during protein secretion, we analyzed the mammoth Mb gel band by liquid chromatography coupled to mass spectrometry (LC-MS/MS) shotgun measurement. We recovered peptides covering of 90.3% of the full protein sequence and observed complete removal of the signal peptide and the first methionine, as expected (Fig. 6).

*Example 2.4. Color and color stability of recombinant myoglobin*

As discussed above, Mb plays a central role in meat color. In parallel with the mass spectrometry analysis, we therefore measured absorbance of the concentrated cell-free supernatants in the UV and visible range, and compared it with the absorbance of commercial myoglobin purified from equine muscle. In all cases, an absorbance peak (Soret band) characteristic of the presence of heme was observed (Tang et al. 2004). The Soret band was detected at 410 nm for the cattle and horse Mb, while it was slightly red-shifted to 415 nm for the mammoth Mb (Fig. 7A). Based on the absorbance ratios at the wavelength maxima representative for metmyoglobin, deoxymyoglobin, and oxymyoglobin, respectively (Tang et al. 2004), we observed that recombinant myoglobin in the concentrated cell-free supernatant is mainly found in the reduced form: 47.3% oxymoyglobin and 10.0% deoxymyoglobin for the mammoth Mb, 48.3% oxymoyglobin and 9.9% deoxymyoglobin for the cattle Mb. Of note, the Soret peak of mammoth Mb was consistently observed at a slightly higher wavelength than for the cattle and horse Mb (415 nm versus 410 nm, respectively). This is consistent with previous observations made for elephant Mb (Tada et al. 1998). In order to assess the auto-oxidation behavior of Mb, we recorded absorbance spectra every two hours during a 24-hour incubation at pH 5.6 and 25°C (Fig. 7B). We then examined the ratio between absorbance at 580nm (peak value for oxymyoglobin, carrying Fe(II)) and at 505 nm (peak value for metmyoglobin, carrying Fe(III)). At the start of the incubation, we observed that this ratio was significantly higher for the recombinant mammoth and cattle Mb than for commercially available Mb purified from equine muscle (Fig. 7B, insert), indicating that a larger fraction of recombinant Mb is present in the reduced form. This difference with horse Mb was maintained over time at acidic pH (Fig. 7B, insert). Over the course of 24 hours, the absorbance ratio decreased by 26.0% for the mammoth Mb and 38.3% for the cattle Mb, suggesting that the former shows a greater resistance to auto-oxidation.

Meat color is affected by multiple parameters, including Mb concentration, moisture and fat content. For traditional meat, which is typically purchased raw, the red color imparted by oxymyoglobin plays a key role in consumers purchase decision. We therefore tested the effect of recombinant Mb on meat analogues in terms of color. Addition of cattle or mammoth Mb to plant-based burgers (Fig. 8A-A') caused a decrease in lightness (L*-value), and an increase in redness (a*-value) and yellowness (b*-value) (Fig. 8B).

Next, we assessed color stability of meat analogues containing various amounts of myoglobin, which were stored in a cold room and exposed to constant light for 5 days. A commercial plant-based burger containing a soy heme protein was included for comparison. While we observed significant color change for the burger prepared without Mb, the addition of Mb was associated with a greater color stability (Fig. 9A-B).

*Example 2.5. Aromatic analysis*

Besides its role in meat color, myoglobin is generally assumed to contribute to the "bloody" or "metallic" smell of raw meat. During meat cooking, aroma formation occurs essentially by lipid oxidation and the Maillard reaction. The later takes place between amino groups in proteins and carbonyl groups from reduced sugars and/or reaction products of lipid oxidation. Heme iron can influence these reactions and/or their kinetics (van Ba et al. 2012). Yet, to our knowledge there is no conclusive data in the literature about the influence of myoglobin on aroma formation in meat. Using lab-made burgers as described above, we analyzed volatile compounds from raw and cooked plant-based burger containing recombinant Mb by gas chromatography coupled with mass spectrometry.

We found that the addition of Mb to a plant-based burger significantly increased the number of volatile compounds, both in the raw state and after grilling (Fig. 10A). Noticeably, the number of volatiles produced was higher in both case than with a commercial plant-based burger containing soy leghemoglobin (Fig. 10A), i.e. the Impossible Burger. Using Principal Component Analysis (PCA) on the CG-MS data, we observed a clear separation between lab-based burgers and commercially available plant-based burger containing soy leghemoglobin on the one hand, and between raw and baked products on the other hand (Fig. 10B).

When analyzing the volatile compounds from the grilled lab-made plant-based burgers, we found that addition of Mb was associated with the presence of oxidized lipids, lipid oxidation products and pyrazines. In particular, the presence of steppe mammoth or cattle Mb led to increased amounts of compounds known to confer a "roasted" taste to grilled meat (van Ba et al. 2012) (Fig. 12). The addition of mammoth Mb was consistently associated with a higher amount of these compounds than what was observed with the cattle Mb. This suggests that, without being bound to this theory, when used as an ingredient for plant-based meat analogues, lower amounts of mammoth Mb could be added to obtain the same aromatic properties than when using cattle Mb. Additionally, the mammoth Mb and cattle Mb might provide a different sensory experience.

*Example 2.6. Nutritional analysis*

In order to assess the bioavailability of iron from Mb, we turned to the epithelial-like intestinal cell line Caco-2, which is commonly used to study human intestinal iron uptake. We measured the formation of intracellular ferritin as a readout of iron uptake by a living Caco-2 cell monolayer, and therefore an indicator of iron bioavailability (Glahn et al. 1998). We observed that increasing doses of Mb led to an increase in iron uptake (Fig. 12), indicating that heme-iron carried by Mb is bioavailable to human intestinal cells.

*Example 2.7. Safety testing*

*Cytotoxicity*

In order to assess the potential toxicity of recombinant Mb, differentiated Caco-2 cells were treated with the mammoth or cattle Mb, with purified bovine Mb or with complete medium (CM) as a control. To test for potential cytotoxic effects, we then examined the levels of lactate dehydrogenase (LDH) in the culture supernatant. LDH is an oxidoreductase present in all cells, which catalyzes the interconversion of pyruvate to lactate with concomitant interconversion of NADH to NAD+. Upon cell membrane damage following apoptosis or necrosis, LDH is released in the supernatants and its concentration can be determined by a colorimetric assay based on the reduction of NAD+ to NADH by LDH (Decker and Lohmann-Matthes 1988). We did not observe an increase in cytotoxicity upon incubation with mammoth Mb at any of the concentrations tested (Fig. 13). In contrast, the cattle Mb showed significantly increased toxicity compared to the medium alone (Fig. 13). At the high concentrations tested here, the mammoth Mb therefore appears to have a better safety profile than cattle Mb.

*Genotoxicity*

In order to determine the mutagenic potential of recombinant myoglobin, we used a bacterial reverse mutagenesis test (AMES test) and four histidine-requiring strains of *Salmonella typhimurium* (TA98, TA100, TA1535, TA1537) and one tryptophan-requiring strain of Escherichia coli (*WP2 uvrA*) (Ames et al. 1975). Bacteria were exposed to mammoth or cattle myoglobin at levels of 0.8, 2.5, 8.0, 25.0, 80.0 or 250 $\mu$g/ml, either with or without exogenous metabolic activation (S9 mix) (Table 1). The positive control substances caused the expected increase in number of revertants, both the absence and the presence of S9, confirming the sensitivity of the test and the activity of the S9 mix. We did not observe any significant mutagenic activity for the cattle or mammoth Mb at any of the concentrations tested.

Table 1. Number of revertants and mutation factor after exposure to mammoth myoglobin. The mean number of revertants observed for a total of 48 wells per condition is indicated in black. The corresponding mutation factor (ratio of the revertant counts in presence of a compound versus the vehicle alone) is indicated in grey. All assays were performed in triplicates. Conditions where significant mutagenesis was observed are highlighted in bold. Compounds used as positive controls were [a] 2-nitrofluorene 2 $\mu$g/ml, [b] aminoanthracene 4 $\mu$g/ml, [c] N4-aminocytidine 100 $\mu$g/ml.

| | AMES test | | | | | |
|---|---|---|---|---|---|---|
| Strain | TA98 | | TA100 | | TA1535 | |
| Test compound | -S9 | +S9 | -S9 | +S9 | -S9 | +S9 |
| Vehicle | 1.33±1.53 | 1.67±2.08 | 8.00±2.00 | 6.00±5.20 | 2.33±1.53 | 3.00±1.0 |
| Mb 0.003 $\mu$g/$\mu$l | 0.00±0.00 | 1.00±0.00 | 10.0±2.65 | 7.00±2.65 | 2.33±0.58 | 2.33±0.58 |
| | 0.00±0.00 | 0.60±0.00 | 1.25±0.33 | 1.17±0.44 | 1.0±0.25 | 0.78±0.19 |
| Mb 0.003 $\mu$g/$\mu$l | 2.33±2.52 | 1.33±1.58 | 9.33±2.52 | 4.67±2.08 | 1.00±1.00 | 2.67±0.58 |
| | 1.75±1.89 | 0.80±0.35 | 1.17±0.31 | 0.78±0.69 | 0.43±0.43 | 0.89±0.19 |
| Mb 0.003 $\mu$g/$\mu$l | 1.33±1.53 | 1.33±2.31 | 12.33±7.77 | 9.00±1.73 | 2.37±1.15 | 2.33±1.15 |
| | 1.00±1.15 | 0.80±1.39 | 1.54±0.97 | 1.50±0.58 | 1.14±0.49 | 0.78±0.38 |
| Mb 0.003 $\mu$g/$\mu$l | 0.33±0.58 | 1.00±1.00 | 14.67±5.51 | 7.00±2.00 | 1.00±1.00 | 3.00±1.00 |
| | 0.25±0.43 | 0.60±0.60 | 1.83±0.69 | 1.17±0.67 | 0.43±0.43 | 1.00±0.33 |
| Positive control | **37.33±2.08[a]** | **48.00±0.00[b]** | **47.67±0.58[c]** | **44.00±5.20[b]** | **48.00±0.00[c]** | **21.33±1.50[b]** |
| | 28.0±1.56 | 28.80±0.00 | 5.96±0.07 | 7.33±1.73 | 20.57±0.00 | 7.11±0.51 |

The following numbered items are preferred items of the invention:

1. A meat substitute or a food ingredient comprising an animal myoglobin from mammoth, pig, sheep, cow, chicken or tuna or derived therefrom.

2. A meat substitute or food ingredient according to item 1, wherein the myoglobin is from steppe mammoth, woolly mammoth or derived therefrom.

3. A meat substitute or food ingredient according to item 1 or 2, wherein said animal myoglobin is represented by one of the following amino acid sequences:

a) comprising at least 70% sequence identity with SEQ ID NO: 3, and having at least one of the following amino acid combinations:

◦ F at position 30, and/or
◦ Q at position 65, and/or
◦ H at position 92, and/or
◦ H at position 94, and/or
◦ F at position 30 and Q at position 65, and/or
◦ F at position 30 and H at position 92, and/or
◦ F at position 30 and H at position 94, and/or
◦ Q at position 65 and H at position 92, and/or
◦ Q at position 65 and H at position 94, and/or
◦ H at position 92 and H at position 94, and/or
◦ F at position 30 and Q at position 65 and H at position 92, and/or
◦ F at position 30 and Q at position 65 and H at position 94, and/or
◦ F at position 30 and H at position 92 and H at position 94, and/or
◦ Q at position 65 and H at position 92 and H at position 94, and/or
◦ F at position 30 and Q at position 65 and H at position 92 and H at position 94;

b) comprising at least 70% sequence identity with SEQ ID NO: 2 or 3, Q or H at position 65 and H at position 94 and optionally having at least one of the following amino acids at the following places within SEQ ID NO: 2 or 3: E at position 9, K at position 13, T as position 14, P at position 23, L at position 27, V at position 31, G at position 54, Q at position 65, V at position 67, Q at position 84, Q at position 88, I at position 102, E at position 123 and/or I at position 143;

c) comprising at least 70% sequence identity with SEQ ID NO: 1, Q or H at position 65 and H at position 94 and optionally having at least one of the following amino acids at the following places within SEQ ID NO: 1: E at position 9, K at position 13, T as position 14, P at position 23, L at position 27, V at position 31, G at position 54, Q at position 65, V at position 67, Q at position 84, Q at position 88, I at position 102 and/or E at position 123;

d) comprising at least 70% identity with SEQ ID NO: 4, 5 or 6, Q or H at position 65 and

H at position 94 and optionally having at least one of the following amino acids at the following places within SEQ ID NO: 4, 5 or 6: N at position 13, Q at position 27, I at position 31, N at position 67, A at position 128, S at position 133, A at position 145 and/or L at position 150;

e) comprising at least 70% identity with SEQ ID NO: 7, Q or H at position 65 and H at position 94 and optionally having at least one of the following amino acids at the following places within SEQ ID NO: 7: Q at position 6, Q at position 10, T at position 13, I at position 14, H at position 27, M at position 31, H at position 35, D at position 36, D at position 42, R at position 43, G at position 49, P at position 53, Q at position 55, G at position 58, A at position 67, Q at position 72, K at position 75, Q at position 79, N at position 82, S at position 85, T at position 93, V at position 111, I at position 116, A at position 117, E at position 118, A at position 121, S at position 128, K at position 133, S at position 145 and/or F at position 150; or

f) comprising at least 70% identity with SEQ ID NO: 8, Q or H at position 65 and H at position 94.

5. A meat substitute according to any one of items 1 to 4, which does not comprise a leghemoglobin which has been produced by a bacterium living in symbiosis in the root nodules of a soy plant and/or which comprises as sole heme-containing protein the myoglobin as defined in any one of items 1 to 3.

5. A meat substitute according to any one to items 1 to 4, wherein the meat substitute mimicks the aspect, form, structure, composition, flavor, texture, color, aroma, appearance and/or nutritional value of meat.

6. A food ingredient according to any one of items 1 to 4, wherein the food ingredient mimicks the composition, flavor, color, aroma and/or nutritional value of meat.

7. A gene construct comprising a nucleic acid encoding a myoglobin as defined in any one of items 1 to 4.

8. A gene construct according to item 7, wherein the nucleic acid is selected from the group consisting of:

(a) a nucleotide sequence encoding a polypeptide represented by an amino acid sequence comprising a sequence that has at least 75% sequence identity or similarity with the amino acid sequence defined in claim 3 a), b), c), or d)
(b) a nucleotide sequence that has at least 60% sequence identity with the nucleotide sequence of SEQ ID NO: , 9, 10, 11, 12, 13, 14, 15, 16; and
(c) a nucleotide sequence the sequence of which differs from the sequence of a nucleotide sequence of (b) due to the degeneracy of the genetic code.

9. A gene construct according to claim 7 or 8, further comprising a promoter.

10. A gene construct according to any one of items 7-9, further comprising a signal peptide, preferably a signal peptide that facilitates excretion of the expressed myoglobin.

11. A gene construct according to any one of items 6 to 10, wherein said nucleotide sequence is represented by SEQ ID NO: 19, 20, 21, 22, 23 or 24.

12. A gene construct according to item 11, wherein said gene construct is represented by SEQ ID NO: 25, 26, 27, 28, 29 or 30.

13. A vector comprising a gene construct as defined in any one of items 7 to 12.

14. A host cell comprising a gene construct as defined in any one of items 7 to 12.

15. A host cell according to item 14 which is a prokaryote or a eukaryote.

16. A method for producing a myoglobin as defined in any one of items 1 to 3, comprising culturing the host cell of items 14 or 15 in a suitable medium and optionally recovering the host cell and/or the myoglobin.

17. A method according to item 16, wherein the produced myoglobin does not comprise a signal peptide.

18. A method according to items 16 or 17, wherein the recovered myoglobin is purified, preferably substantially purified.

19. A method according to any one of items 16 to 18, wherein the host cell produces the myoglobin extracellularly.

20. A method for producing a meat substitute or a food ingredient as defined in any one of items 1 to 6, comprising formulating the myoglobin obtainable from the method of any one of items 16 to 19 into a meat substitute or a food ingredient.

21. A protein, wherein said protein may be represented by a sequence comprising SEQ ID NO: 3.

**Claims**

1. A method for producing a meat substitute comprising a myoglobin from cow, comprising the steps of:

a) providing a host cell comprising a gene construct, wherein the gene construct is represented by SEQ ID NO: 21 or 27;

b) culturing the host cell in a suitable medium to produce a myoglobin from cow represented by SEQ ID NO: 5;
c) recovering the myoglobin;
d) purifying the myoglobin;
e) formulating the purified myoglobin into a meat substitute.

2. The method of claim 1, wherein the host cell produces the myoglobin extracellularly.

3. The method of claim 1 or 2, wherein the host cell secretes the myoglobin during step b).

4. The method of any one of claims 1 to 3, wherein the host cell is not lysed during step d).

5. The method of any one of claims 1 to 4, wherein the host cell is a yeast.

6. The method of claim 5, wherein the yeast is *Komagataella phaffii.*

7. The method of any one of claims 1 to 6, wherein the concentration of the myoglobin in the meat substitute obtained at the end of step e) is from 0.1 wt.% up to 0.5 wt.%.

Fig. 1

Fig. 2

```
                  10          20          30          40
Thunnus orientalis     - - - -MADF DAVL KCWG PVEADYTT I GGL VL TRL F KEHPETQKL FPKFAG
Gallus gallus          MGL SDQEWQQVL T I WGKVEAD I AGHGHEVLMRL FHDHPETLDRFDKFKG
Mammuthus trogontherii MGL SDGEWEL VL KTWGKVEAD I PGHGLEVF VRL FTGHPETL EKFDKFKH
Sus scrofa             MGL SDGEWQL VL NVWGKVEADVAGHGQEVL I RL FKGHPETL EKFDKFKH
Bos taurus             MGL SDGEWQL VL NAWGKVEADVAGHGQEVL I RL FTGHPETL EKFDKFKH
Ovis aries             MGL SDGEWQL VL NAWGKVEADVAGHGQEVL I RL FTGHPETL EKFDKFKH

                  50          60    65    70          80       90 92 94
Thunnus orientalis     I AQA - D I AGNAAVSA HGATVL KKL GEL L KAKGSHAA I L KPL ANS HATKHK
Gallus gallus          L KTPDQMKGSEDL KK HGATVL TQL GK I L KQKGNHESEL KPL AQT HATKHK
Mammuthus trogontherii L KTEGEMKASEDL KK QGVTVL TAL GG I L KKKGHHQAE I QPL AHS HATKHK
Sus scrofa             L KSEDEMKASEDL KK HGNTVL TAL GG I L KKKGHHEAEL TPL AQS HATKHK
Bos taurus             L KTEAEMKASEDL KK HGNTVL TAL GG I L KKKGHHEAEVKHL AES HANKHK
Ovis aries             L KTEAEMKASEDL KK HGNTVL TAL GG I L KKKGHHEAEVKHL AES HANKHK

                  100         110         120         130         140         150
Thunnus orientalis     I P I NNF KL I SEVL VKVMHEKAGL D - - AGGQTAL RNVMG I I I ADL EANYKEL GF SG
Gallus gallus          I PVKYL EF I SEV I I KV I AEKHAADF GADSQAAMKKAL EL FRNDMASKYKEFGFQG
Mammuthus trogontherii I P I KYL EF I SDA I I HVL QSKHPAEF GADAQGAMKKAL EL FRND I AAKYKEL GFQG
Sus scrofa             I PVKYL EF I SEA I I QVL QSKHPGDF GADAQGAMSKAL EL FRNDMAAKYKEL GFQG
Bos taurus             I PVKYL EF I SDA I I HVL HAKHPSDF GADAQAAMSKAL EL FRNDMAAQYKVL GFHG
Ovis aries             I PVKYL EF I SDA I I HVL HAKHPSDF GADAQGAMSKAL EL FRNDMAAQYKVL GFQG
```

44

**Fig. 3**

asian elephant

steppe mammoth

Fig. 4

**A**

mammoth Mb +    cattle Mb +    cattle Mb -    methanol

**B**

Fig. 5

Fig. 6

log10 of peptide intensity

**Fig. 7**

**Fig.8**

Fig. 9

Fig. 10

**A**

total volatiles

**B**

Scores

**Fig. 11**

**A**

**B**

| Compound name | Aroma flavor characteristics |
| --- | --- |
| methylpyrazine | Roasted, nutty, cocoa, chocolate, peanut, green |
| 2,5-dimethylpyrazine | Roasted, beefy, nutty, fried rice, pungent, green |
| 2-ethyl-6-methylpyrazine | Roasted, potato, hazelnut |
| pyrrole | Sweet, warm, nutty, cereal-like |
| 2-methylbutanal | Roasty, pungent, sweet, nutty, caramellic |
| 3-methylbutanal | Meaty, fish, fatty, cocoa, pungent |

**Fig. 12**

**Fig. 13**

## REFERENCES CITED IN THE DESCRIPTION

### Patent documents cited in the description

- US 3983000 A
- US 4071409 A
- US 3519538 A
- US 3652761 A

### Non-patent literature cited in the description

- **PROULX A.K. et al.** *J. Agric. Food. Chem.*, 2006 (54), 1518-1522
- **VAN'T RIET, K.** ; **TRAMPER, J.** Basic Bioreactor Design. CRC Press, 1991
- **BENNETT, W.** ; **LASURE, L.** More Gene Manipulations in Fungi. Academic Press, 1991
- **WESSELINGH, J.A** ; **KRIJGSMAN, J.** Downstream Processing in Biotechnology. Delft Academic Press, 2013
- **SAMBROOK** ; **RUSSEL**. Molecular Cloning: A Laboratory Manual. Cold Spring Harbor Laboratory Press, 2001
- Current protocols in molecular biology. Green Publishing and Wiley Interscience, 2003
- **GENTZ et al.** *Proc. Natl. Acad. Sci. USA*, 1989, vol. 86, 821-824
- **GUISAN, J.M.** ; **BOLIVAR, J.M.** ; **LÓPEZ-GALLEGO, F.** ; **ROCHA-MARTIN, J.** Immobilization of Enzymes and Cells: Methods and Protocols. Springer US, 2020
- **COLIN R.** ; **PHILLIPS C.R.** ; **POON Y. C.** Immobilization of Cells: In Biotechnology Monographs book series. Springer, 1988, vol. 5
- **TAMPION J.** ; **TAMPION M. D.** Immobilized Cells: Principles and Applications. Cambridge University Press, 1987
- **XIA X.** Bioinformatics and the Cell: Modern Computational Approaches in Genomics, Proteomics and transcriptomics. Springer International Publishing, 2018
- **MOUNT D.** Bioinformatics: Sequence and Genome Analysis. Cold Spring Harbor Laboratory Press, 2004
- **HENIKOFF** ; **HENIKOFF**. *PNAS*, 1992, vol. 89, 915-919
- **ALTSCHUL et al.** *J. Mol. Biol.*, 1990, vol. 215, 403-10
- **ALTSCHUL et al.** *Nucleic Acids Res.*, 1997, vol. 25 (17), 3389-3402
- **AUSUBEL et al.** Current Protocols in Molecular Biology. Greene Publishing and Wiley-Interscience, 1987
- **KUNKEL**. *Proc. Natl. Acad. Sci.*, 1985, vol. 82, 488
- **ROBERTS et al.** *Nature*, 1987, vol. 328, 731-734
- **WELLS, J.A. et al.** *Gene*, 1985, vol. 34, 315
- **METZGER et al.** *Nature*, 1988, vol. 334, 31-36
- **VAN BA et al.** *The Good Scent Company information system*, 2021
- **GIETZ RD et al.** *Methods Enzymol.*, 2002, vol. 350, 87-96
- **VERDUYN C. et al.** *Yeast*, 1992, vol. 8, 501-517